# EUROPEAN PATENT SPECIFICATION

(11) **EP 3 508 120 B1**
(45) Date of publication and mention of the grant of the patent: **08.06.2022**
(21) Application number: 16915086.9
(22) Date of filing: 30.08.2016
(51) Int. Cl.: A61B 5/11

(54) **INFORMATION PROCESSING DEVICE, INFORMATION PROCESSING SYSTEM AND INFORMATION PROCESSING METHOD**
INFORMATIONSVERARBEITUNGSVORRICHTUNG, INFORMATIONSVERARBEITUNGSSYSTEM UND INFORMATIONSVERARBEITUNGSVERFAHREN
DISPOSITIF DE TRAITEMENT D'INFORMATIONS, SYSTÈME DE TRAITEMENT D'INFORMATIONS ET PROCÉDÉ DE TRAITEMENT D'INFORMATIONS

(43) Date of publication of application: 10.07.2019
(73) Proprietor: Fujitsu Limited, Kawasaki-shi, Kanagawa 211-8588 (JP)
(72) Inventor: SASAMOTO, Yuki, Kawasaki-shi Kanagawa 211-8588 (JP); INOMATA, Akihiro, Kawasaki-shi Kanagawa 211-8588 (JP); HOTTA, Shinji, Kawasaki-shi Kanagawa 211-8588 (JP); MAEDA, Kazuho, Kawasaki-shi Kanagawa 211-8588 (JP); NAKATA, Yasuyuki, Kawasaki-shi Kanagawa 211-8588 (JP)
(74) Representative: Haseltine Lake Kempner LLP
(86) International application number: PCT/JP2016/075373
(87) International publication number: WO 2018/042525

(56) References cited:
- WO-A1-2008/129442
- WO-A1-2015/009228
- WO-A1-2015/054312
- WO-A1-2015/150931
- JP-A- 2005 168 590
- JP-A- 2012 110 713
- JP-A- 2016 158 954
- US-A1- 2010 262 045

## Description

### Technical Field

The present invention relates to an information processing apparatus, an information processing system, and an information processing method.

### Background Art

In the related art, in a laboratory, the recovery of a living body is sometimes evaluated by an operator performing an experiment visually observing a motion of the living body such as a patient, or by measuring the motion of the living body by a sensor.

As a related prior art, for example, there is a technique of calculating at least one standard feature quantity based on feature quantities of at least one or more reference individuals, calculating a transformation matrix between the standard feature quantity and a subject feature quantity, and transforming an identification feature quantity of identification target motion data by using the transformation matrix. In addition, there is a technique of superimposing a rhythm cycle candidate wave related to a rhythmic motion obtained by performing a pattern matching process on body motion signal information, on an auxiliary wave produced by performing coarse-graining on a motion trajectory that is obtained by performing one or more times integration on the body motion signal information, and selecting a cycle of a candidate wave which has a peak in the auxiliary wave. Also, there is a technique of creating a three-dimensional bone model specific to a patient, registering the actual patient bone with the bone model, tracking a bone motion of the patient who makes a motion in a predetermined range, and comparing the bone motion of the patient with a database including bone motion data in order to diagnose the damage.

### Citation List

### Patent Literature

PTL 1: Japanese Laid-open Patent Publication No. 2011-177300
PTL 2: Japanese Laid-open Patent Publication No. 2011-92696
PTL 3: Japanese National Publication of International Patent Application No. 2012-516719

Reference may be made to any of:
WO 2015/150931 A1, which relates to a real time visualization method and system for evaluating and monitoring recovery progress of Anterior Cruciate Ligament reconstructed / injured subject, wherein the method includes receiving multiple super-imposed bio-signals from an electromyogram (EMG) for muscles of limbs of the patient and signals pertaining to three dimensional kinematics measurement from a motion sensor set, processing the signals to extract wavelet features and evaluating the wavelet features to determine the extent of rehabilitation and a recovery classification of the patient's limb from ACL injury, and wherein the system includes an input interface circuit, a processing module, a comparator module and an output interface circuit for providing an estimate of the extent of rehabilitation of the patient's limb from ACL injury; and
WO 2008/129442 A1, which relates to a method of assessing a movement pattern, a system for assessing a movement pattern using the method, and uses of the system for training of a movement and for a motor impairment rehabilitation therapy, wherein personalized motion analysis algorithms take the changing movement patterns of a person due to training or treatment into account and allow real-time feedback and an easy-to-understand performance measure for the patient, and the algorithms can be generically used for a variety of sensor types, implementation details and for all applications where training or recovery of motor abilities is essential.

### Summary of Invention

### Technical Problem

However, according to the related art, the reliability for the recovery evaluation of a living body such as a patient may decrease. For example, in a case where a method of operating the experiment or a worker in the experiment is different, the measurement result may change.

In one aspect, an object of the present invention is to provide an information processing apparatus, an information processing system and an information processing method capable of improving the reliability for the recovery evaluation of a living body.

### Solution to Problem

The present invention is defined by the independent claims, to which reference should now be made. Specific embodiments are defined in the dependent claims. According to the invention, there are provided an information processing apparatus, an information processing system and an information processing method which specify, based on time series data obtained from a sensor that measures a state and/or a motion of a living body and a model having one or more feature quantities indicating a predetermined motion or a predetermined state, each data section in which the living body makes the predetermined motion or is in the predetermined state from the time series data, by determining a score for the time series data based on similar feature quantities appearing for each feature quantity of the model and defining a data section if the score exceeds a threshold value; store each specified data section in association with the model, and calculate a conformance degree that is a suitable degree of each data section for recovery evaluation of the living body, based on a relationship between the stored data sections or a relationship between each data section and a past data section that has data measured before data of each data section and is associated with the model by executing a conformance degree calculation process of calculating a conformance degree obtained by comparing an occurrence time of the motion, an occurrence location of the motion, and the feature quantity of the data section with a distribution of past occurrence times of the motions, a distribution of past occurrence locations of the motions, and a distribution of past feature quantities, respectively, in such a manner that a high value is set as the conformance degree when the occurrence time, the occurrence location or the feature quantity is positioned at a corresponding average value or a median value of the corresponding distribution and a low value is set as the conformance degree when the occurrence time, the occurrence location or the feature quantity is not positioned at a corresponding average value or a median value of the corresponding distribution, wherein the conformance degree is based on the position of each of the time, the location, and the motion feature quantity.

### Advantageous Effects of Invention

According to the aspect of the present invention, it is possible to improve the reliability for the recovery evaluation of a living body.

### Brief Description of Drawings

[FIG. 1] FIG. 1 is an explanatory diagram illustrating an operation example of an information processing apparatus 101 according to an embodiment.
[FIG. 2] FIG. 2 is an explanatory diagram illustrating a configuration example of an information processing system 200.
[FIG. 3] FIG. 3 is an explanatory diagram illustrating an example of a hardware configuration of the information processing apparatus 101, a data acquisition apparatus 201, and a mobile terminal 202.
[FIG. 4] FIG. 4 is an explanatory diagram illustrating a functional configuration example of the information processing system 200 in Example 1.
[FIG. 5] FIG. 5 is a flowchart illustrating an example of a recovery evaluation process procedure.
[FIG. 6] FIG. 6 is an explanatory diagram illustrating an example of data collection.
[FIG. 7] FIG. 7 is an explanatory diagram illustrating an example of defining data.
[FIG. 8] FIG. 8 is an explanatory diagram (part 1) illustrating an example of calculating a situation conformance degree.
[FIG. 9] FIG. 9 is an explanatory diagram (part 2) illustrating the example of calculating a situation conformance degree.
[FIG. 10] FIG. 10 is an explanatory diagram (part 3) illustrating the example of calculating a situation conformance degree.
[FIG. 11] FIG. 11 is an explanatory diagram (part 1) illustrating an example of calculating a habit conformance degree.
[FIG. 12] FIG. 12 is an explanatory diagram (part 2) illustrating the example of calculating a habit conformance degree.
[FIG. 13] FIG. 13 is an explanatory diagram illustrating an example of a model indicating a state change.
[FIG. 14] FIG. 14 is an explanatory diagram illustrating an example of a temporal change due to intervention.
[FIG. 15] FIG. 15 is an explanatory diagram illustrating an example of the state of walking of a patient uA in Example 1.
[FIG. 16] FIG. 16 is an explanatory diagram illustrating an example of a sensor waveform at the time of walking of the patient uA in Example 1.
[FIG. 17] FIG. 17 is an explanatory diagram (part 1) illustrating an example of defining a data section in Example 1.
[FIG. 18] FIG. 18 is an explanatory diagram (part 2) illustrating the example of defining a data section in Example 1.
[FIG. 19] FIG. 19 is an explanatory diagram illustrating an example of calculating the situation conformance degree in Example 1.
[FIG. 20] FIG. 20 is an explanatory diagram illustrating an example of calculating the habit conformance degree in Example 1.
[FIG. 21] FIG. 21 is an explanatory diagram illustrating an example of calculating a temporal-change conformance degree in Example 1.
[FIG. 22] FIG. 22 is an explanatory diagram illustrating an example of specifying an evaluation condition matching section in Example 1.
[FIG. 23] FIG. 23 is an explanatory diagram illustrating an example of selecting an evaluation condition matching section in Example 1.
[FIG. 24] FIG. 24 is an explanatory diagram illustrating an example of calculating a recovery score in Example 1.
[FIG. 25] FIG. 25 is an explanatory diagram (part 1) illustrating a functional configuration example of an information processing system 2500 in Example 2.
[FIG. 26] FIG. 26 is an explanatory diagram (part 2) illustrating the functional configuration example of the information processing system 2500 according to Example 2.
[FIG. 27] FIG. 27 is a flowchart (part 1) illustrating an example of a process procedure of a health state evaluation unit 2601.
[FIG. 28] FIG. 28 is a flowchart (part 2) illustrating the example of the process procedure of the health state evaluation unit 2601.
[FIG. 29] FIG. 29 is a flowchart (part 1) illustrating an example of a process procedure of a walking start motion evaluation unit 2602.
[FIG. 30] FIG. 30 is a flowchart (part 2) illustrating the example of the process procedure of the walking start motion evaluation unit 2602.
[FIG. 31] FIG. 31 is a flowchart (part 1) illustrating an example of a process procedure of a direction change motion evaluation unit 2603.
[FIG. 32] FIG. 32 is a flowchart (part 2) illustrating the example of the process procedure of the direction change motion evaluation unit 2603.
[FIG. 33] FIG. 33 is a flowchart (part 1) illustrating an example of a process procedure of a long-term stationary state evaluation unit 2604.
[FIG. 34] FIG. 34 is a flowchart (part 2) illustrating the example of the process procedure of the long-term stationary state evaluation unit 2604.
[FIG. 35] FIG. 35 is a flowchart (part 1) illustrating an example of a process procedure of a long-term walking motion evaluation unit 2605.
[FIG. 36] FIG. 36 is a flowchart (part 2) illustrating the example of the process procedure of the long-term walking motion evaluation unit 2605.
[FIG. 37] FIG. 37 is a flowchart illustrating an example of a process procedure of a total evaluation unit 2606.
[FIG. 38] FIG. 38 is an explanatory diagram illustrating an example of calculating a situation conformance degree of a long-term stationary state during a standing balance test.
[FIG. 39] FIG. 39 is an explanatory diagram illustrating an example of calculating a recovery score in a long-term stationary state during a standing balance test.
[FIG. 40] FIG. 40 is an explanatory diagram illustrating an example of calculating a situation conformance degree of a long-term walking motion during a walking test.
[FIG. 41] FIG. 41 is an explanatory diagram illustrating an example of calculating a recovery score in a long-term walking motion during a walking test.
[FIG. 42] FIG. 42 is an explanatory diagram illustrating an example of calculating a recovery score in a walking start motion during a walking test.
[FIG. 43] FIG. 43 is an explanatory diagram illustrating an example of calculating a situation conformance degree of a direction change motion during mobility test.
[FIG. 44] FIG. 44 is an explanatory diagram illustrating an example of calculating a recovery score in a direction change motion during a mobility test. Description of Embodiments

Embodiments of an information processing apparatus, an information processing system, an information processing method, and an information processing program of the disclosure will be described in detail below with reference to the drawings.

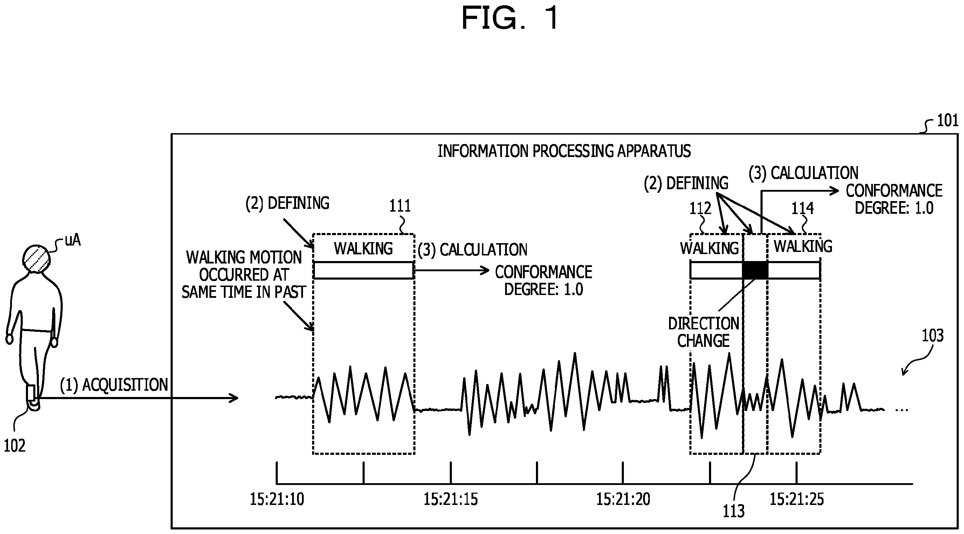

FIG. 1 is an explanatory diagram illustrating an operation example of an information processing apparatus 101 according to an embodiment. The information processing apparatus 101 is a computer that quantifies an evaluation of a recovery of a living body to be treated. Here, the living body is, for example, a person who is treated by a doctor, that is, a patient. In the following description, the living body is described as a patient.

A quantitative evaluation of a recovery of a patient is required to optimize medical care. For example, in falls, cerebral infarction, and the like, there is a demand to grasp an improvement situation of a patient after disease and to grasp effects of treatment and medication. In rheumatism/arthritis, lumbago, diabetes, and the like, there is a demand to grasp efforts for improving disease and to give appropriate daily guidance. Also, in diabetes, dementia, Chronic Obstructive Pulmonary Disease (COPD), and the like, there is a demand of a patient who wants disease control, maintaining of the state, and prophylaxis by himself/herself. In concussion, in order to avoid second impact, there is a demand to know whether a player has really recovered, or when a player returns to the field.

In response to such demands described above, the following methods are available as methods for quantifying the evaluation of a recovery of a patient. There are an evaluation method using a result of question paper answered by a patient, an evaluation method by observing the patient's motion in a test environment in a laboratory, a method based on a brain measurement and a gaze measurement, a method based on a motion measurement of a person by using a wearable sensor or the like.

However, with the methods described above, the reliability for the recovery evaluation of a patient may be reduced. For example, in a case where a method of operating the experiment or a worker in the experiment is different, the measurement result may change. In addition, there is a possibility that an influence due to a difference in the situation of a patient is erroneously determined to be a change in a recovery state of a patient. Also, unless the method provides an index that matches the findings of a doctor, such as a set of causes and results, the method may not be accepted as an evaluation of a recovery of a patient. In this way, it is required to extract data after the evaluation condition of individuals is grasped, accumulate the data, and visualize the data.

Therefore, in the present application, defining each data section by using a model indicating a state or a motion from time series data obtained by measuring a motion of a patient, and calculating a conformance degree of each data section from a relationship of data sections or a relationship of a data section and a past data section will be described.

An operation example of the information processing apparatus 101 will be described with reference to FIG. 1. In the example of FIG. 1, a user uA is a patient to be treated. As a sensor for measuring the motion of the user uA, a sensor 102 is attached to a leg of the user uA. Here, the sensor 102 may not be attached to the user uA, and may be attached to, for example, a floor on which the user uA walks, or a door that the user uA opens and closes. The sensor 102 is, for example, an acceleration sensor or a gyro sensor.

As illustrated by (1) in FIG. 1, the information processing apparatus 101 acquires time series data from the sensor 102. A graph 103 illustrated in FIG. 1 illustrates data from 15:21:10 as time series data.

Then, the information processing apparatus 101 specifies, based on time series data obtained from the sensor 102 and a model indicating a predetermined motion or a predetermined state, each data section in which a patient makes the predetermined motion or is in the predetermined state, from the time series data. The model described above indicates a feature appearing in the data obtained by measuring a motion of a person when the person makes the predetermined motion or is in the predetermined state. Examples of the model indicating the predetermined motion or the predetermined state include a walking model, a direction change model, a standing model, a seated model, and a stationary state model. In addition, the model indicating the predetermined operation or the predetermined state used by the information processing apparatus 101 may be one or plural.

Then, the information processing apparatus 101 stores each identified data section in association with the model. In the following description, specifying each data section and storing each specified data section in association with the model is referred to as "a data section is defined as a section in which a user performs a predetermined motion or is in a predetermined state". Also, the defined data section is referred to as a "defined model name + section". For example, a data section defined that a user walks by a walking model is referred to as a "walking section".

In the example of FIG. 1, the information processing apparatus 101 illustrates an example in which a walking model and a direction change model are used as a model indicating a predetermined motion or a predetermined state. As illustrated by (2) in FIG. 1, the information processing apparatus 101 defines data sections 111, 112, and 114 in the time series data as a walking section and defines a data section 113 as a direction change section. Hereinafter, the data sections 111, 112, and 114 are referred to as walking sections 111, 112, and 114, respectively, and the data section 113 is referred to as a direction change section 113.

Next, the information processing apparatus 101 calculates a conformance degree of each data section based on a relationship between a data section and a past data section which has data measured before data of each data section and is associated with a model, and optionally based on a relationship between data sections. Here, the conformance degree of the data section is a degree that the data section is suitable for the evaluation of the patient.

For example, as an example of using the relationship between data sections, regarding a direction change section, in a case where walking sections are present before and after the direction change section, a conformance degree of the direction change section is calculated to be high, for example, 1.0 is added to the conformance degree of the direction change section. As illustrated in FIG. 1, since the walking sections 112 and 114 are present before and after the direction change section 113, the information processing apparatus 101 calculates the conformance degree of the direction change section 113 as 1.0 by adding 1.0 to an initial value 0 of the conformance degree of the direction change section 113 as illustrated by (3) of FIG. 1.

Further, as an example of using the relationship between the past data section and the data section, in a case where a walking motion occurred at the same time in the past, a conformance degree of the data section is calculated to be high, for example, 1.0 is added to the conformance degree of the direction change section. As illustrated in FIG. 1, since the walking motion occurred at the same time in the past as the time when the walking section 111 has occurred, as illustrated by (3) in FIG. 1, the information processing apparatus 101 calculates a conformance degree of the walking section 111 as 1.0 by adding 1.0 to an initial value 0 of the conformance degree of the walking section 111.

As a result, the doctor treating the user uA views the conformance degree 1.0 of the walking section 111 and the conformance degree 1.0 of the direction change section 113, and is able to grasp that the walking section 111 and the direction change section 113 are data sections suitable for evaluating a recovery of the user uA. In this manner, the information processing apparatus 101 is able to improve the reliability for the quantification of the recovery state of the user uA. In addition, the information processing apparatus 101 may discard data other than the walking section 111 and the direction change section 113 among the time series data obtained from the sensor 102, and is able to reduce a data amount to be stored.

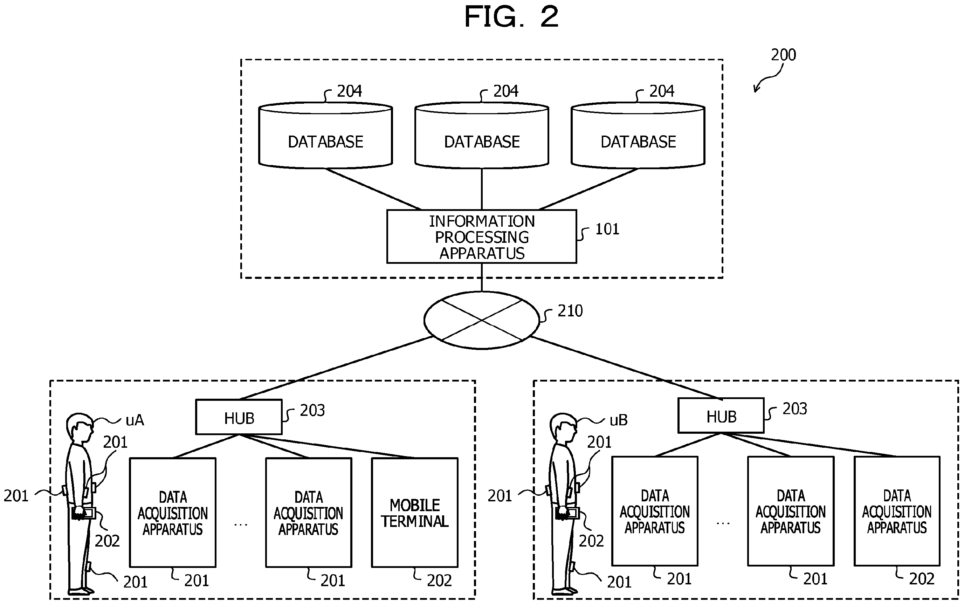

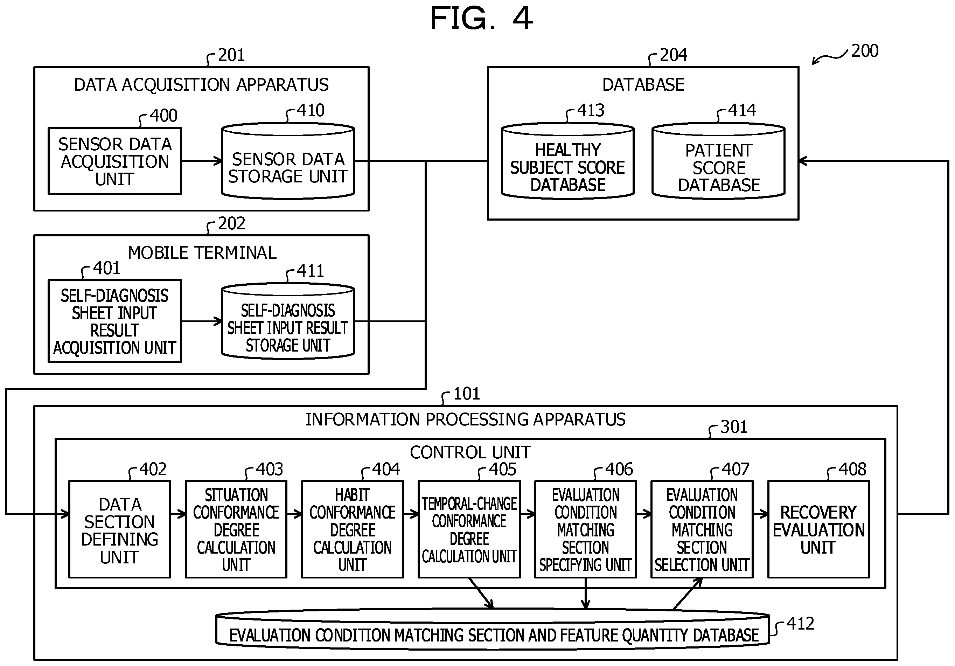

Further, in the example of FIG. 1, the information processing apparatus 101 calculates one conformance degree, but it is not limited thereto. The information processing apparatus 101 calculates at least a conformance degree of a second scene corresponding to a patient's habit of the data section, from conformance degrees including a conformance degree of a first scene corresponding to a situation of the data section, the conformance degree of a second scene corresponding to a patient's habit of the data section, a conformance degree of a third scene corresponding to a state change of the patient of the data section. The conformance degrees of the first to third scenes will be described with reference to FIG. 4 and the following drawings. In the example of FIG. 1, the living body is a patient, but the living body may be a so-called affected animal such as a dog, a cat, or a horse. Next, an information processing system including the information processing apparatus 101 and the sensor 102 will be described with reference to FIG. 2.

FIG. 2 is an explanatory diagram illustrating a configuration example of the information processing system 200. The information processing system 200 includes the information processing apparatus 101, a data acquisition apparatus 201, a mobile terminal 202, a hub 203, and a database 204. The information processing apparatus 101 and the data acquisition apparatus 201 are coupled to each other via a network 210 and the hub 203.

The data acquisition apparatus 201 is an apparatus that is attached to each part of the user and measures the movement of each part. Then, the data acquisition apparatus 201 has the sensor 102, and measures an acceleration of three axes and an angular velocity of three axes of the attached part. In the example of FIG. 2, a plurality of data acquisition apparatuses 201 are attached to respective parts of the users uA and uB. The mobile terminal 202 is a computer owned by each of the users uA and uB. For example, the mobile terminal 202 is a cellular phone, a smartphone, or the like.

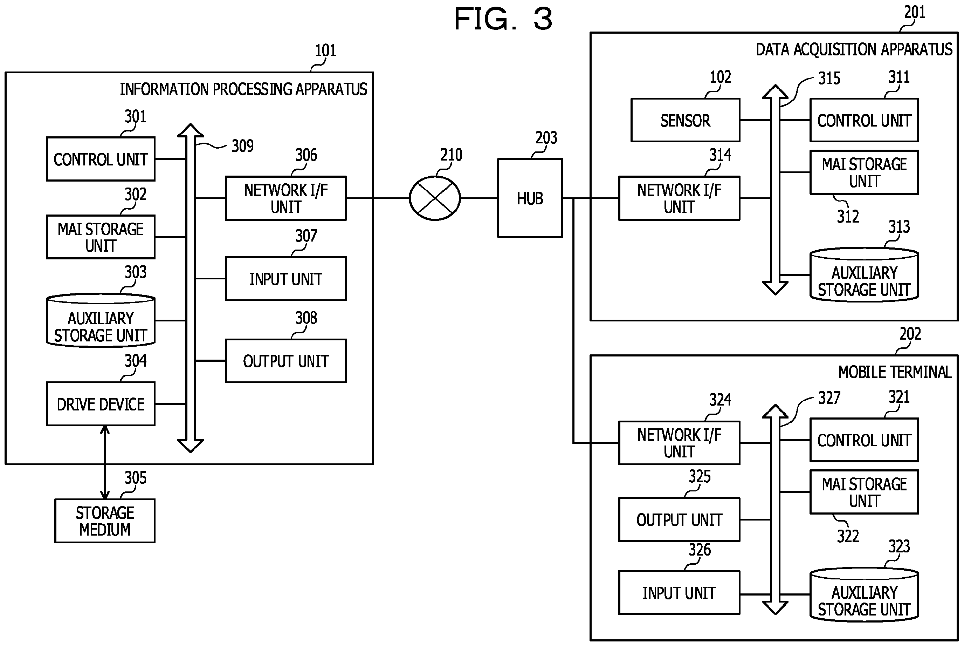

The database 204 stores data acquired from the data acquisition apparatus 201, data converted from the acquired data, and feature quantities extracted from the converted data. Next, with reference to FIG. 3, a hardware configuration of the information processing apparatus 101, the data acquisition apparatus 201, and the mobile terminal 202 will be described.

### (Example of Hardware Configuration of Information Processing Apparatus 101, Data Acquisition Apparatus 201, and Mobile Terminal 202)

FIG. 3 is an explanatory diagram illustrating an example of a hardware configuration of the information processing apparatus 101, the data acquisition apparatus 201, and the mobile terminal 202. In FIG. 3, the information processing apparatus 101 includes a control unit 301, a main storage unit 302, an auxiliary storage unit 303, a drive device 304, a network interface (I/F) unit 306, an input unit 307, and an output unit 308. The control unit 301 to the drive device 304 and the network I/F unit 306 to the output unit 308 are coupled via a bus 309.

The control unit 301 is an arithmetic processing device that controls the entire information processing apparatus 101. For example, the control unit 301 is a central processing unit (CPU). The main storage unit 302 is a volatile memory used as a work area of the control unit 301. The volatile memory may be, for example, a dynamic random access memory (DRAM), a static random access memory (SRAM), and the like.

The auxiliary storage unit 303 is a storage device that assists the main storage unit 302. For example, the auxiliary storage unit 303 may be a hard disk drive (HDD) or a solid state drive (SSD).

The drive device 304 is a control device that controls reading/writing of data with respect to a storage medium 305 according to the control of the control unit 301. For example, in a case where the storage medium 305 is an optical disk, the drive device 304 is an optical disk drive. In addition, in a case where the storage medium 305 is a magnetic disk, the drive device 304 is a magnetic disk drive. The storage medium 305 is a portable storage medium. In addition, the information processing apparatus 101 may incorporate a semiconductor memory formed by semiconductor elements and read and write data with respect to a Universal Serial Bus (USB) flash drive including a drive device.

The network I/F unit 306 is coupled to the network 210 such as a local area network (LAN), a wide area network (WAN), the Internet via a communication line, and is coupled to another apparatus via the network 210. The network I/F unit 306 controls the interface between the network 210 and the inside, and controls input and output of data from an external device. As the network I/F unit 306, for example, a modem, a LAN adapter, or the like may be adopted.

The input unit 307 is an input device that inputs information to the information processing apparatus 101. For example, the input unit 307 is a mouse, a keyboard, or the like. The mouse is a device that performs cursor movement, cursor range selection, window movement, window size change, and the like. The keyboard is a device that has keys for inputting letters, numbers, various instructions, and the like, and inputs data.

The output unit 308 is an input device that outputs information from the information processing apparatus 101. For example, the output unit 308 is a display or the like. The display is a device for displaying data such as a document, an image, and function information, including a cursor, an icon and a tool box. As the display, for example, a cathode ray tube (CRT), a thin film transistor (TFT) liquid crystal display, a plasma display, or the like may be adopted.

The data acquisition apparatus 201 includes a control unit 311, a main storage unit 312, an auxiliary storage unit 313, the sensor 102, and a network I/F unit 314. Further, the control unit 311 to the network I/F unit 314 are coupled via a bus 315.

The control unit 311 is an arithmetic processing device that controls the entire data acquisition apparatus 201. For example, the control unit 311 is a CPU. The main storage unit 312 is a volatile memory used as a work area of the control unit 311. The volatile memory may be, for example, a DRAM, a SRAM, and the like.

The auxiliary storage unit 313 is a storage device that assists the main storage unit 312. For example, the auxiliary storage unit 313 is an SSD or the like.

The network I/F unit 314 is coupled to the hub 203 by wireless communication or wired communication, and is coupled to another device via the hub 203 and the network 210. As the network I/F unit 314, for example, a short-range wireless communication interface or the like may be adopted.

The mobile terminal 202 includes a control unit 321, a main storage unit 322, an auxiliary storage unit 323, a network I/F unit 324, an output unit 325, and an input unit 326. The control unit 321 to the input unit 326 are coupled via a bus 327.

The control unit 321 is an arithmetic processing device that controls the entire mobile terminal 202. For example, the control unit 321 is a CPU. The main storage unit 322 is a volatile memory used as a work area of the control unit 321. The volatile memory may be, for example, a DRAM, a SRAM, and the like.

The auxiliary storage unit 323 is a storage device that assists the main storage unit 322. For example, the auxiliary storage unit 323 is an SSD or the like.

The network I/F unit 324 is coupled to the hub 203 by wireless communication or wired communication, and is coupled to another device via the hub 203 and the network 210. As the network I/F unit 324, for example, a short-range wireless communication interface or the like may be adopted.

The output unit 325 is an input device that outputs information from the information processing apparatus 101. For example, the output unit 325 is a display or the like. The display is a device for displaying data such as a document, an image, and function information, including a cursor, an icon and a tool box. As the display, for example, a TFT liquid crystal display or the like may be adopted.

The input unit 326 is an input device for inputting information to the mobile terminal. For example, the input unit 326 is a sensor or a hardware key for detecting a tap operation or a flick operation or the like. Here, the above-described sensor is arranged on the display in an overlapped manner, and a device in which the sensor and the display are combined is referred to as a touch panel. In addition, the above-described sensor is a sensor employing, for example, a resistive film method, a surface type or a projection type electrostatic capacity method, or the like.

### (Functional Configuration Example of Information Processing System 200)

FIG. 4 is an explanatory diagram illustrating a functional configuration example of the information processing system 200 in Example 1. The data acquisition apparatus 201 includes a sensor data acquisition unit 400. The mobile terminal 202 has a self-diagnosis sheet input result acquisition unit 401. The control unit 301 includes a data section defining unit 402, a situation conformance degree calculation unit 403, a habit conformance degree calculation unit 404, a temporal-change conformance degree calculation unit 405, an evaluation condition matching section specifying unit 406, an evaluation condition matching section selection unit 407, and a recovery evaluation unit 408. The control unit 301 realizes functions of the respective units by executing programs stored in the main storage unit 302 and the auxiliary storage unit 303. The processing results of each unit are stored in the main storage unit 302 or the like.

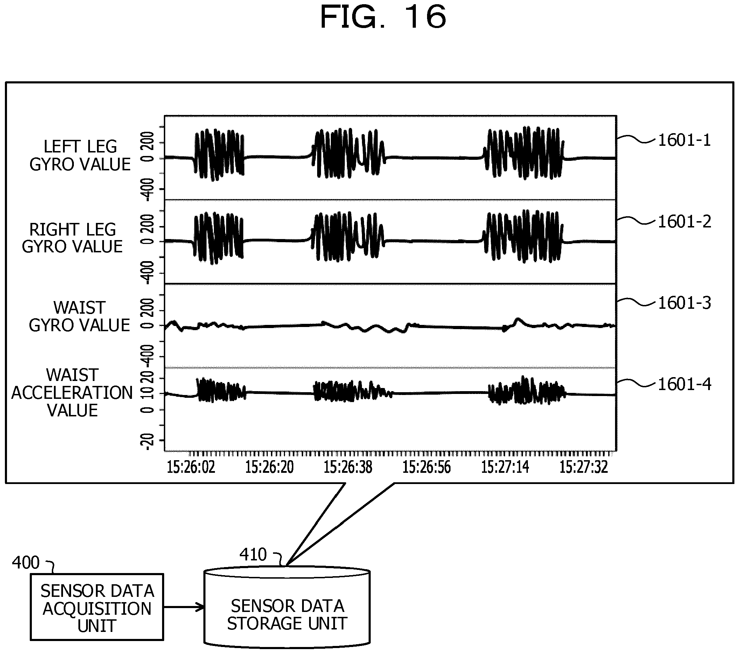

Further, the data acquisition apparatus 201 is able to access a sensor data storage unit 410. The sensor data storage unit 410 is stored in a storage area such as the main storage unit 312 and the auxiliary storage unit 313. An example of storage contents of the sensor data storage unit 410 is illustrated in FIG. 16.

Further, the mobile terminal 202 is able to access a self-diagnosis sheet input result storage unit 411. The self-diagnosis sheet input result storage unit 411 is stored in a storage area such as the main storage unit 322 and the auxiliary storage unit 323.

Further, the information processing apparatus 101 is able to access an evaluation condition matching section-and-feature quantity database 412. The evaluation condition matching section-and-feature quantity database 412 is stored in the storage areas such as the main storage unit 302 and the auxiliary storage unit 303.

The database 204 includes a healthy subject score database 413 and a patient score database 414.

The sensor data acquisition unit 400 acquires sensor data measured by the sensor 102 and accumulates the sensor data in the sensor data storage unit 410. In addition, the self-diagnosis sheet input result acquisition unit 401 accumulates the input result of the self-diagnosis sheet input by the user in the self-diagnosis sheet input result storage unit 411. Here, the self-diagnosis sheet is a diagnosis sheet to which the user himself/herself answers. For example, the self-diagnosis sheet may be Short-Form (SF)-36 (registered trademark), Sport Concussion Assessment Tool version 3 (SCAT3), and the like. For example, the mobile terminal 202 displays a response form of the self-diagnosis sheet on the touch panel. Then, the self-diagnosis sheet input result acquisition unit 401 accumulates the input result input by an operation of the user in the self-diagnosis sheet input result storage unit 411.

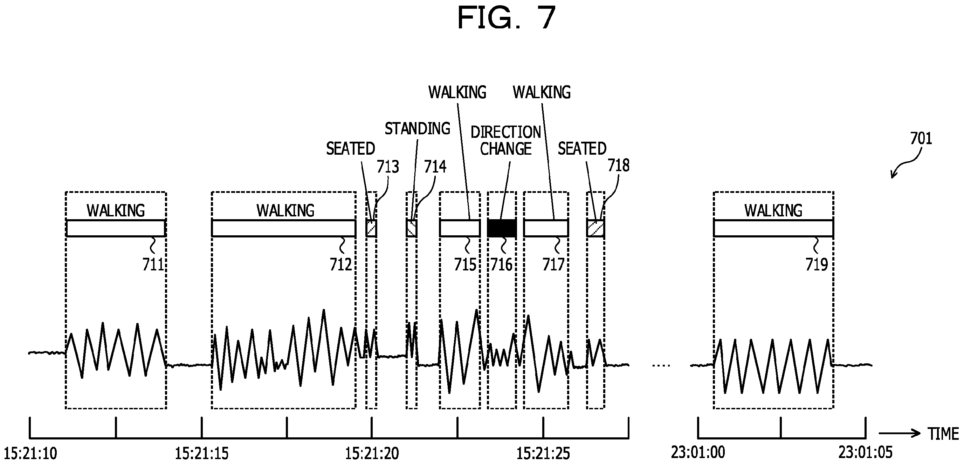

The data section defining unit 402 defines each data section in the time series data obtained from the sensor 102 as a section in which a user performs a predetermined motion or is in a predetermined state. A specific example of the data section defining unit 402 will be described with reference to FIG. 7.

In addition, the data section defining unit 402 may use a plurality of models indicating different operations or states, or the same operation or state with different parameter values. As an example of a plurality of models indicating different operations or states, there are a walking model and a stationary state model. Also, as an example of a plurality of models indicating the same operation with different parameter values, there are a high-speed walking model and a low-speed walking model in which walking speeds are different as parameters. As an example of a plurality of models indicating the same state with different parameter values, there are a short-term stationary state model and a long-term stationary state model in which the lengths of the stationary time are different as parameters. Then, the data section defining unit 402 defines each data section in the time series data as a section in which a user makes a motion of any one model or is in a state of any one model from among a plurality of models, based on the plurality of models and the time series data.

In addition, the data section defining unit 402 specifies each data section in which a user makes a motion of any one model or is in a state of any one model from among a plurality of models, from the time series data, based on the plurality of models and the time series data. Then, the data section defining unit 402 may calculate a likelihood degree indicating likelihood that the patient makes any motion or is in a state of any one model in each data section. Then, the data section defining unit 402 defines each data section in the time series data as a section in which the patient makes a motion of a model in which the calculated likelihood is equal to or greater than a predetermined threshold value, or is in any one state of a model.

Then, the control unit 301 calculates a conformance degree of each data section based on the relationship between the data section and a past data section that has data measured before data of each data section and is associated with a model, and optionally based on the relationship between data sections. Also, it is assumed that a plurality of models are used. In this case, the control unit 301 calculates a conformance degree of each data section based on the relationship between the data section and a past data section which has data measured before data of each data section and is associated with any model and optionally based on the relationship between data sections.

In the following description, an example of calculating conformance degrees of first to third scenes of the data section as a conformance degree of the data section will be described.

The situation conformance degree calculation unit 403 executes a first conformance degree calculation process of calculating a conformance degree of a first scene, which corresponds to a situation of a patient's operation or patient's state indicated by the model associated with each data section, of each data section based on the relationship between the models associated with data sections. Hereinafter, the conformance degree of the first scene is referred to as a "situation conformance degree".

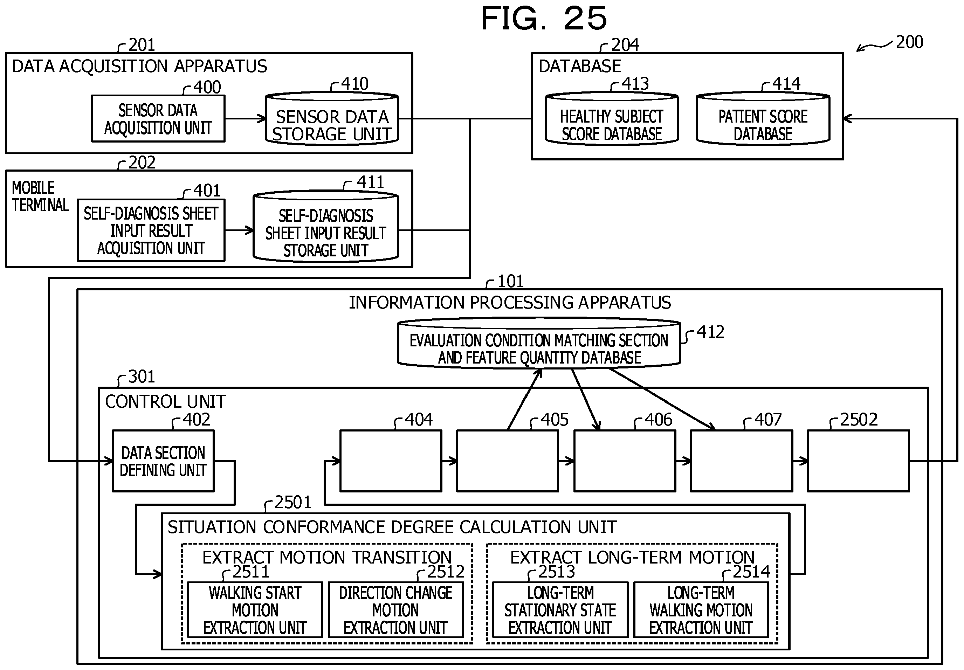

Further, the situation conformance degree calculation unit 403 may calculate a situation conformance degree of each data section depending on whether the type of operation or state of the model associated with each data section, the length of time of each data section, and the feature quantity of the data of each data section satisfy a predetermined condition. For example, as a predetermined condition, there is a case where the length of time of each data section exceeds a certain period of time or the like. The predetermined condition will be described in Example 2 described with reference to FIG. 25.

The habit conformance degree calculation unit 404 executes a second conformance degree calculation process of calculating a conformance degree of a second scene corresponding to a patient's habit of each data section based on the comparison result of the occurrence time, the occurrence location, and the feature quantity of each data section and the past data section. Hereinafter, the conformance degree of the second scene is referred to as a "habit conformance degree".

Further, the habit conformance degree calculation unit 404 calculates a habit conformance degree of each data section based on the frequencies of the occurrence time, the occurrence location, and the feature quantity of each data section on the distributions of the appearance frequencies of the occurrence time, the occurrence location, and the feature quantity of past data section.

The temporal-change conformance degree calculation unit 405 executes a third conformance degree calculation process of calculating a conformance degree of a third scene corresponding to a state change of a patient of each data section, based on a state change model based on the data of the past data section and the data of each data section. Hereinafter, the conformance degree of the third scene is referred to as a "temporal-change conformance degree".

The temporal-change conformance degree calculation unit 405 may calculate a temporal-change conformance degree of each data section based on the comparison result of the data at the same time as the occurrence time of each data section estimated from the state change model and the data of each data section.

The control unit 301 causes at least the habit conformance degree calculation unit 404 to execute its conformance degree calculation process. Then, the control unit 301 calculates the conformance degree of each data section based on the processing result of at least the second conformance degree calculation process.

The evaluation condition matching section specifying unit 406 specifies an evaluation condition matching section suitable for evaluating a recovery of a patient from data sections based on the processing result of at least one conformance degree calculation process for each data section. The evaluation condition matching section specifying unit 406 may calculate a conformance degree of the evaluation condition matching section based on the processing result of at least one conformance degree calculation process for the specified evaluation condition matching section.

The evaluation condition matching section selection unit 407 selects an evaluation condition matching section that satisfies a predetermined condition from the evaluation condition matching sections specified by the evaluation condition matching section specifying unit 406. Then, the evaluation condition matching section selection unit 407 may calculate a conformance degree of the selected evaluation condition matching section based on the processing result of at least one conformance degree calculation process for the selected evaluation condition matching section.

The recovery evaluation unit 408 calculates a recovery degree of a patient based on the data of the evaluation condition matching section specified by the evaluation condition matching section specifying unit 406. The recovery degree of a patient is hereinafter referred to as a "recovery score". Here, the recovery score may be set such that the higher value indicates the patient's recovery, or the lower value indicates the patient' recovery. In the following example, it is assumed that the recovery score is set such that the higher value indicates the patient's recovery.

Next, the recovery evaluation process executed by the sensor data acquisition unit 400 to the recovery evaluation unit 408 will be described. First, the sensor data acquisition unit 400 and the self-diagnosis sheet input result acquisition unit 401 collect data. Next, the data section defining unit 402 to the temporal-change conformance degree calculation unit 405 extract scenes from the collected data. Then, the evaluation condition matching section specifying unit 406 and the evaluation condition matching section selection unit 407 extract data used for a recovery index. Next, the recovery evaluation unit 408 compares and evaluates the extracted data. Here, the recovery evaluation unit 408 may perform comparison and evaluation using a model obtained by extracting the collected scenes, the likelihood of the model, and the feature quantity.

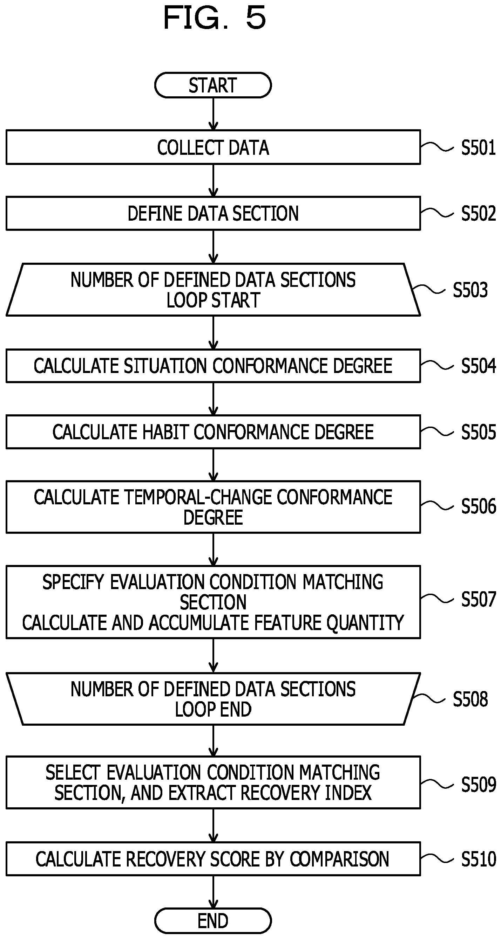

Here, the extraction of a scene will be described in more detail. The information processing apparatus 101 may use any or all of the following processes for the extraction of a scene. The first process is extraction of a scene corresponding to a sensor attachment state. The second process is extraction of a scene corresponding to an individual. The third process is extraction of a scene corresponding to a situation. The fourth process is extraction of a scene corresponding to data. The fifth process is extraction of a scene corresponding to a time change. The sixth process is extraction of a scene according to an event such as treatment or medication. Here, as the extraction of the scene according to the event, the information processing apparatus 101 may register the event in advance and extract the scene based on the registered content. Alternatively, the information processing apparatus 101 may perform determination by using a model used for the extraction of a scene corresponding to an individual as an event. Next, a specific recovery evaluation process including the extraction of the first scene to the fourth scene will be described with reference to the flowchart of FIG. 5.

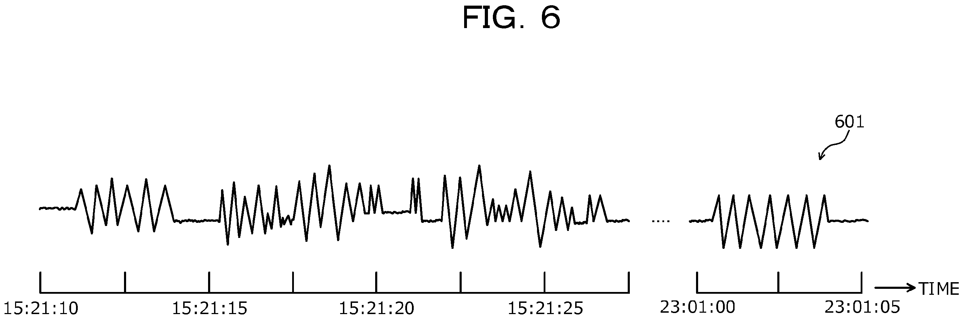

FIG. 5 is a flowchart illustrating an example of the recovery evaluation process procedure. The sensor data acquisition unit 400 and the self-diagnosis sheet input result acquisition unit 401 collect data (step S501). An example of collecting data will be described with reference to FIG. 6. Next, the data section defining unit 402 defines a data section as the extraction of the scene corresponding to the individual (step S502). The defining of the data section will be described with reference to FIG. 7. Then, the control unit 301 repeats processes of steps S503 to S508 for the number of defined data sections (steps S503 and S508).

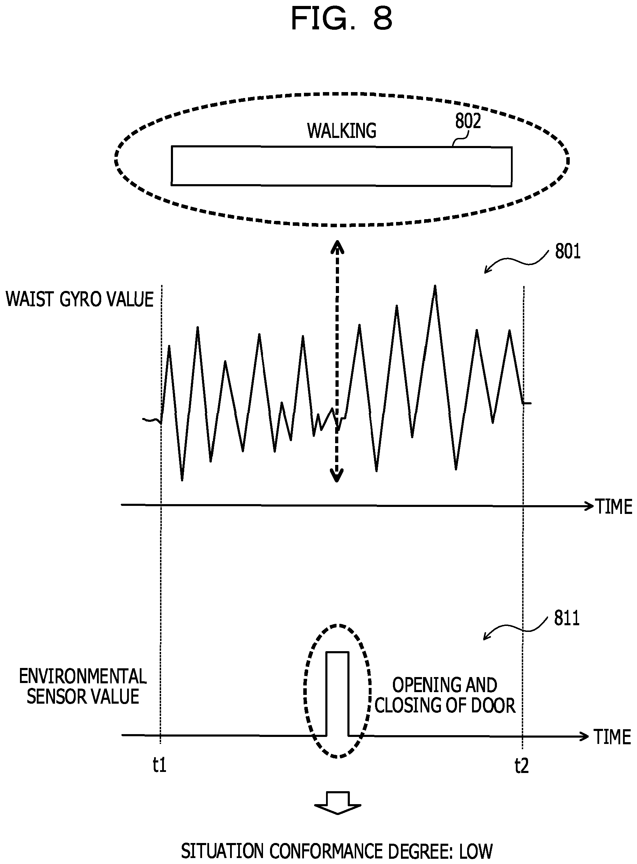

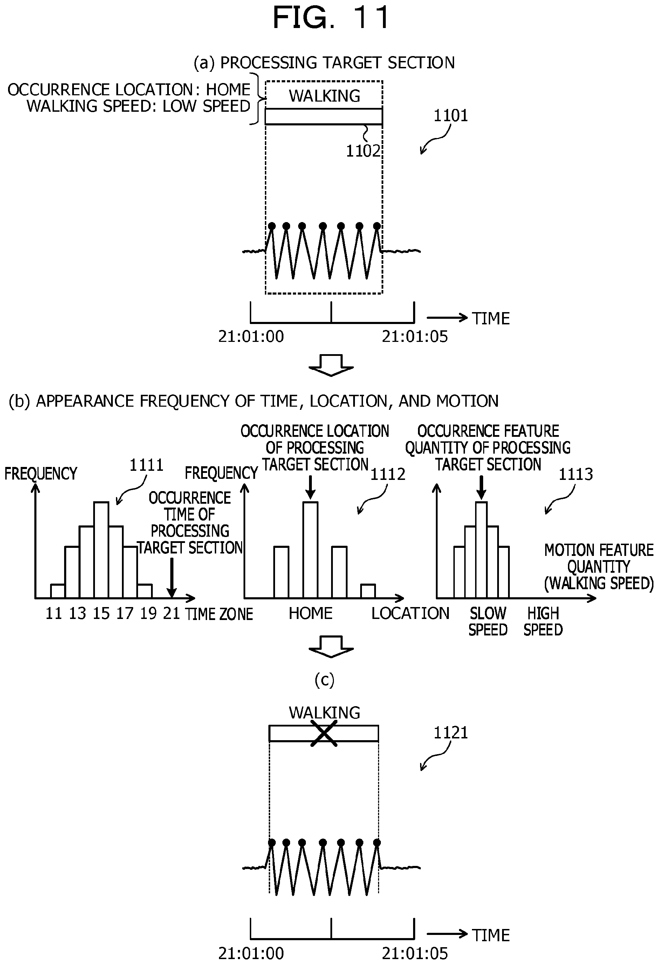

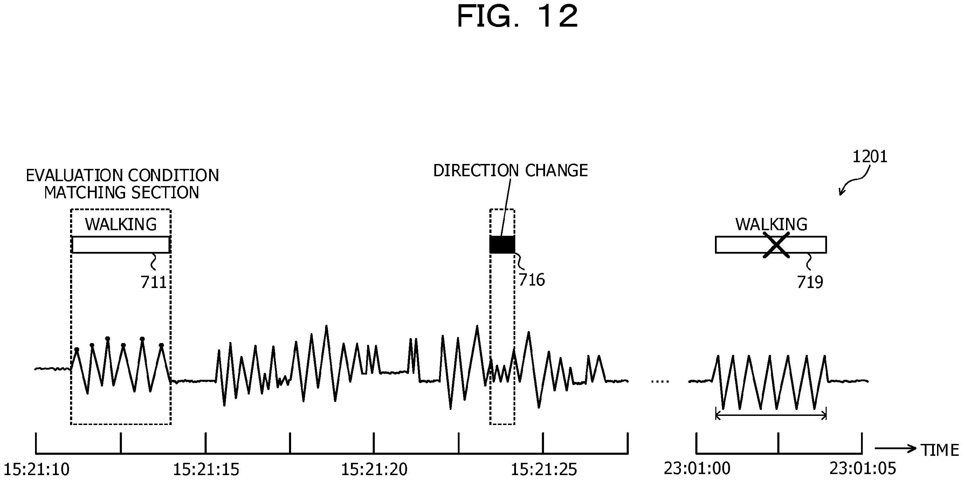

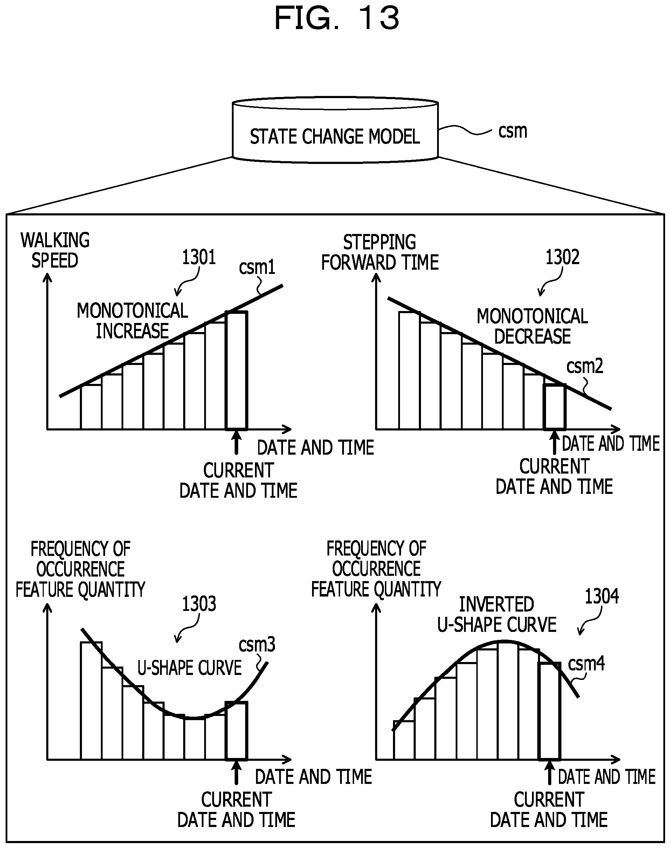

The situation conformance degree calculation unit 403 calculates a situation conformance degree of the defined data section as the extraction of the scene corresponding to the situation (step S504). The calculation of the situation conformance degree will be described with reference to FIGs. 8 to 10. In addition, the habit conformance degree calculation unit 404 calculates a habit conformance degree of the defined data section as the extraction of the scene corresponding to the data of each data section (step S505). The calculation of the habit conformance degree will be described with reference to FIGs. 11 and 12. In addition, the temporal-change conformance degree calculation unit 405 calculates a temporal-change conformance degree of the defined data section as the extraction of the scene corresponding to the temporal change (step S506). The calculation of the temporal-change conformance degree will be described with reference to FIG. 13. The situation conformance degree calculation unit 403 to the temporal-change conformance degree calculation unit 405 may perform the processes of steps S504 to S506 in the order illustrated in FIG. 5, in different orders, or in parallel.

After the processes of steps S504 to S506, the evaluation condition matching section specifying unit 406 specifies an evaluation condition matching section, and calculates and accumulates the feature quantity (step S507). Then, when the processes of S503 to S508 are repeated for the number of defined data sections, the evaluation condition matching section selection unit 407 selects an evaluation condition matching section to be used for the recovery evaluation and extracts a recovery index (step S509). Next, the recovery evaluation unit 408 calculates a recovery score by performing comparison (step S510). After step S510, the information processing apparatus 101 ends the recovery evaluation process.

FIG. 6 is an explanatory diagram illustrating an example of data collection. The sensor data acquisition unit 400 acquires data on the state of the patient and the operation of the patient. The data acquired by the sensor data acquisition unit 400 is, for example, information acquired by a sensor attached to the environment, or information on a state or operation measured by a sensor attached to the patient's body. In addition, the self-diagnosis sheet input result acquisition unit 401 acquires information described in the question paper and the self-diagnosis sheet.

Here, the sensor attached to the environment is, for example, a sensor mounted in a room such as a door, equipment, appliances, or furniture. Further, the sensor attached to the patient's body is, for example, an electrocardiogram sensor or an inertial sensor attached to a part of the body such as the chest, both legs, and the waist. The information on the state or the operation of the patient measured by the sensor is, for example, a heart rate, a value of acceleration of three axes or a value of gyro sensors of three axes at positions of both legs and the waist.

A graph 601 illustrated in FIG. 6 illustrates a waveform of a sensor value measured by a gyro sensor attached to a leg of a patient. More specifically, the graph 601 illustrates a waveform from 15:21:10 to 23:01:05 as the measurement period of time.

### (Example of Defining Data)

Next, the defining of data performed by the data section defining unit 402 will be described with reference to FIG. 7.

FIG. 7 is an explanatory diagram illustrating an example of defining data. The data section defining unit 402 performs a likelihood determination using a plurality of models indicating a predetermined situation and a predetermined state or motion, and defines a data section. Here, the model is a model of a situation and a state or an operation having features as an evaluation index of a patient's state, and for example, a model of the following operations which are noticed in a test, in which a diagnosis is performed by observing a movement of a patient, performed in a hospital, a rehabilitation facility, or the like may be used. Here, as the plurality of models, models that have the same features and different parameter values may be used. The model may be, for example, a walking model, a direction change model, a standing model, a seated model, and the like.

The model as a walking model is, for example, a model having a feature quantity of a motion for putting out one leg forward, or a model having feature quantities of periodic forward and backward rotation motions of both legs at a certain speed and a certain number of steps. In addition, when the example of the walking model is used as the model having the same features and different parameter values as described above, the data section defining unit 402 may use a low-speed walking model and a high-speed walking model. The low-speed walking model is a model in which the walking speed as one of the feature quantities of walking, is around a first threshold value. On the other hand, the high-speed walking model is a model in which the walking speed is in the vicinity of a second threshold value greater than the first threshold value.

The model as the direction change model is a model having feature quantities of forward, backward, left and right rotation motions of both legs at a certain speed and a certain number of steps.

The model as a standing model or a seated model is a model having a feature in which a movement of acceleration equal to or greater than a certain level occurs in the direction of gravity after forward and backward rotation of a waist occurs at a certain speed. More specifically, the model as a standing model or a seated model is a model having a feature quantity of forward and backward rotation motion of a waist at a certain speed and a feature quantity such as acceleration equal to or greater than a certain level in the direction of gravity.

Likelihood determination is to determine which data section in the measurement period corresponds to which model. The data section defining unit 402 uses the number of similar feature quantities as likelihood. For each model, the data section defining unit 402 scores the data section in which similar feature quantities appear for each feature quantity of the model. For example, the data section defining unit 402 adds 1 as the score when the feature quantities are similar, and defines a data section having a certain score or greater. In addition, in a case of extracting the same or overlapping data sections of a plurality of models, the data section defining unit 402 may define all the data sections or only the data section of the model having the maximum score.

Further, the information processing apparatus 101 may use a model determined based on the likelihood as a meaning of the entire defined data sections in the subsequent processes, or as a meaning of a part of the defined data section in the subsequent processes. Further, the information processing apparatus 101 may use a model determined based on the likelihood as a meaning of the data section immediately before or after the defined data section in the subsequent processes. In the following description, the entire defined data section will be described as a "defined model name + section".

A graph 701 illustrated in FIG. 7 illustrates an example in which the data section defining unit 402 defines the measurement period of the graph 601. Specifically, in the example of FIG. 7, the data section defining unit 402 defines sequentially from the beginning of the measurement period, a walking section 711, a walking section 712, a seated section 713, a standing section 714, a walking section 715, a direction change section 716, a walking section 717, a seated section 718, and a walking section 719.

### (Example of Calculating Situation Conformance Degree)

Next, the calculation of the situation conformance degree performed by the situation conformance degree calculation unit 403 will be described with reference to FIGs. 8 to 10. The situation conformance degree is a conformance degree based on the situation of the data section. For example, in a case where a certain data section is regarded as the same situation as a test in which a diagnosis is performed by observing a movement of a patient, performed in a hospital, a rehabilitation facility, or the like, the situation conformance degree calculation unit 403 calculates a conformance degree to be high. Further, for example, when the situation is the same distance, time or road surface condition as the test, or is in the state measured by the test, or when a continuous motion measured by the test is occurring, the situation conformance degree calculation unit 403 calculates a situation conformance degree to be high. In addition, when a motion in which the symptom of the target patient easily appears is occurring, or when a motion feature indicating the symptom of the target patient is found in the extracted data section, the situation conformance degree calculation unit 403 calculates a situation conformance degree to be high.

More specifically, the situation conformance degree calculation unit 403 may calculate a situation conformance degree by any one of the following three methods or by combining two or more of the three methods. The first method is a method of calculating a situation conformance degree based on the determination of the type of state or motion. For example, the situation conformance degree calculation unit 403 may calculate a situation conformance degree based on the type of model used in the example of defining data. More specifically, the situation conformance degree calculation unit 403 may set a situation conformance degree of each type of state or motion in advance, or may calculate, as a situation conformance degree, the score determined by the data section defining unit 402 itself, or the score of the data section having likelihood equal to or greater than a certain level.

The second method is a method of calculating a situation conformance degree based on the determination of the feature quantity of the state or the motion. For example, the situation conformance degree calculation unit 403 may calculate a situation conformance degree based on the feature quantity of the model used in the example of defining data. Further, for example, the situation conformance degree calculation unit 403 may set a situation conformance degree of each feature quantity in advance, or may calculate, as a situation conformance degree, the feature quantity obtained by the data section defining unit 402 or the feature quantity of the data section having likelihood equal to or greater than a certain level. For example, the situation conformance degree calculation unit 403 sets a situation conformance degree of a data section having a certain walking speed or greater and a certain walking period of time, as a walking speed in the data section defined as a walking model.

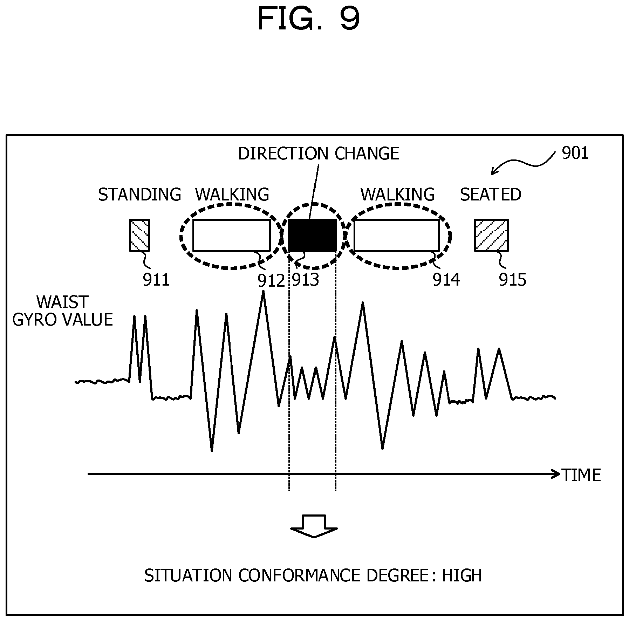

The third method is a method of calculating a situation conformance degree based on the determination of the execution state of the state or the motion. For example, the situation conformance degree calculation unit 403 may calculate the situation conformance degree based on a combination of models of the defined data sections. Further, for example, the situation conformance degree calculation unit 403 may set the situation conformance degree in advance for each combination of predetermined models, or may calculate the situation conformance degree according to the appearance frequency or the ratio of the combination. For example, an example of calculating the situation conformance degree of each combination of models will be described with reference to FIGs. 8 and 9.

FIG. 8 is an explanatory diagram (part 1) illustrating an example of calculating a situation conformance degree. Further, FIG. 9 is an explanatory diagram (part 2) illustrating an example of calculating the situation conformance degree. A graph 801 illustrated in FIG. 8 illustrates a waist gyro value measured by a gyro sensor attached to the patient's waist. Then, it is assumed that the data section defining unit 402 defines a data section 802 from time t1 to time t2 as a walking section. A graph 811 illustrated in FIG. 8 illustrates a value of a sensor attached to a door as environmental sensor values from time t1 to time t2 as the same time zone. The graph 811 illustrates that the door to which the sensor is attached is opened and closed.

Therefore, in FIG. 8, since the patient walks while opening and closing the door, the motion is different from the pure walking motion, and thus the situation conformance degree calculation unit 403 calculates the situation conformance degree to be low. For example, in the case of FIG. 8, the situation conformance degree calculation unit 403 may set the situation conformance degree to the minimum value, or may set a value obtained by subtracting a fixed value from a value calculated from the first method or the second method in the example of calculating the situation conformance degree, as the situation conformance degree.

Further, a graph 901 illustrated in FIG. 9 illustrates a waist gyro value measured by a gyro sensor attached to the patient's waist. In addition, the doctor treating the patient wishes to obtain the direction change motion of when the patient walks, changes the direction, and then walks, as a quantitative evaluation. It is assumed that the data section defining unit 402 defines sequentially from the beginning of the measurement period of the graph 901, a standing section 911, a walking section 912, a direction change section 913, a walking section 914, and a seated section 915.

In this case, since the data section defined by the data section defining unit 402 matches a condition that the patient walks, changes the direction, and then walks, the situation conformance degree calculation unit 403 calculates the situation conformance degree to be high. For example, in the case of FIG. 9, the situation conformance degree calculation unit 403 may set the situation conformance degree to the maximum value, or may set a value obtained by adding a fixed value to a value calculated from the first method or the second method in the example of calculating the situation conformance degree, as the situation conformance degree.

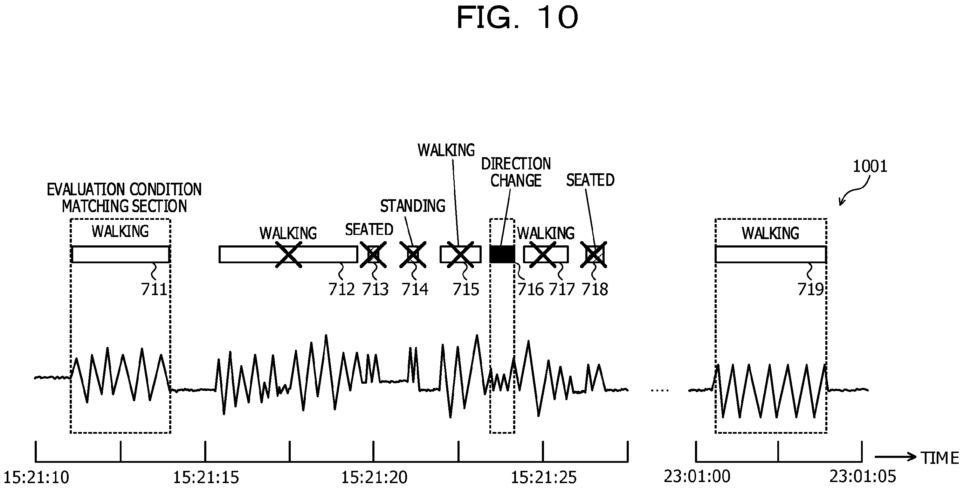

In addition, as described above, the first to third methods may be combined. Further, the situation conformance degree calculation unit 403 may determine a section in which the situation conformance degree is equal to or greater than a certain value as the evaluation condition matching section. Next, an example in which any of the walking section 711 to the walking section 719 defined in the graph 701 is determined as the evaluation condition matching section will be described with reference to FIG. 10.

FIG. 10 is an explanatory diagram (part 3) illustrating an example of calculating the situation conformance degree. A graph 1001 illustrated in FIG. 10 illustrates that among the walking section 711 to the walking section 719, the data section determined as the evaluation condition matching section by the situation conformance degree calculation unit 403 remains as it is, and a cross mark is given for the section which is not determined as the evaluation condition matching section. Specifically, in the example of FIG. 10, the situation conformance degree calculation unit 403 specifies the walking section 711, the direction change section 716, and the walking section 719 as the evaluation condition matching section. On the other hand, since the situation conformance degree is low, the situation conformance degree calculation unit 403 determines that the walking section 711 to the walking section 715, the walking section 717, and the seated section 718 are not the evaluation condition matching section.

### (Example of Calculating Habit Conformance Degree)

Next, the calculation of the habit conformance degree performed by the habit conformance degree calculation unit 404 will be described with reference to FIGs. 11 and 12. The habit conformance degree is a conformance degree based on a patient's habit. More specifically, the habit conformance degree is a degree indicating how much the data section of interest is the same as or different from "usual" of the patient.

Here, as to how much the data section is the same as or different from "usual", the habit conformance degree calculation unit 404 performs estimation by comparing the motion, the feature quantity, the time, and the location of the data section of interest with the distribution of the past appearance frequencies thereof. For example, in a case where the feature quantity of the data section of interest is positioned at the average value or the median value of the distribution of the past appearance frequencies, the habit conformance degree calculation unit 404 sets a high value as the habit conformance degree, and in a case where the feature quantity of the data section of interest is not positioned at the average value or the median value of the distribution of the past appearance frequencies, the habit conformance degree calculation unit 404 sets a low value as the habit conformance degree. Further, the habit conformance degree calculation unit 404 calculates the habit conformance degree based on the position of each of the time, the location, and the motion feature quantity on the distributions thereof. Here, the information processing apparatus 101 may acquire the occurrence time and location by using the time of a timer of the data acquisition apparatus 201 or another sensor such as a Global Positioning System (GPS).

Further, the habit conformance degree calculation unit 404 may use the calculated habit conformance degree for specifying or evaluating the data section. Further, the habit conformance degree calculation unit 404 may determine the data section in which the habit conformance degree is equal to or greater than a predetermined value as the evaluation condition matching section. Next, an example of calculating the habit conformance degree will be described with reference to FIG. 11.

FIG. 11 is an explanatory diagram (part 1) illustrating an example of calculating a habit conformance degree. A graph 1101 illustrated by (a) in FIG. 11 illustrates a walking section 1102 defined by the data section defining unit 402 as a processing target section for calculating the habit conformance degree. The occurrence time of the walking section 1102 is between 21:01:00 and 21:01:05. Further, it is assumed that the occurrence location of the walking section 1102 is the patient's house and the walking speed is low.

The habit conformance degree calculation unit 404 specifies which position the walking section 1102 belongs to on the respective distributions of the appearance frequencies of the time, the location, and the motion feature quantity, and then determines whether the walking section 1102 is the same motion as usual or a different motion. A graph 1111 illustrated by (b) in FIG. 11 illustrates the occurrence frequency of a past walking section for a time zone. As illustrated by the graph 1111, it is seen that the target patient is not walking usually in the occurrence time of the walking section 1102 which is a processing target section. Therefore, the habit conformance degree calculation unit 404 determines that the walking section 1102 is a motion different from usual for the time zone.

Similarly, a graph 1112 illustrated by (b) in FIG. 11 illustrates the occurrence frequency of a past walking section for a location. As illustrated in the graph 1112, it is seen that the target patient is walking usually at the occurrence location of the walking section 1102, which is a processing target section. Therefore, the habit conformance degree calculation unit 404 determines that the walking section 1102 is a usual motion for the location.

Similarly, a graph 1113 illustrated by (b) in FIG. 11 illustrates the frequency of a past walking section for the walking speed which is the motion feature quantity. As illustrated by the graph 1113, it is seen that the target patient is usually walking at the walking speed of the walking section 1102 which is a processing target section. Therefore, the habit conformance degree calculation unit 404 determines that the walking section 1102 is a normal motion for the motion feature quantity. Here, there may be a plurality of motion feature quantities of the motion. In this case, the habit conformance degree calculation unit 404 may determine whether all the motion feature quantities of the motion are the same as usual, or determine whether some motion feature quantities of all the motion feature quantities of the motion are the same as usual.

Since the walking section 1102 is determined to be a motion different from usual for the time zone, as illustrated by a graph 1121 of (c) in FIG. 11, the habit conformance degree calculation unit 404 determines that the walking section 1102 is a motion different from usual. In the graph 1121, the habit conformance degree calculation unit 404 determines that the processing target section is the same motion as usual when all the time, the location, and the motion feature quantity are the same as usual, but it is not limited thereto. For example, the habit conformance degree calculation unit 404 may determine that the processing target section is the same motion as usual when the majority of the time, the location, and the motion feature quantity is the same as usual. Next, an example of further narrowing down the data sections determined as the evaluation condition matching section by the situation conformance degree calculation unit 403 as illustrated by the graph 1001 will be described with reference to FIG. 12.

FIG. 12 is an explanatory diagram (part 2) illustrating an example of calculating the habit conformance degree. A graph 1201 illustrated in FIG. 12 illustrates that the data section determined as the evaluation condition matching section by the habit conformance degree calculation unit 404 remains as it is, and a cross mark is given for the section which is not determined as the evaluation condition matching section. Specifically, in the example of FIG. 12, the habit conformance degree calculation unit 404 specifies the walking section 711 and the direction change section 716 as the evaluation condition matching section. On the other hand, since the situation conformance degree is low, the habit conformance degree calculation unit 404 determines that the walking section 719 is not the evaluation condition matching section.

### (Example of Calculating Temporal-Change Conformance Degree)

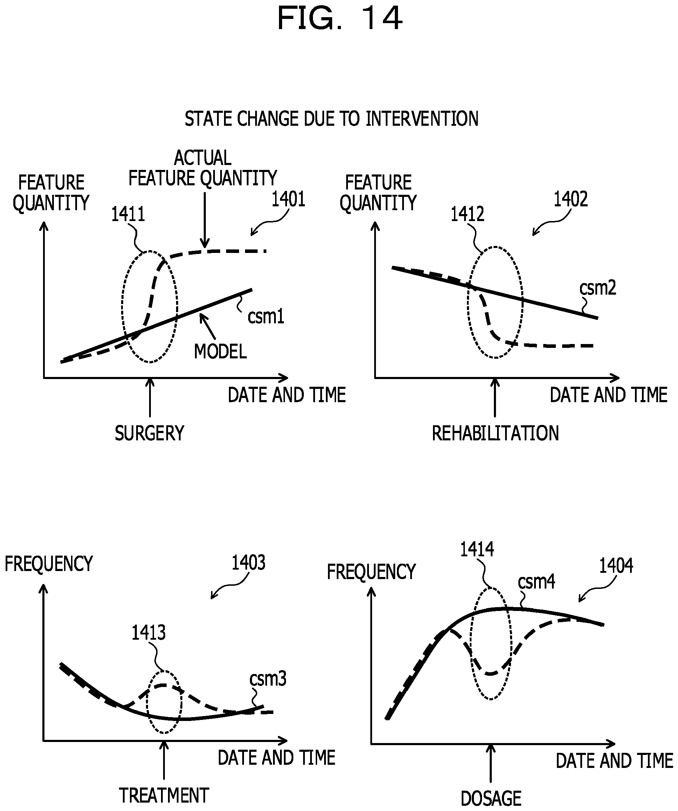

Next, the calculation of the temporal-change conformance degree performed by the temporal-change conformance degree calculation unit 405 will be described with reference to FIGs. 13 and 14. The temporal-change conformance degree is a conformance degree based on the temporal change of the motion of the patient. More specifically, the temporal-change conformance degree is a degree indicating whether the type, the feature quantity, or the execution state of the state or the motion of the data section of interest is suitable as the state change of the target patient.

Here, as to whether it is suitable as the state change, for example, the temporal-change conformance degree calculation unit 405 performs estimation based on the current distribution predicted from the past appearance frequency distribution, or based on the likelihood of the model indicating the state change such as a recovery or a deterioration. The model indicating the state change such as recovery or a deterioration is, for example, a model generated by focusing on the following concept. Specifically, the model indicating the state change will be described with reference to FIG. 25.

FIG. 13 is an explanatory diagram illustrating an example of a model indicating a state change. A state change model csm illustrated in FIG. 13 includes a recovery model csm1 indicating a recovery state change, a deterioration model csm2 indicating a deterioration state change, and rebound models csm3 and csm4 indicating a rebound.

The recovery model csm1 is a model generated by focusing on the fact that the feature quantity of the state or the motion, or the frequency of the feature quantity thereof monotonically increases from the idea that "the state change by a recovery is basically a gradual change indicating gradual improvement". A graph 1301 illustrated in FIG. 13 illustrates a change of a walking speed with respect to the date and time as an example of the recovery model csm1.

The deterioration model csm2 is a model generated by focusing on the fact that the feature quantity, or the frequency of the feature quantity monotonically decreases from the idea that "the state change by a deterioration is basically a gradual change indicating gradual worsening". A graph 1302 illustrated in FIG. 13 illustrates a change of a stepping-forward time with respect to the date and time as an example of the deterioration model csm2.

The rebound models csm3 and csm4 are models generated by focusing on the fact that the feature quantity, or the frequency of the feature quantity illustrates a U-shape or an inverted U-shape from the idea that "during the state change by a recovery or a deterioration, there may be a rebound depending on the unstable period of time or the diseases". Graphs 1303 and 1304 illustrated in FIG. 13 illustrate changes of an occurrence feature quantity with respect to the date and time as an example of rebound models csm3 and csm4, respectively. As illustrated in FIG. 13, the rebound model csm3 is a model that draws a U-shape, and the rebound model csm4 is a model that draws an inverted U-shape.

Then, for example, when the feature quantity of the state or the motion of the processing target section is similar to the current feature quantity estimated from the model as the likelihood of the model, the temporal-change conformance degree calculation unit 405 sets the temporal-change conformance degree to a high value. In addition, the temporal-change conformance degree calculation unit 405 may determine the section in which the temporal-change conformance degree is equal to or greater than a certain value as the evaluation condition matching section. The temporal-change conformance degree calculation unit 405 may further narrow down the data sections determined as the evaluation condition matching section by the situation conformance degree calculation unit 403, or the data sections determined as the evaluation condition matching section by the habit conformance degree calculation unit 404.

### (Specific Example of Evaluation Condition Matching Section)

Next, the specifying of the evaluation condition matching section performed by the evaluation condition matching section specifying unit 406 will be described. The evaluation condition matching section specifying unit 406 specifies the evaluation condition matching section based on the situation conformance degree, the habit conformance degree, and the temporal-change conformance degree calculated by the situation conformance degree calculation unit 403 to the temporal-change conformance degree calculation unit 405. Further, the evaluation condition matching section specifying unit 406 may specify the data section that is determined to be the evaluation condition matching section by the situation conformance degree calculation unit 403 to the temporal-change conformance degree calculation unit 405, as the evaluation condition matching section.

Further, the evaluation condition matching section specifying unit 406 calculates and accumulates the motion feature quantity of the all or a part of the specified evaluation condition matching sections, or the motion feature quantity of the data section immediately before or immediately after the specified evaluation condition matching section. Here, for example, the motion feature quantity may be a motion feature quantity calculated from the data waveform of the specified evaluation condition matching section, a feature quantity of the model used in the data section defining unit 402, a situation conformance degree, a habit conformance degree, a temporal-change conformance degree, or the like.

### (Example of Selecting Evaluation Condition Matching Section)

Next, the selection of the evaluation condition matching section performed by the evaluation condition matching section selection unit 407 will be described. Here, the evaluation condition matching section selection unit 407 may accept the operation of the doctor in advance or afterward by the doctor viewing, for example, the situation conformance degree, the habit conformance degree, and the temporal-change conformance degree, and select the evaluation condition matching section based on the acceptance result. Alternatively, the evaluation condition matching section selection unit 407 may select the evaluation condition matching section based on the situation conformance degree, the habit conformance degree, and the time variation conformance degree. Alternatively, the evaluation condition matching section selection unit 407 may perform selection based on the past data or a predicted value of the future of the target patient.

In addition, the evaluation condition matching section selection unit 407 may select, as the evaluation condition matching section, only the section in which all or any of the situation conformance degree, the habit conformance degree, and the temporal-change conformance degree is equal to or greater than a certain value. Alternatively, the evaluation condition matching section selection unit 407 may select, as the evaluation condition matching section, only the section in which the likelihood of the model, or the position or the distance of the appearance frequency distribution used in the data section defining unit 402 to the temporal-change conformance degree calculation unit 405 is equal to or greater than or equal to or less than a certain value.

### (Example of Evaluating Recovery)

Next, the calculation of the recovery score performed by the recovery evaluation unit 408 will be described. The recovery evaluation unit 408 calculates a recovery score of the evaluation condition matching section selected by the evaluation condition matching section selection unit 407 based on the processing result of the data section defining unit 402 to the temporal-change conformance degree calculation unit 405.

In addition, the recovery evaluation unit 408 may cause the data section defining unit 402 to temporal-change conformance degree calculation unit 405 to execute the processing again for the selected evaluation condition matching section. Then, the recovery evaluation unit 408 calculates the recovery score by comparing the likelihood of the model, the position or the distance of the appearance frequency distribution, the situation conformance degree, the habit conformance degree, and the temporal-change conformance degree calculated as a result of the execution, or the feature quantity thereof with a reference feature quantity.

Here, the reference feature quantity may be, for example, a feature quantity of a healthy subject, a feature quantity of another person, a feature quantity in the same situation, a past feature quantity of the target patient, a prediction value as a future feature quantity, and a feature quantity illustrated in a paper. In addition, a plurality of reference feature quantities may be used, or a temporal change of a certain feature quantity may be used.

In addition, the recovery evaluation unit 408 may perform comparison with the reference feature quantity for each selected evaluation condition matching section, and calculate a recovery score by adding or subtracting an evaluation score when a predetermined condition is satisfied. Here, the predetermined condition may be, for example, that the feature quantity of the evaluation condition matching section is the same as, greater than, or less than the reference feature quantity, or becomes a multiple of the reference feature quantity.

### (Additional Example of Temporal-Change Conformance Degree Calculation Process)

Next, an additional example of the temporal-change conformance degree calculation unit 405 will be described with reference to FIG. 14. When there is intervention such as surgery, medication, or rehabilitation, the temporal-change conformance degree calculation unit 405 may re-learn the model before and after the intervention, or the temporal-change conformance degree calculation unit 405 may perform calculation such that the temporal-change conformance degree is not to be low even when the likelihood decreases due to a sudden change. Here, as to whether there is intervention, when the user knows the intervention in advance, the temporal-change conformance degree calculation unit 405 may determine the presence or absence of intervention based on information that there is intervention, which is input by the user. Alternatively, the temporal-change conformance degree calculation unit 405 may detect a change point indicating a sudden state change, and determine the presence or absence of intervention based on the detected change point. Next, an example of a state change due to intervention will be described with reference to FIG. 14.

FIG. 14 is an explanatory diagram illustrating an example of a temporal change due to intervention. Graphs 1401 to 1404 illustrated in FIG. 14 illustrate comparative examples with the recovery model csm1 to the rebound model csm4, respectively. Further, in the graphs 1401 to 1404, the solid line indicates the state change model, the broken line indicates the feature quantity of the processing target section or the frequency of the feature quantity, and ranges 1411 to 1414 enclosed by the dotted line indicate a range where there is intervention. In the graphs 1401 to 1404, there are interventions of surgery, rehabilitation, treatment, and medication, respectively, and there are deviations from the recovery model csm1 to the rebound model csm4.

In the case of changes as illustrated by the graphs 1401 to 1404, the temporal-change conformance degree calculation unit 405 regards such changes as the state change due to intervention, and thus re-learn the state change model in the calculation of the temporal-change conformance degree or performs calculation such that the temporal-change conformance degree is not to be low.

### (Specific Example of Example 1)

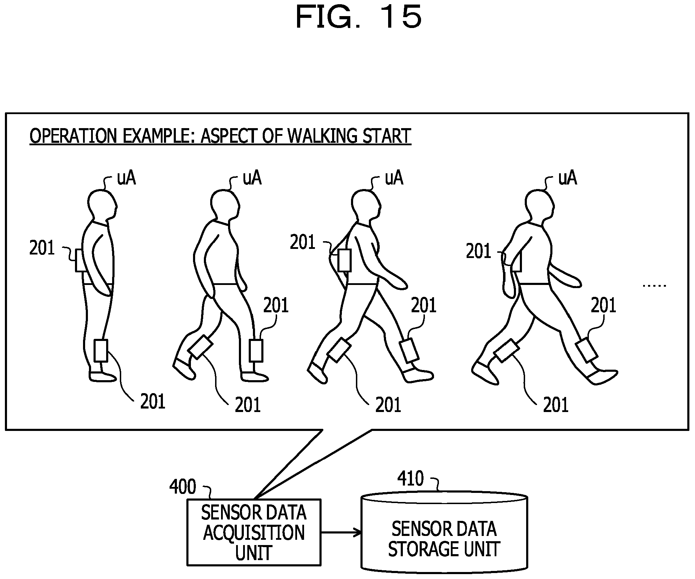

Next, as a specific example of Example 1, an example of performing a recovery evaluation for a patient uA who is a concussion patient will be described with reference to FIGs. 15 to 24.

FIG. 15 is an explanatory diagram illustrating an example of the state of walking of the patient uA in Example 1. In Example 1, it is assumed that the data acquisition apparatus 201 is attached to each of the right and left legs and the back of the waist of the user uA. FIG. 15 illustrates how the patient uA starts walking in daily life. The sensor data acquisition unit 400 of the data acquisition apparatus 201 attached to each part of the patient uA measures the movement of each part and stores the measured sensor waveform in the sensor data storage unit 410.

FIG. 16 is an explanatory diagram illustrating an example of a sensor waveform at the time of walking of the patient uA in Example 1. In FIG. 16, one gyro value of each part is illustrated. Specifically, sensor data 1601-1 to sensor data 1601-4 respectively indicate sensor data of a left leg gyro value, a right leg gyro value, a waist gyro value, and a waist acceleration value, for each time. Here, the sensor 102 of the data acquisition apparatus 201 measures acceleration in three axial directions and a gyro value, but for simplification of the display, FIG. 16 displays only the forehead axis rotation direction for the gyro value, and displays only the vertical direction for the acceleration. The following description is assumed to be the same unless otherwise specified. The forehead axis is an axis in the right and left direction in a human body. The information processing apparatus 101 acquires the sensor data of each part from the sensor data storage unit 410.

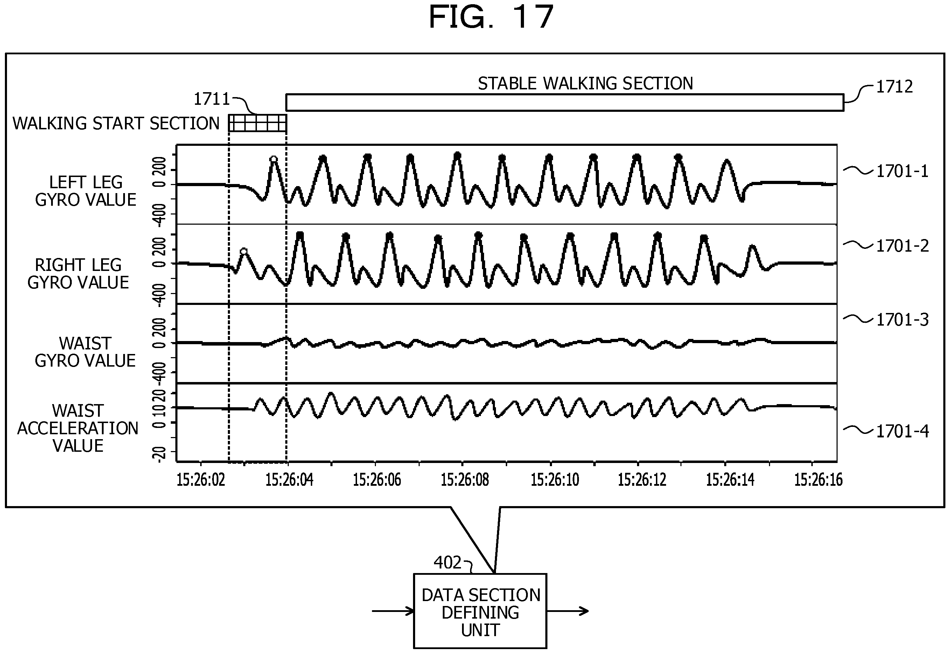

FIG. 17 is an explanatory diagram (part 1) illustrating an example of defining the data section in Example 1. The data section defining unit 402 determines the likelihood using a plurality of models indicating a specific situation and a specific state or motion, and defines the data section. In the example of FIG. 17, the data section defining unit 402 defines a walking start section 1711 and a stable walking section 1712 for the sensor data 1701-1 to the sensor data 1701-4 acquired from the sensor data storage unit 410. In the example of FIG. 17, the walking start section 1711 is indicated by a rectangle with grid shading and the stable walking section 1712 is indicated by a hollow rectangle.

Here, the walking start section is a section defined by the walking start model. Then, the walking start model is present before the stable walking section and has features similar to the feature quantities of the stable walking section. In addition, the stable walking section is a section defined by the stable walking model. The stable walking model has a condition that the feature is that the peak values of the gyro values of the right and left legs are equally spaced.

In addition, the data section defining unit 402 adds the likelihood score when the feature of the walking start model is satisfied. Then, when the added total value is equal to or greater than the threshold value, the data section defining unit 402 defines the corresponding section as a walking start section.

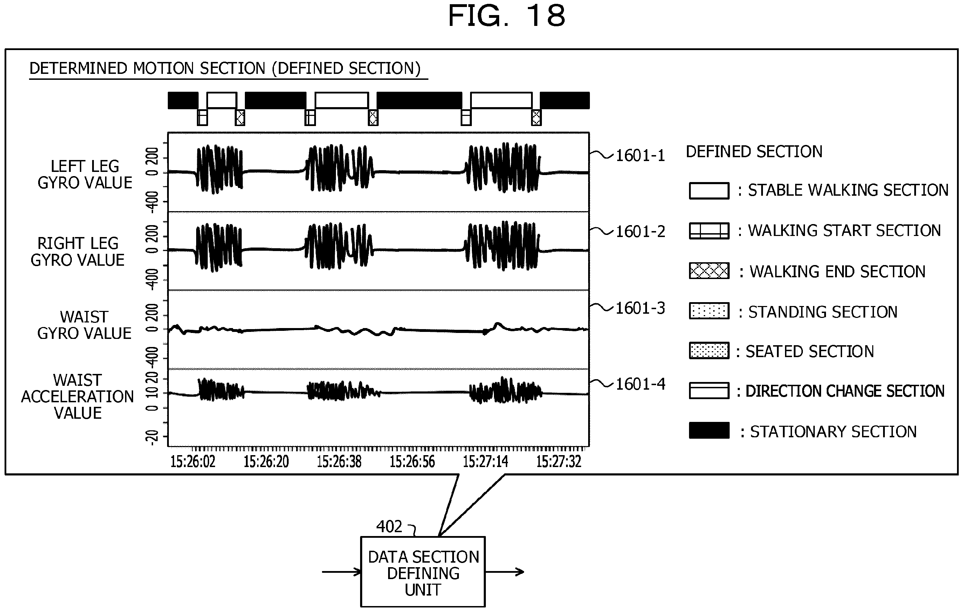

FIG. 18 is an explanatory diagram (part 2) illustrating an example of defining the data section in Example 1. FIG. 18 illustrates a defining result of the sensor data 1601-1 to the sensor data 1601-4.

In FIG. 18 and the following drawings, regarding the defined sections, the stable walking section is indicated by a hollow rectangle. Also, the walking start section is indicated by a rectangle with grid shading. Also, a walking end section is indicated by a rectangle with oblique grid shading. Also, the standing section is indicated by a rectangle with pale shading. Also, the seated section is indicated by a rectangle with dark shading. Also, the direction change section is indicated by a rectangle with shading of the vertical line. Also, the stationary section is indicated by a filled rectangle.

FIG. 18 illustrates an example of a defining result of the sensor data 1601-1 to sensor data 1601-4 by the data section defining unit 402.

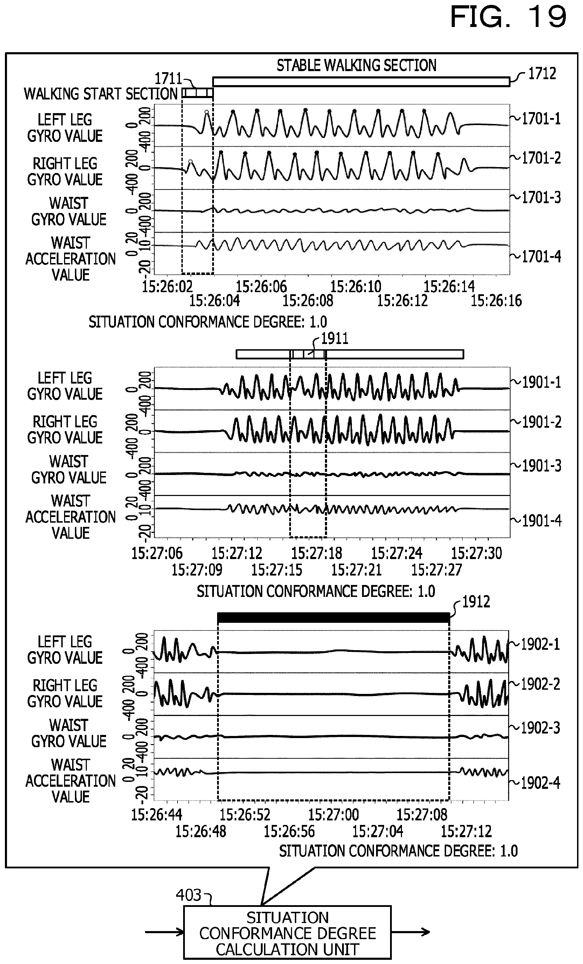

FIG. 19 is an explanatory diagram illustrating an example of calculating the situation conformance degree in Example 1. The situation conformance degree calculation unit 403 calculates the situation conformance degree of the data section defined by the data section defining unit 402. In the example of FIG. 19, for data sections, the situation conformance degree calculation unit 403 sets the initial value of the situation conformance degree of each data section to 0, and calculates the situation conformance degree by adding 1.0 each time the following condition is satisfied.

The first condition is a case where the processing target data section is a walking start data section. The second condition is a case where the processing target data section is a direction change data section before and after walking. The third condition is a case where the processing target data section is a stable walking section or a stationary section having a section length of a certain level or greater. The certain level or greater may be any time length, for example, 20 seconds or longer.

Since the walking start section 1711 of the sensor data 1701-1 to the sensor data 1701-4 illustrated in the upper part of FIG. 19 satisfies the above-described first condition, the situation conformance degree calculation unit 403 calculates the situation conformance degree of the walking start section 1711 as 1.0.

Further, since a direction change section 1911 of sensor data 1901-1 to sensor data 1901-4 illustrated in the middle part of FIG. 19 satisfies the above-described second condition, the situation conformance degree calculation unit 403 calculates the situation conformance degree of the direction change section 1911 as 1.0.

Further, since a stationary section 1912 of sensor data 1902-1 to sensor data 1902-4 illustrated in the lower part of FIG. 19 satisfies the above-described third condition, the situation conformance degree calculation unit 403 calculates the situation conformance degree of the stationary section 1912 as 1.0.

Then, the situation conformance degree calculation unit 403 determines the data section of which the situation conformance degree is 1.0 or greater, as the evaluation condition matching section.

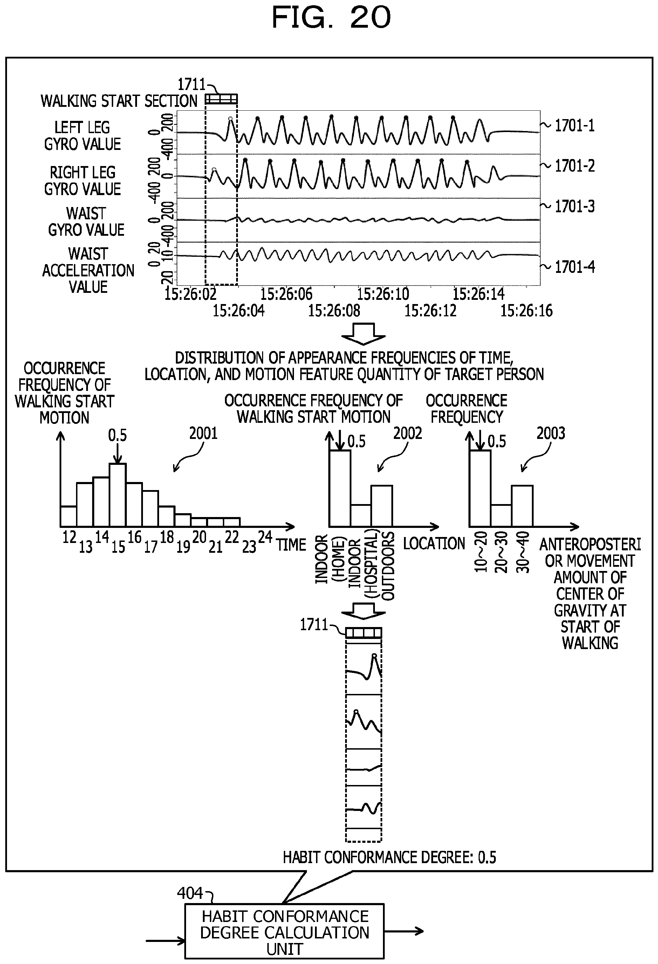

FIG. 20 is an explanatory diagram illustrating an example of calculating the habit conformance degree in Example 1. The habit conformance degree calculation unit 404 calculates the habit conformance degree of the data section which is determined as the evaluation condition matching section by the situation conformance degree calculation unit 403, based on the appearance frequencies of the time, the location, and the motion. In the example of FIG. 20, the habit conformance degree calculation unit 404 calculates the habit conformance degree of the walking start section 1711.

Here, as the feature quantity of the walking start section 1711, the occurrence time of the walking start section 1711 is 2016/07/19 15:26:02. The occurrence location of the walking start section 1711 is within the patient's house. Here, the habit conformance degree calculation unit 404 estimates the occurrence location of the walking start section 1711 from GPS data or the like. In addition, as the motion feature quantity of the walking start section 1711, the forward and backward movement amount of the center of gravity at the start of walking is 19.7 [mm]. Here, the habit conformance degree calculation unit 404 calculates the forward and backward movement amount of the center of gravity based on the waist acceleration value of the walking start section 1711.

The habit conformance degree calculation unit 404 specifies which position the walking start section 1711 belongs to on the respective distributions of the appearance frequencies of the time, the location, and the motion feature quantity, and then determines whether the walking start section 1711 is the same motion as usual or a different motion. A graph 2001 illustrated in FIG. 20 illustrates the past occurrence frequency of the walking start motion with respect to the time. Also, a graph 2002 illustrated in FIG. 20 illustrates the past occurrence frequency of the walking start motion with respect to the location. Further, a graph 2003 illustrated in FIG. 20 illustrates the past frequency of the feature quantity of the walking start section with respect to the motion feature quantity. As illustrated by the graphs 2001 to 2003, the frequencies of the time, the location, and the motion feature quantity of the walking start section 1711 are all 0.5.

In the example of FIG. 20, the habit conformance degree calculation unit 404 calculates the average value of the habit conformance degrees evaluated using the distribution of each appearance frequency, as the habit conformance degree of the processing target data section. Therefore, in the example of FIG. 20, the habit conformance degree calculation unit 404 calculates the habit conformance degree of the walking start section 1711 as 0.5. In addition, the situation conformance degree calculation unit 403 determines the data section, of which the habit conformance degree is equal to or greater than 0.4 as the certain value or greater, as the evaluation condition matching section.

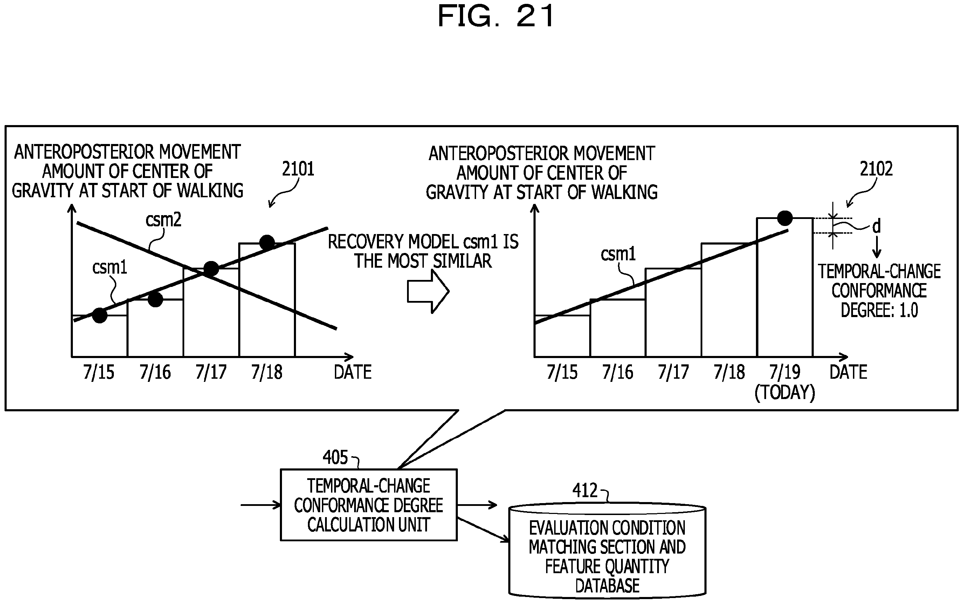

FIG. 21 is an explanatory diagram illustrating an example of calculating the temporal-change conformance degree in Example 1. The temporal-change conformance degree calculation unit 405 calculates the temporal-change conformance degree for the data section, which is determined as the evaluation condition matching section by the habit conformance degree calculation unit 404, based on a model in which the temporal change of the state of the target person is taken into consideration.

As illustrated by a graph 2101 in FIG. 21, the temporal-change conformance degree calculation unit 405 compares the recovery model csm1 and the deterioration model csm2 with the past temporal change of the target patient, respectively, and specifies the most similar state change model csm. As indicated by the graph 2101, the temporal-change conformance degree calculation unit 405 specifies that the recovery model csm1 is most similar to the temporal change.

Then, the temporal-change conformance degree calculation unit 405 sets the initial value of the temporal-change conformance degree of the processing target data section to 0. Next, the temporal-change conformance degree calculation unit 405 calculates the temporal-change conformance degree by adding 1.0 when a difference between the feature quantity of the processing target data section and the feature quantity estimated by the state change model csm that is specified to be most similar is within a predetermined threshold value.

As illustrated by a graph 2102 in FIG. 21, the temporal-change conformance degree calculation unit 405 determines that a difference d between the feature quantity of the walking start section 1711 and the feature quantity estimated by the state change model csm1 that is specified to be most similar is within a predetermined threshold value. Therefore, the temporal-change conformance degree calculation unit 405 calculates the temporal-change conformance degree of the walking start section 1711 as 1.0.

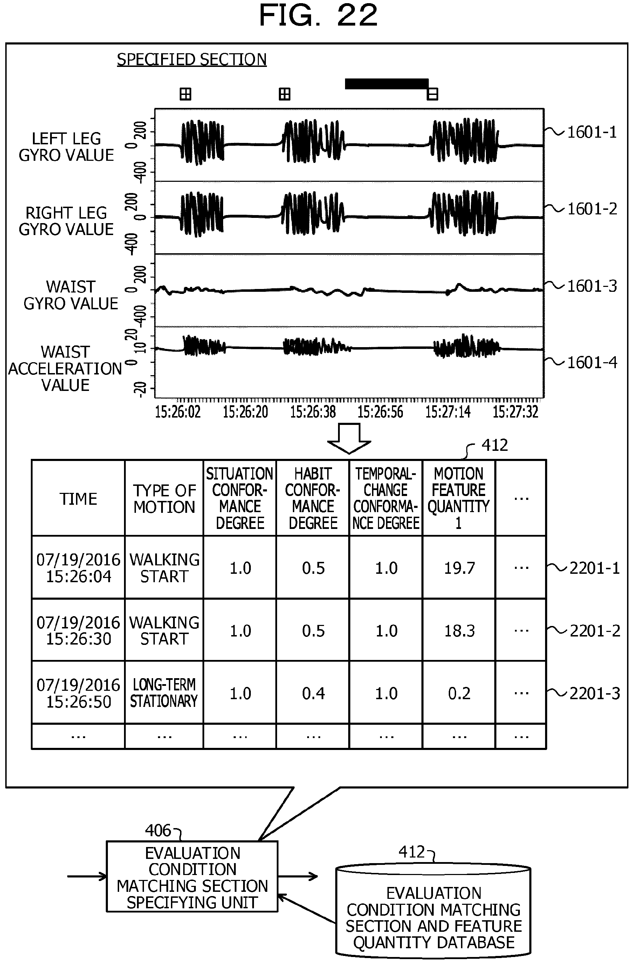

FIG. 22 is an explanatory diagram illustrating an example of specifying the evaluation condition matching section in Example 1. The evaluation condition matching section specifying unit 406 specifies the evaluation condition matching section based on the results of the situation conformance degree calculation unit 403, the habit conformance degree calculation unit 404, and the temporal-change conformance degree calculation unit 405. More specifically, the evaluation condition matching section specifying unit 406 specifies the data section in which the situation conformance degree, the habit conformance degree, and the temporal-change conformance degree are equal to or greater than a certain value, as the evaluation condition matching section, and accumulates the feature quantity of the specified evaluation condition matching section.

The evaluation condition matching section-and-feature quantity database 412 indicates the type of motion, the situation conformance degree, the habit conformance degree, the temporal-change conformance degree, the motion feature quantity 1, and the like of the evaluation condition matching section specified by the evaluation condition matching section specifying unit 406. The evaluation condition matching section-and-feature quantity database 412 illustrated in FIG. 22 has records 2201-1 to 2201-3.

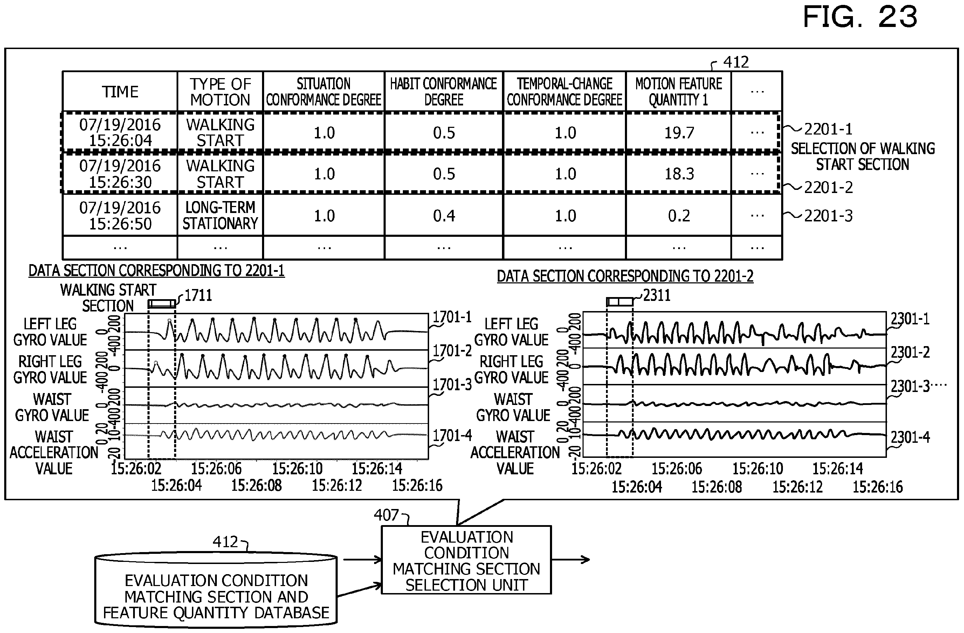

FIG. 23 is an explanatory diagram illustrating an example of selecting the evaluation condition matching section in Example 1. The evaluation condition matching section selection unit 407 refers to the evaluation condition matching section-and-feature quantity database 412 and selects an evaluation condition matching section to be used for the recovery evaluation.

In the example of FIG. 23, the evaluation condition matching section selection unit 407 selects the records 2201-1 and 2201-2 in which the type of motion is the "walking start" that was preset by the doctor. In FIG. 23, as the data section corresponding to the record 2201-1, the walking start section 1711 is illustrated in the sensor data 1701-1 to the sensor data 1701-4. Also, as the data section corresponding to the record 2201-2, a walking start section 2311 is illustrated in sensor data 2301-1 to sensor data 2301-4.

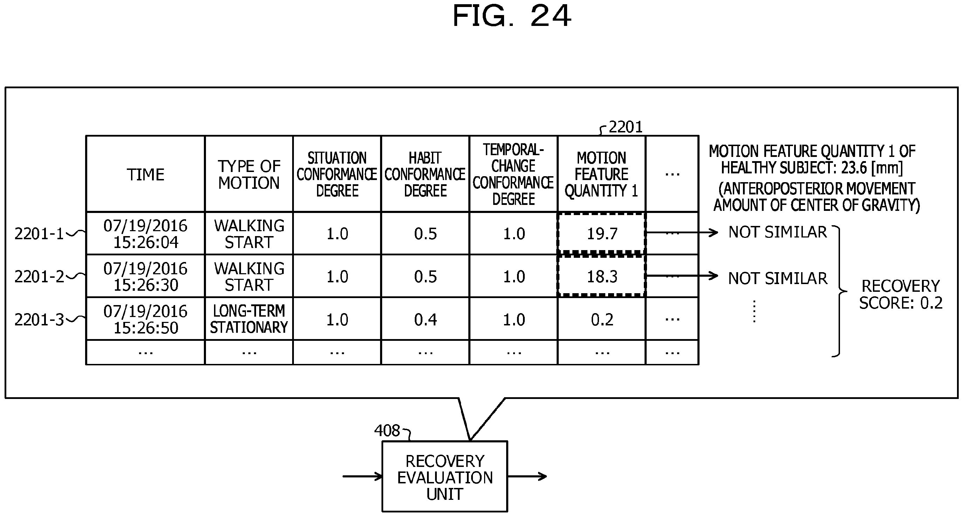

FIG. 24 is an explanatory diagram illustrating an example of calculating a recovery score in Example 1. The recovery evaluation unit 408 calculates a recovery score of the evaluation condition matching section, which is selected by the evaluation condition matching section selection unit 407, based on the processing or the processing result of the data section defining unit 402 to the habit conformance degree calculation unit 404.

In the example of FIG. 24, the recovery evaluation unit 408 calculates a recovery score by comparing the feature quantity with the feature of the healthy subject. The recovery evaluation unit 408 calculates a difference between the motion feature quantity of the evaluation condition matching section and the motion feature quantity of the healthy subject for each selected evaluation condition matching sections, and uses, as the recovery score, a ratio of the case where the calculated difference is within a certain value. Here, in the example of FIG. 24, the comparison with the healthy subject is performed by using the forward and backward movement amount of the center of gravity as the motion feature quantity 1. It is assumed that the forward and backward movement amount of the center of gravity of the healthy subject is 23.6 [mm]. For each of the records 2201-1 and 2201-2, the recovery evaluation unit 408 determines that the difference between the motion feature quantity of the evaluation condition matching section and the motion feature quantity of the healthy subject is greater than a certain value, and thus determines that the motion of the evaluation condition matching section is not similar to the motion of the healthy subject. In addition, the recovery evaluation unit 408 determines whether the motion of the evaluation condition matching section after the record 2201-2 is similar to the motion of the healthy subject.

Finally, the recovery evaluation unit 408 calculates the ratio of the motion feature quantity determined to be similar as the recovery score. In the example of FIG. 24, since the ratio of the motion feature quantity determined to be similar is 0.2, the recovery evaluation unit 408 calculates the recovery score as 0.2.

### (Description of Example 2)

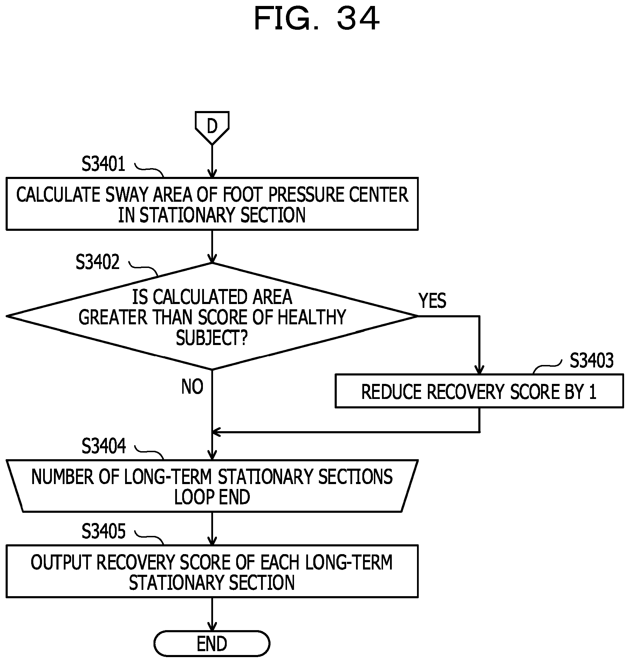

Next, as Example 2 not covered by the claimed invention, a process of a case in which a concussion patient is the target will be described with reference to FIGs. 25 to 44. Note that the same parts as those described in Example 1 are denoted by the same reference numerals, and illustration and description thereof are omitted.

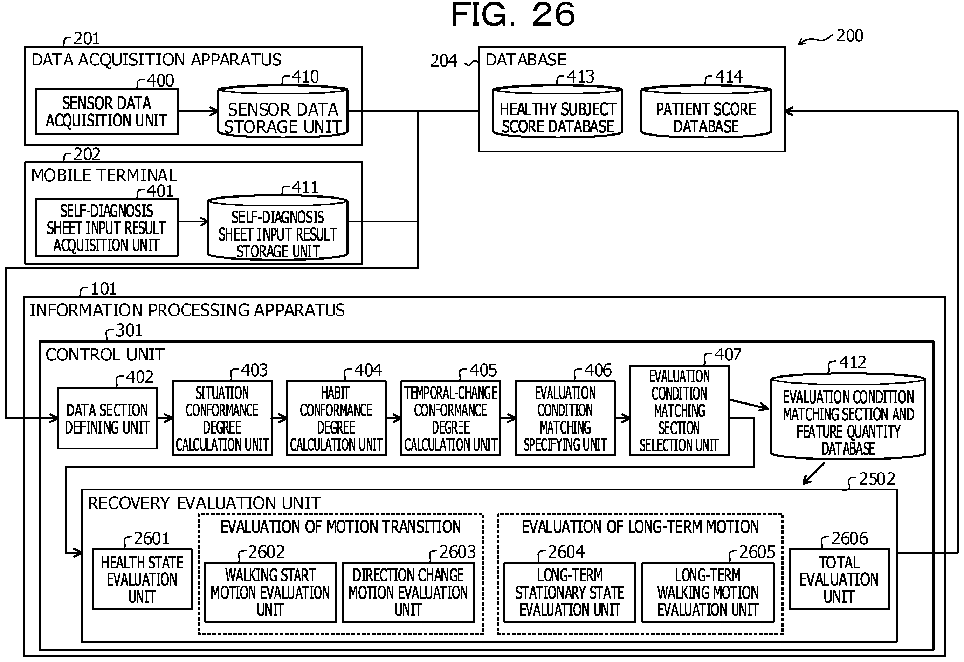

FIG. 25 is an explanatory diagram (part 1) illustrating a functional configuration example of an information processing system 2500 in Example 2. FIG. 26 is an explanatory diagram (part 2) illustrating a functional configuration example of the information processing system 2500 in Example 2. The control unit 301 included in the information processing system 2500 includes a situation conformance degree calculation unit 2501 and a recovery evaluation unit 2502 instead of the situation conformance degree calculation unit 403 and the recovery evaluation unit 408 of Example 1.

The situation conformance degree calculation unit 2501 includes a walking start motion extraction unit 2511 and a direction change motion extraction unit 2512 for the extraction of motion transitions, and includes a long-term stationary state extraction unit 2513 and a long-term walking motion extraction unit 2514 for the extraction of long-term motions.

In addition, the situation conformance degree calculation unit 2501 may calculate a situation conformance degree of each data section depending on whether the type of motion or state of the model associated with the data section, the time length of the data section, and the feature quantity of the data of the data section satisfy a predetermined condition. Then, the predetermined condition is at least one of the following three conditions.

The first condition is a case where the type of motion of the model associated with the data section is a motion transition. The motion transition may be, for example, a motion of starting walking from a stationary state, a direction change motion or the like. Specifically, the walking start motion extraction unit 2511 extracts a walking start motion section. Further, the direction change motion extraction unit 2512 extracts a direction change motion section.

The second condition is a case where the time length of the data section is a period longer than a predetermined period. A motion for a period longer than the predetermined period is referred to as "long-term motion". The long-term motion may be, for example, a walking motion for a long period of time or a long distance, a stationary state for a long period of time and the like. Specifically, the long-term stationary state extraction unit 2513 extracts a long-term stationary state. In addition, the long-term walking motion extraction unit 2514 extracts the long-term walking motion.

The third condition is a case where a load amount, which is extracted based on a duration time or a continuation number of the motion of the data section, or the momentum of the model associated with the data section, is a predetermined threshold value or greater. Here, the third condition is a condition focusing on the fact that concussion patients tend to have balance abnormality when the load is large. For example, the situation conformance degree calculation unit 2501 sets the situation conformance degree to be high in a situation where it is determined that a load is applied to the patient. In addition, the situation conformance degree calculation unit 2501 calculates the load as the load amount as the weight of the situation conformance degree. Then, the situation conformance degree calculation unit 2501 determines the load amount based on, for example, the duration time of the motion, the continuation number of the motion, the momentum of the motion or the like. The situation conformance degree calculation unit 2501 may determine the load amount from, for example, the length of the walking section, the number of steps, the walking speed, the angle or the rotation amount of the direction change or the like. In addition, the situation conformance degree calculation unit 2501 may perform determination depending on whether the patient was concurrently executing another task at the time of performing the motion.

For example, the situation conformance degree calculation unit 2501 may determine that a load is applied in a case where the patient is walking while holding a heavy object or in a case where the patient walks 200 steps than in a case where the patient walks 20 steps. In addition, the situation conformance degree calculation unit 2501 may determine that the load is also applied in a case where the patient is walking on the stairs or in a case where it is determined that the patient is suddenly standing up.

In addition, when the health-related quality of life (QOL) is low, the severity of symptoms is high, and the number of symptoms is large in the data acquired by the self-diagnosis sheet, the situation conformance degree calculation unit 2501 may set the situation conformance degree of the data of the day to be high.

The recovery evaluation unit 2502 includes a health state evaluation unit 2601, a walking start motion evaluation unit 2602, a direction change motion evaluation unit 2603, a long-term stationary state evaluation unit 2604, a long-term walking motion evaluation unit 2605, and a total evaluation unit 2606.

The function of the health state evaluation unit 2601 will be described. At this time, the healthy subject score database 413 stores scores obtained by evaluating a health state, a restriction degree of an activity, a degree of physical health, an amount of social activity, a degree of pain, a magnitude of the energy and emotion, and the severity and the number of diseases in the self-diagnosis sheet of the healthy subject. Similarly, the self-diagnosis sheet input result storage unit 411 stores scores obtained by evaluating a health state, a restriction degree of an activity, a degree of physical health, an amount of social activity, a degree of pain, a magnitude of the energy and emotion, and the severity and the number of diseases in the self-diagnosis sheet of the patient. Then, the health state evaluation unit 2601 refers to the healthy subject score database 413 and the self-diagnosis sheet input result storage unit 411, and calculates the recovery score of the patient based on the comparison result between the score corresponding to the patient and the score corresponding to the healthy subject.

Next, the function of the walking start motion evaluation unit 2602 will be described. At this time, the healthy subject score database 413 stores the anteroposterior acceleration value and the anteroposterior movement amount of the center of gravity of the healthy subject during the walking start motion, and the mediolateral and anteroposterior movement amount of the foot pressure center of the healthy subject in a backward section during the walking start motion. Then, when the motion of the model associated with the specified evaluation condition matching section is the walking start motion, the walking start motion evaluation unit 2602 executes at least one walking start motion feature quantity calculation process of first and second walking start motion feature quantity calculation processes.

Here, the first walking start motion feature quantity calculation process is a process of calculating the anteroposterior acceleration value and the anteroposterior movement amount of the center of gravity of the patient in the evaluation condition matching section. The second walking start motion feature quantity calculation process is a process of calculating the mediolateral and anteroposterior movement amount of the foot pressure center of the patient based on the data of the backward section of the evaluation condition matching section. Then, the walking start motion evaluation unit 2602 calculates the recovery score of the patient based on the processing result of at least one walking start motion feature quantity calculation process and the storage contents of the healthy subject score database 413. Regarding the backward section of the evaluation condition matching section, the walking start motion evaluation unit 2602 may set the second half section of the evaluation condition matching section as the backward section, set a partial section of the second half section as the backward section, or set a section in which it is determined that the trunk is moved in the backward direction based on the acceleration value of the center of gravity of the patient, as the backward section. Hereinafter, the section in which it is determined that the trunk is moved in the backward direction based on the acceleration value of the center of gravity of the patient is referred to as a "backward movement section". The backward section during the walking start motion stored in the healthy subject score database 413 is also the section calculated based on the same criterion as the backward section of the evaluation condition matching section.

In the first walking start motion feature quantity calculation process, the walking start motion evaluation unit 2602 may calculate the above-described value based on the data of the evaluation condition matching section among the time series data obtained from the acceleration sensor attached to the patient's waist as the sensor 102. In the second walking start motion feature quantity calculation process, the walking start motion evaluation unit 2602 may calculate the above-described value based on the data of the evaluation condition matching section among the time series data obtained from the acceleration sensor attached to the patient's waist as the sensor 102.

Next, the function of the direction change motion evaluation unit 2603 will be described. At this time, the healthy subject score database 413 stores a ratio of the anteroposterior movement amount of the center of gravity of the healthy subject in the direction change motion to the anteroposterior movement amount of the center of gravity of the healthy subject in the walking motion before the direction change motion, and a ratio of the vertical movement amount of the center of gravity of the healthy subject in the direction change motion to the vertical movement amount of the center of gravity of the healthy subject in the walking motion. Furthermore, the healthy subject score database 413 stores a step interval of the healthy subject in each of the direction change motion and the walking motion, a time length of the direction change motion, and a time length of a direction change motion between a walking section after a direction change motion and a seated section after the walking section. Then, when the motion of the model associated with the specified evaluation condition matching section is the direction change motion, the direction change motion evaluation unit 2603 executes at least one direction change motion feature quantity calculation process of first to third direction change motion feature quantity calculation processes. Regarding the step interval, the direction change evaluation unit 2603 may determine a time interval required for stepping forward one step, based on the values of the acceleration or gyro sensor attached to the waist or the leg, and may set the determined time interval as the above-described step interval.

Here, the first direction change motion feature quantity calculation process specifies a walking section before the evaluation condition matching section. The first direction change motion feature quantity calculation process is a process of calculating a ratio of the anteroposterior movement amount of the center of gravity of the patient in the evaluation condition matching section to the anteroposterior movement amount of the center of gravity of the patient in the walking section, and a ratio of the vertical movement amount of the center of gravity of the patient in the evaluation condition matching section to the vertical movement amount of the center of gravity of the patient in the walking section, based on the data of the evaluation condition matching section and the data of the walking section. The second direction change motion feature quantity calculation process is a process of calculating the step interval of the patient in each of the evaluation condition matching section and the walking section based on the data of the evaluation condition matching section and the data of the walking section before the evaluation condition matching section. The third direction change motion feature quantity calculation process specifies a second direction change section between the walking section after the evaluation condition matching section and the seated section after the walking section. Then, the third direction change motion feature quantity calculation process is a process of calculating a time length of the evaluation condition matching section and a time length of the second direction change section based on the data of the evaluation condition matching section and the data of the second direction change section.

Then, the direction change motion evaluation unit 2603 calculates a recovery score of the patient based on the processing result of at least one direction change motion feature quantity calculation process and the storage contents of the healthy subject score database 413.

Next, the function of the long-term stationary state evaluation unit 2604 will be described. At this time, the healthy subject score database 413 stores the sway volume of the center of gravity of the healthy subject in a both-legs standing position in the stationary state, and the sway area of the foot pressure center of the healthy subject in the both-legs standing position. Then, when the state of the model associated with the specified evaluation condition matching section is in the stationary state, the long-term stationary state evaluation unit 2604 executes at least one stationary state feature quantity calculation process of first and second stationary state feature quantity calculation processes.

Here, the first stationary state feature quantity calculation process is a process of calculating the sway volume of the center of gravity of the patient in both-legs standing position in the evaluation condition matching section. The second stationary state feature quantity calculation process is a process of calculating the sway area of the foot pressure center of the patient in the both-legs standing position based on the data of the evaluation condition matching section. Then, the long-term stationary state evaluation unit 2604 calculates a recovery score of the patient based on the processing result of at least one stationary state feature quantity calculation process and the storage contents of the healthy subject score database 413.

Further, in the first stationary state feature quantity calculation process, the long-term stationary state evaluation unit 2604 may calculate the above-described value based on the data of the evaluation condition matching section among the time series data obtained from the acceleration sensor and the gyro sensor attached to the patient's waist as the sensor. In the second stationary state feature quantity calculation process, the long-term stationary state evaluation unit 2604 may calculate the above-described value based on the data of the evaluation condition matching section among the time series data obtained from a floor reaction force meter, as the sensor, installed on the floor on which the patient stands on both legs.

Next, the function of the long-term walking motion evaluation unit 2605 will be described. At this time, the healthy subject score database 413 stores the mediolateral and anteroposterior movement amount of the center of gravity of the healthy subject during the long-term walking motion indicative of walking longer than a predetermined period of time, and the variation degree of the mediolateral and anteroposterior movement amount of the center of gravity of the healthy subject during the long-term walking motion. Then, when the motion of the model associated with the evaluation condition matching section is the long-term walking motion, the long-term walking motion evaluation unit 2605 executes at least one long-term walking motion feature quantity calculation process of first and second long-term walking motion feature quantity calculation processes.

Here, the first long-term walking motion feature quantity calculation process is a process of calculating the mediolateral and anteroposterior movement amount of the center of gravity of the patient in the evaluation condition matching section based on the data of the evaluation condition matching section. The second long-term walking motion feature quantity calculation process is a process of calculating the variation degree of the mediolateral and anteroposterior movement amount of the center of gravity of the patient in the evaluation condition matching section based on the data of the evaluation condition conformance section. Here, the long-term walking motion evaluation unit 2605 may use a variation coefficient, a variance value, and a standard deviation as the variation degree. Then, the long-term walking motion evaluation unit 2605 calculates the recovery score of the patient based on the processing result of at least one of the long-term walking motion feature quantity calculation processing and the storage contents of the healthy subject score database 413.

In addition, the healthy subject score database 413 may store a ratio of the mediolateral and anteroposterior movement amount of the center of gravity of the healthy subject in the first half section to the mediolateral and anteroposterior movement amount of the center of gravity of the healthy subject in the second half section during the long-term walking motion, and a ratio of the variation degree of the mediolateral movement amount of the center of gravity of the healthy subject in the first half section to the variation degree of the mediolateral movement amount of the center of gravity of the healthy subject in the second half section. Furthermore, the healthy subject score database 413 may store the variation degree of the walking speed of the healthy subject in the second half section and the variation degree of the leg swing time of the healthy subject in the second half section. At this time, when the motion of the model associated with the evaluation condition matching section is a long-term walking motion, the long-term walking motion evaluation unit 2605 may execute at least one long-term walking motion feature quantity calculation process of third and fourth long-term walking motion feature quantity calculation processes. Also, the leg swing time is a time while one leg is landing and the other leg is floating during the walking motion.

Here, the third long-term walking motion feature quantity calculation process is a process of calculating a ratio of the mediolateral and anteroposterior movement amount of the center of gravity of the patient in the first half section to the mediolateral and anteroposterior movement amount of the center of gravity of the patient in the second half section of the evaluation condition matching section, and a ratio of the variation degree of the mediolateral movement amount of the center of gravity of the patient in the first half section to the variation degree of the mediolateral movement amount of the center of gravity of the patient in the second half section, based on the data of the evaluation condition matching section. The fourth long-term walking motion feature quantity calculation process is a process of calculating the variation degree of the walking speed of the patient in the second half section and the variation degree of the leg swing time of the patient in the second half section based on the data of the evaluation condition matching section. Then, the long-term walking motion evaluation unit 2605 calculates the recovery score of the patient based on the processing result of at least one of the long-term walking motion feature quantity calculation processing and the storage contents of the healthy subject score database 413.

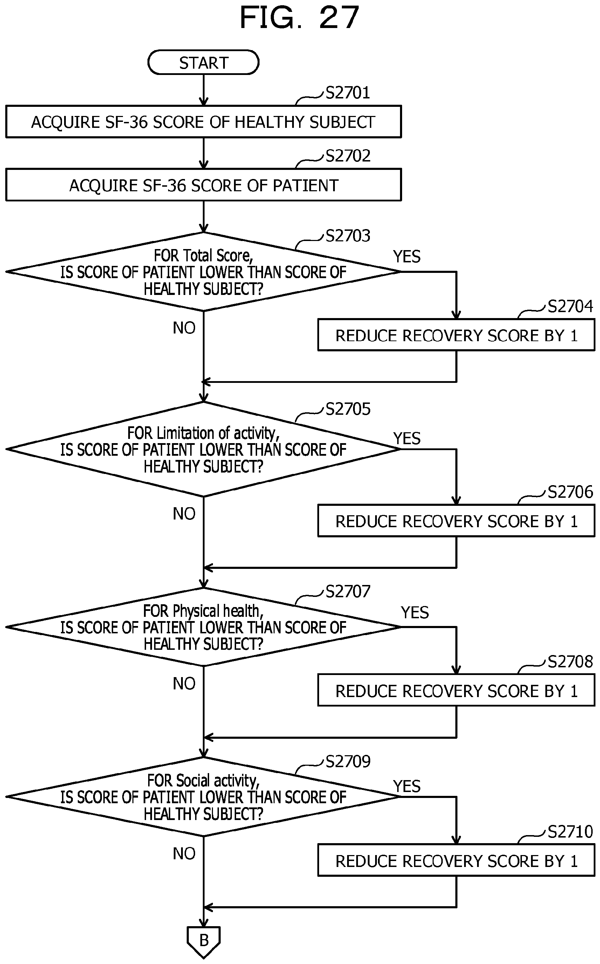

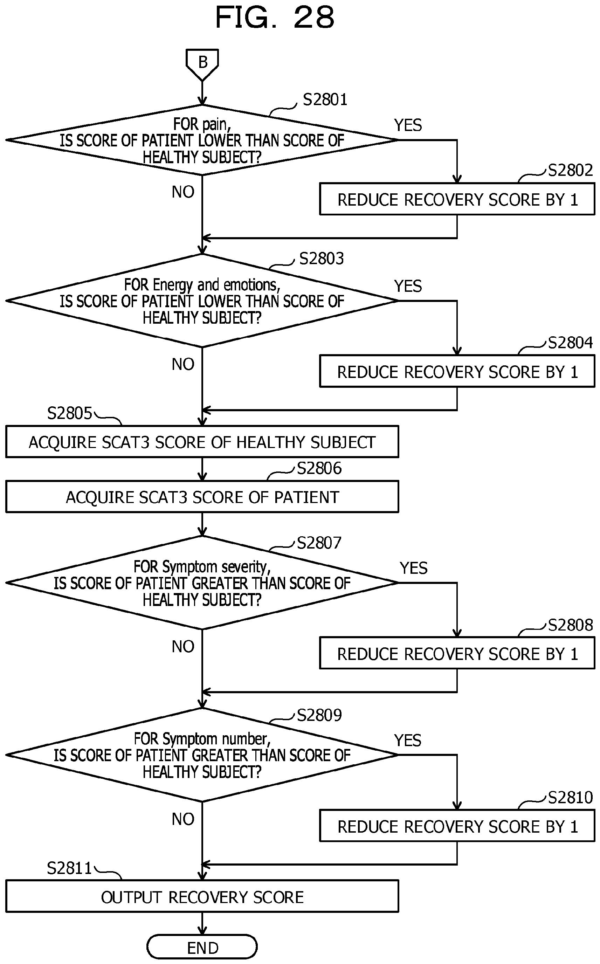

FIG. 27 is a flowchart (part 1) illustrating an example of a process procedure of the health state evaluation unit 2601. Further, FIG. 28 is a flowchart (part 2) illustrating the example of the process procedure of the health state evaluation unit 2601. Here, the health state evaluation unit 2601 executes the processes illustrated in FIGs. 26 and 27 for each day, and calculates the recovery score of each day.

The health state evaluation unit 2601 acquires the SF-36 score of the healthy subject (step S2701). In addition, the health state evaluation unit 2601 acquires the SF-36 score of the patient (step S2702). Next, the health state evaluation unit 2601 refers to the two acquired SF-36 scores and determines whether the score of the patient is less than the score of the healthy subject for the total score (step S2703). When the score of the patient is less than the score of the healthy subject (step S2703: Yes), the health state evaluation unit 2601 decreases the recovery score by 1 (step S2704).

After the process of step S2704 is ended or when the score of the patient is equal to or greater than the score of the healthy subject (step S2703: No), the health state evaluation unit 2601 determines whether the score of the patient is less than the score of the healthy subject for the limitation of activity (step S2705). When the score of the patient is less than the score of the healthy subject (step S2705: Yes), the health state evaluation unit 2601 decreases the recovery score by 1 (step S2706).

After the process of step S2706 or when the score of the patient is equal to or greater than the score of the healthy subject (step S2705: No), the health state evaluation unit 2601 determines whether the score of the patient is less than the score of the healthy subject for the physical health (step S2707). When the score of the patient is less than the score of the healthy subject (step S2707: Yes), the health state evaluation unit 2601 decreases the recovery score by 1 (step S2708).

After the process of step S2708 is ended or when the score of the patient is equal to or greater than the score of the healthy subject (step S2707: No), the health state evaluation unit 2601 determines whether the score of the patient is less than the score of the healthy subject for the social activity (step S2709). When the score of the patient is less than the score of the healthy subject (step S2709: Yes), the health state evaluation unit 2601 decreases the recovery score by 1 (step S2710).

After the process of step S2710 is ended or when the score of the patient is equal to or greater than the score of the healthy subject (step S2709: No), the health state evaluation unit 2601 determines whether the score of the patient is less than the score of the healthy subject for the pain (step S2801). When the score of the patient is less than the score of the healthy subject (step S2801: Yes), the health state evaluation unit 2601 decreases the recovery score by 1 (step S2802).

After the process of step S2802 is ended or when the score of the patient is equal to or greater than the score of the healthy subject (step S2801: No), the health state evaluation unit 2601 determines whether the score of the patient is less than the score of the healthy subject for the energy and emotions (step S2803). When the score of the patient is less than the score of the healthy subject (step S2803: Yes), the health state evaluation unit 2601 decreases the recovery score by 1 (step S2804).

After the process of step S2802 is ended or when the score of the patient is equal to or greater than the score of the healthy subject (step S2803: No), the health state evaluation unit 2601 acquires the SCAT3 score of the healthy subject (step S2805). In addition, the health state evaluation unit 2601 acquires the SCAT3 score of the patient (step S2806).

Then, the health state evaluation unit 2601 determines whether the score of the patient is greater than the score of the healthy subject for the symptom severity (step S2807). When the score of the patient is greater than the score of the healthy subject (step S2807: Yes), the health state evaluation unit 2601 decreases the recovery score by 1 (step S2808).

After the process of step S2808 is ended or when the score of the patient is equal to or less than the score of the healthy subject (step S2807: No), the health state evaluation unit 2601 determines whether the score of the patient is greater than the score of the healthy subject for the symptom number (step S2809). When the score of the patient is greater than the score of the healthy subject (step S2809: Yes), the health state evaluation unit 2601 decreases the recovery score by 1 (step S2810).

After the process of step S2810 is ended or when the score of the patient is equal to or less than the score of the healthy subject (step S2809: No), the health state evaluation unit 2601 outputs the recovery score (step S2811). After the process of step S2811 is ended, the health state evaluation unit 2601 ends the series of processes.

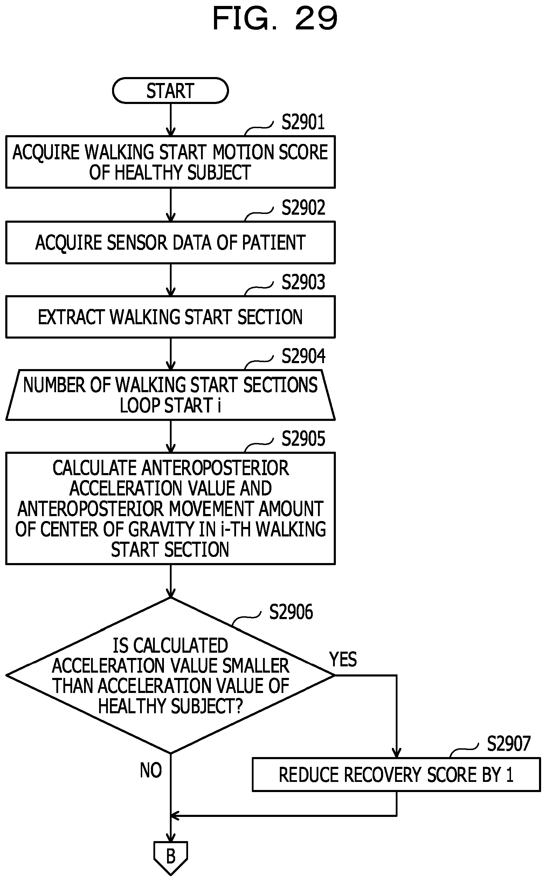

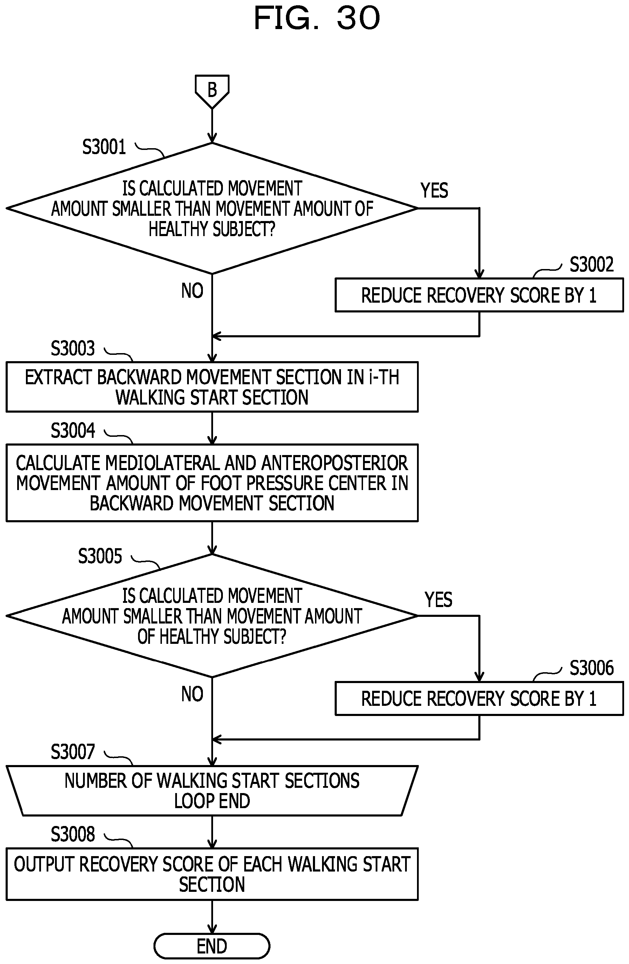

FIG. 29 is a flowchart (part 1) illustrating an example of a process procedure of the walking start motion evaluation unit 2602. Further, FIG. 30 is a flowchart (part 2) illustrating the example of the process procedure of the walking start motion evaluation unit 2602.

The walking start motion evaluation unit 2602 acquires the walking start motion score of the healthy subject (step S2901). Here, the walking start motion score of the healthy subject includes the anteroposterior acceleration and the anteroposterior movement amount of the center of gravity in the walking start section, and the mediolateral and anteroposterior movement amount of the foot pressure center in the backward movement section of the walking start section.

Next, the walking start motion evaluation unit 2602 acquires sensor data of the patient from the sensor data storage unit 410 (step S2902). Then, the walking start motion evaluation unit 2602 extracts a walking start section from the acquired sensor data (step S2903).

Next, the walking start motion evaluation unit 2602 repeats the processes of steps S2904 to S3007 for the number of extracted walking start sections. It is assumed that in the processes of steps S2904 to S3007, a loop counter variable is i. The walking start motion evaluation unit 2602 calculates the anteroposterior acceleration and the anteroposterior movement amount of the center of gravity in the i-th walking start section (step S2905). Next, the walking start motion evaluation unit 2602 determines whether the calculated acceleration value is less than the acceleration value of the healthy subject (step S2906). When the calculated acceleration value is less than the acceleration value of the healthy subject (step S2906: Yes), the walking start motion evaluation unit 2602 decreases the recovery score by 1 (step S2907).

After the process of step S2907 is ended or when the calculated acceleration value is equal to or greater than the acceleration value of the healthy subject (step S2906: No), the walking start motion evaluation unit 2602 determines whether the calculated movement amount is less than the movement amount of the healthy subject (step S3001). When the calculated movement amount is less than the movement amount of the healthy subject (step S3001: Yes), the walking start motion evaluation unit 2602 decreases the recovery score by 1 (step S3002).

After the process of step S3002 is ended or when the calculated movement amount is equal to or greater than the movement amount of the healthy subject (step S3001: No), the walking start motion evaluation unit 2602 extracts the backward movement section of the i-th walking start section (step S3003). Then, the walking start motion evaluation unit 2602 calculates the mediolateral and anteroposterior movement amount of the foot pressure center in the backward movement section (step S3004). Next, the walking start motion evaluation unit 2602 determines whether the calculated movement amount is less than the movement amount of the healthy subject (step S3005). When the calculated movement amount is less than the movement amount of the healthy subject (step S3005: Yes), the walking start motion evaluation unit 2602 decreases the recovery score by 1 (step S3006).

After the process of step S3006 is ended or when the calculated movement amount is equal to or greater than the movement amount of the healthy subject (step S3005: No), the walking start motion evaluation unit 2602 determines whether the processes of steps S2904 to S3007 are repeated for the number of extracted walking start sections (step S3007). In a case where the number of repetitions of the processes of steps S2904 to S3007 is less than the number of extracted walking start sections, the walking start motion evaluation unit 2602 proceeds to the process of step S2904.

When the number of repetitions of the processes of steps S2904 to S3007 reaches the number of extracted walking start sections, the walking start motion evaluation unit 2602 outputs the recovery score for each extracted walking start section (step S3008). After the process of step S3008 is ended, the walking start motion evaluation unit 2602 ends the series of processes.

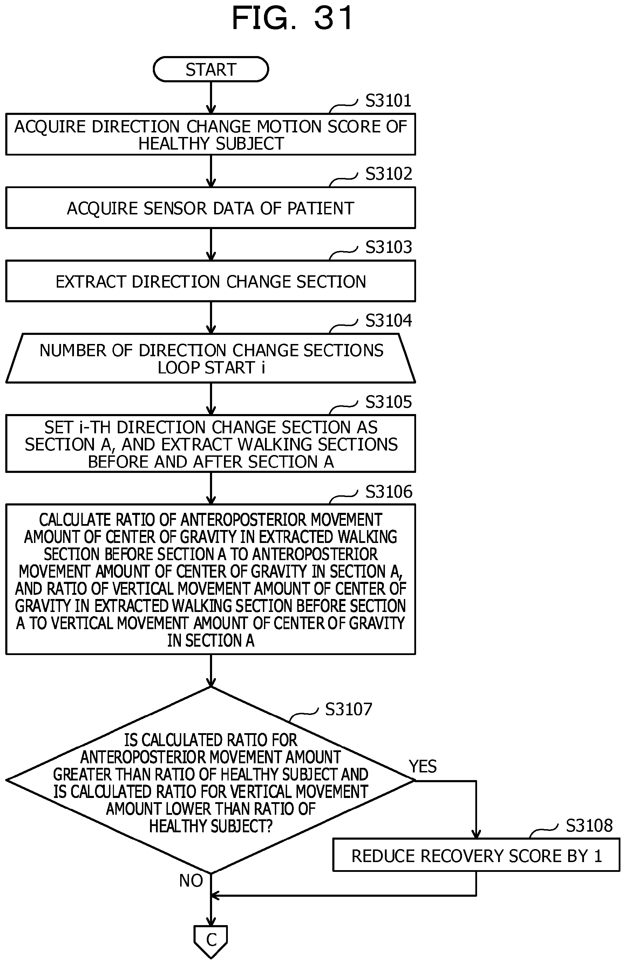

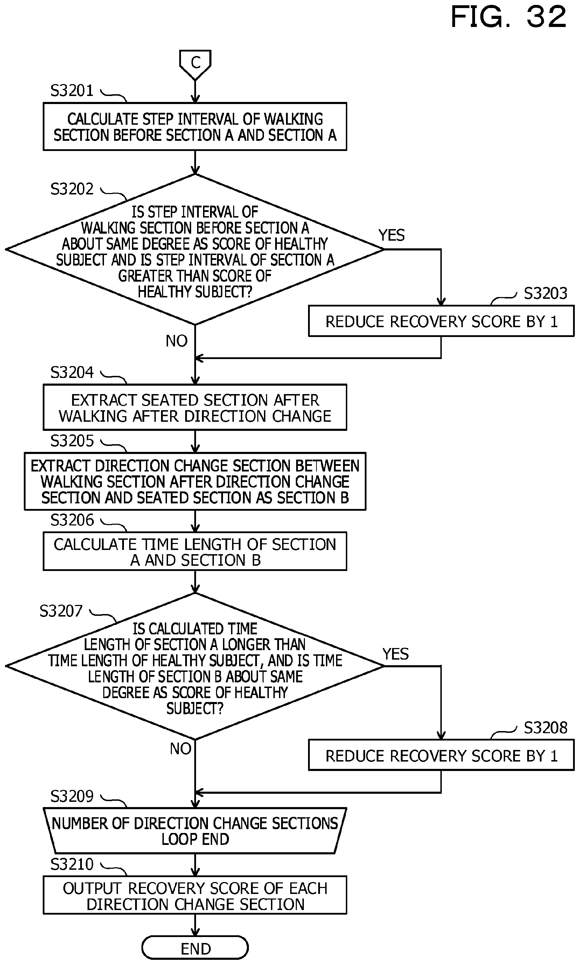

FIG. 31 is a flowchart (part 1) illustrating an example of a process procedure of the direction change motion evaluation unit 2603. FIG. 32 is a flowchart (part 2) illustrating the example of the process procedure of the direction change motion evaluation unit 2603.

The direction change motion evaluation unit 2603 acquires the direction change motion score of the healthy subject (step S3101). Here, the direction change motion score of the healthy subject includes a ratio of the anteroposterior movement amount of the center of gravity in a walking section before a direction change section to the anteroposterior movement amount of the center of gravity in the direction change section, a ratio of the vertical movement amount of the center of gravity in the walking section before the direction change section to the vertical movement amount of the center of gravity in the direction change section, a step interval of the walking section, and a step interval and a time length in the direction change section. Furthermore, in a case where there is a seated section after walking after the direction change section, the direction change motion score of the healthy subject includes a time length of the direction change section between the direction change section and the seated section.

Next, the direction change motion evaluation unit 2603 acquires sensor data of the patient from the sensor data storage unit 410 (step S3102). Then, the direction change motion evaluation unit 2603 extracts a direction change section from the acquired sensor data (step S3103).

Next, the direction change motion evaluation unit 2603 repeats processes of steps S3104 to S3209 for the number of extracted direction change sections. It is assumed that in the processes of steps S3104 to S3209, a loop counter variable is i. The direction change motion evaluation unit 2603 regards the i-th direction change section as a section A and extracts a walking section before and after the section A (step S3105). Next, the direction change motion evaluation unit 2603 calculates a ratio of the anteroposterior movement amount of the center of gravity in the extracted walking section before the section A to the anteroposterior movement amount of the center of gravity in the section A, and a ratio of the vertical movement amount of the center of gravity in the extracted walking section before the section A to the vertical movement amount of the center of gravity in the section A (step S3106).

Then, the direction change motion evaluation unit 2603 determines whether the calculated ratio is greater than the ratio of the healthy subject for the ratio of the anteroposterior movement amount, and the calculated ratio is less than the ratio of the healthy subject for the ratio of the vertical movement amount (step S3107). When the calculated ratio is greater than the ratio of the healthy subject for the ratio of the anteroposterior movement amount, and the calculated ratio is less than the ratio of the healthy subject for the ratio of the vertical movement amount (step S3107: Yes), the direction change motion evaluation unit 2603 decreases the recovery score by 1 (step S3108).

After the process of step S3108 is ended, the direction change motion evaluation unit 2603 calculates the step interval of the walking section before the section A and the section A (step S3201). Even when the calculated ratio is equal to or less than the ratio of the healthy subject for the ratio of the anteroposterior movement amount, and the calculated ratio is equal to or greater than the ratio of the healthy subject for the ratio of the vertical movement amount (step S3107: No), the direction change motion evaluation unit 2603 executes the process of step S3201.

Next, the direction change motion evaluation unit 2603 determines whether the step interval in the walking section before the section A is about the same degree as the score of the healthy subject and the step interval in the section A is greater than the score of the healthy subject (step S3202). Here, the first score of the healthy subject in step S3202 is the step interval in the walking section before the section A, which is included in the direction change motion score of the healthy subject acquired in the process of step S3101. The second healthy subject score in step S3202 is the step interval of the direction change section included in the direction change motion score of the healthy subject acquired in the process of step S3101. Regarding the expression "about the same degree", the direction change motion evaluation unit 2603 determines about the same degree when a difference between the step interval in the walking section before the section A and the score of the healthy subject is within a predetermined threshold value.

When the step interval in the walking section before the section A is about the same degree as the score of the healthy subject and the step interval in the section A is greater than the score of the healthy subject (step S3202: Yes), the direction change motion evaluation unit 2603 decreases the recovery score by 1 (step S3203).

Then, the direction change motion evaluation unit 2603 extracts the seated section after walking after the direction change (step S3204). In a case where the step interval in the walking section before the section A is not about the same degree as the score of the healthy subject or the step interval in the section A is equal to or less than the score of the healthy subject (step S3202: No), the direction change motion evaluation unit 2603 executes the process of step S3204.

After the process of step S3204 is ended, the direction change motion evaluation unit 2603 extracts the direction change section between the walking section after the direction change and the seated section, as a section B (step S3205). Next, the direction change motion evaluation unit 2603 calculates a time length of the section A and the section B (step S3206).

Then, the direction change motion evaluation unit 2603 determines whether the calculated time length of the section A is greater than the score of the healthy subject and the time length of the section B is about the same degree as the score of the healthy subject (step S3207). Here, the first score of the healthy subject in step S3207 is the time length of the direction change section, which is included in the direction change motion score of the healthy subject acquired in the process of step S3101. The second score of the healthy subject in step S3207 is the time length of the direction change section between the direction change section and the seated section, which is included in the direction change motion score of the healthy subject acquired in the process of step S3101.

When the calculated time length of the section A is longer than the score of the healthy subject and the time length of the section B is about the same degree as the score of the healthy subject (step S3207: Yes), the direction change motion evaluation unit 2603 decreases the recovery score by 1 (step S3208).

Then, the direction change motion evaluation unit 2603 determines whether the processes of steps S3104 to S3209 are repeated for the number of extracted direction change sections (step S3209). Similarly, when the time length of the section A is equal to or less than the score of the healthy subject or the time length of the section B is not about the same degree as the score of the healthy subject (step S3207: No), the direction change motion evaluation unit 2603 determines whether the processes of steps S3104 to S3209 are repeated for the number of extracted direction change sections. In a case where the number of repetitions of the processes of steps S3104 to S3209 is less than the number of extracted direction change sections, the direction change motion evaluation unit 2603 proceeds to the process of step S3104.

When the number of repetitions of the processes of steps S3104 to S3209 reaches the number of extracted direction change sections, the direction change motion evaluation unit 2603 outputs the recovery score for each extracted direction change section (step S3210). After the process of step S3210 is ended, the direction change motion evaluation unit 2603 ends the series of processes.

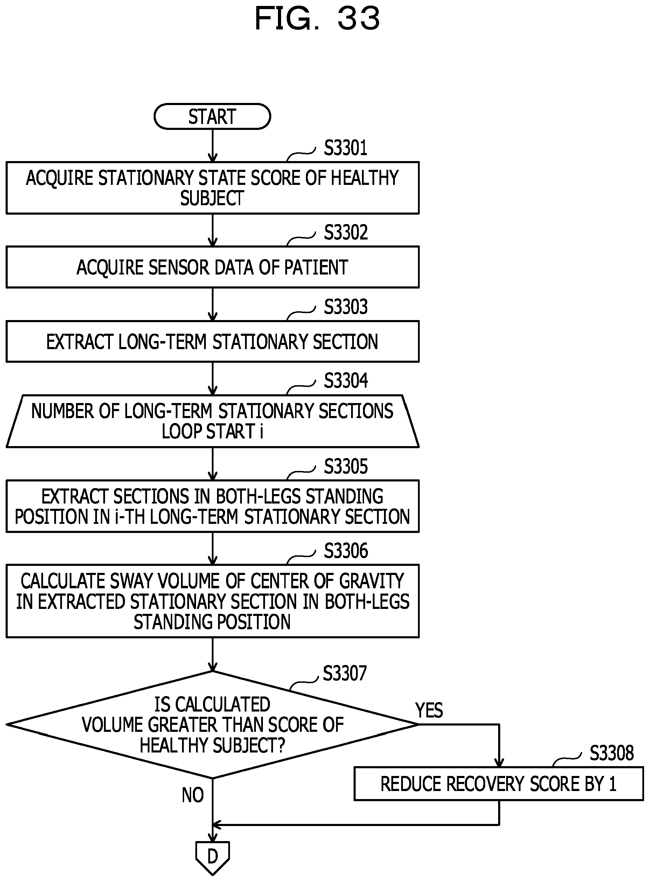

FIG. 33 is a flowchart (part 1) illustrating an example of a process procedure of the long-term stationary state evaluation unit 2604. 34 is a flowchart (part 2) illustrating the example of the process procedure of the long-term stationary state evaluation unit 2604.

The long-term stationary state evaluation unit 2604 acquires a stationary state score of the healthy subject (step S3301). Here, the stationary state score of the healthy subject includes the sway volume of the center of gravity and the sway area of the foot pressure center in the both-legs standing position in the stationary state.

Next, the long-term stationary state evaluation unit 2604 acquires sensor data of the patient from the sensor data storage unit 410 (step S3302). Then, the long-term stationary state evaluation unit 2604 extracts a long-term stationary section from the acquired sensor data (step S3303).

Next, the long-term stationary state evaluation unit 2604 repeats processes of steps S3304 to S3404 for the number of extracted long-term stationary sections. It is assumed that in the processes of steps S3304 to S3404, a loop counter variable is i. The long-term stationary state evaluation unit 2604 extracts a section in the both-legs standing position in the i-th long-term stationary section (step S3305). Next, the long-term stationary state evaluation unit 2604 calculates the sway volume of the center of gravity in the extracted stationary section in the both-legs standing position (step S3306).

Then, the long-term stationary state evaluation unit 2604 determines whether the calculated volume is greater than the score of the healthy subject (step S3307). Here, the score of the healthy subject in step S3307 is the sway volume of the center of gravity in the both-legs standing position in the stationary state, which is included in the stationary state score of the healthy subject acquired in the process of step S3302. When the calculated volume is greater than the score of the healthy subject (step S3307: Yes), the long-term stationary state evaluation unit 2604 decreases the recovery score by 1 (step S3308).

After the process of step S3308 is ended or when the calculated volume is equal to or less than the score of the healthy subject (step S3307: No), the long-term stationary state evaluation unit 2604 calculates the sway area of the foot pressure center in the stationary section (step S3401). Then, the long-term stationary state evaluation unit 2604 determines whether the calculated area is greater than the score of the healthy subject (step S3402). Here, the score of the healthy subject in step S3402 is the sway area of the foot pressure center in the both-legs standing position in the stationary state, which is included in the stationary state score of the healthy subject acquired in the process of step S3302.

When the calculated area is greater than the score of the healthy subject (step S3402: Yes), the long-term stationary state evaluation unit 2604 decreases the recovery score by 1 (step S3403).

After the process of step S3403 is ended or when the calculated area is equal to or less than the score of the healthy subject (step S3402: No), the long-term stationary state evaluation unit 2604 determines whether the processes of steps S3304 to S3404 are repeated for the number of extracted long-term stationary sections (step S3404). In a case where the number of repetitions of the processes of steps S3304 to S3404 is less than the number of extracted long-term stationary sections, the long-term stationary state evaluation unit 2604 proceeds to the process of step S3304.

When the number of repetitions of the processes of steps S3304 to S3404 reaches the number of extracted long-term stationary sections, the long-term stationary state evaluation unit 2604 outputs the recovery score for each of the extracted long-term stationary sections (step S3405). After the process of step S3405 is ended, the long-term stationary state evaluation unit 2604 ends the series of processes.

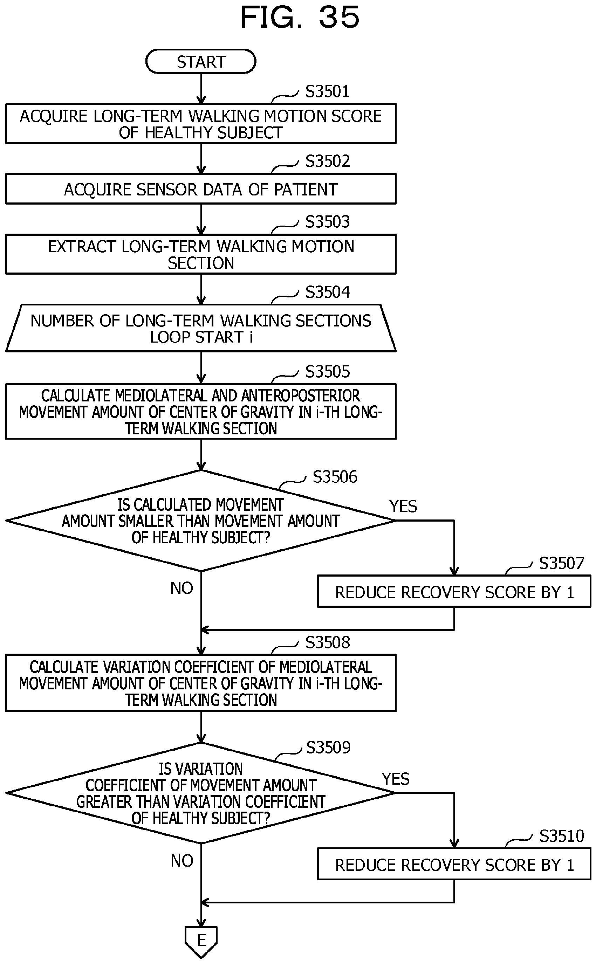

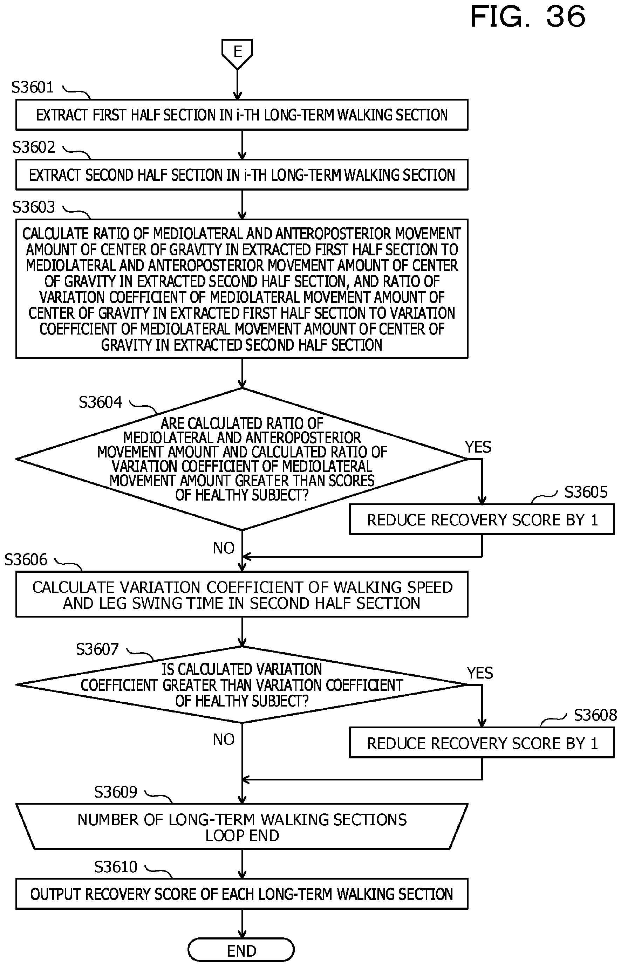

FIG. 35 is a flowchart (part 1) illustrating an example of a process procedure of the long-term walking motion evaluation unit 2605. FIG. 36 is a flowchart (part 2) illustrating the example of the process procedure of the long-term walking motion evaluation unit 2605.

The long-term walking motion evaluation unit 2605 acquires a long-term walking motion score of the healthy subject (step S3501). Here, the long-term walking motion score of the healthy subject includes the anteroposterior movement amount of the center of gravity during the long-term walking section, the variation degree of the mediolateral movement amount of the center of gravity, a ratio of the mediolateral and anteroposterior movement amount of the center of gravity in the first half section to the mediolateral and anteroposterior movement amount of the center of gravity in the second half section of the long-term walking section, and a ratio of the variation degree of the mediolateral movement amount of the center of gravity in the first half section to the variation degree of the mediolateral movement amount of the center of gravity in the second half section of the long-term walking section. In addition, the long-term walking motion score of the healthy subject includes the variation degree of the walking speed in the second half section of the long-term walking section and the variation degree of the leg swing time.

Next, the long-term walking motion evaluation unit 2605 acquires sensor data of the patient from the sensor data storage unit 410 (step S3502). Then, the long-term walking motion evaluation unit 2605 extracts a long-term walking motion section (step S3503).

Next, the long-term walking motion evaluation unit 2605 repeats processes of steps S3504 to S3609 for the number of extracted long-term walking sections. It is assumed that in the processes of S3504 to S3609, a loop counter variable is i. The long-term walking motion evaluation unit 2605 calculates the mediolateral and anteroposterior movement amount of the center of gravity in the i-th long-term walking section (step S3505). Next, the long-term walking motion evaluation unit 2605 determines whether the calculated movement amount is less than the movement amount of the healthy subject (step S3506). When the calculated movement amount is less than the movement amount of the healthy subject (step S3506: Yes), the long-term walking motion evaluation unit 2605 decreases the recovery score by 1 (step S3507).

After the process of step S3507 is ended or when the calculated movement amount is equal to or greater than the movement amount of the healthy subject (step S3506: No), the long-term walking motion evaluation unit 2605 calculates the variation degree of the movement amount of the center of gravity in the mediolateral direction in the i-th long-term walking section (step S3508). Then, the long-term walking motion evaluation unit 2605 determines whether the variation degree of the movement amount is greater than the variation degree of the healthy subject (step S3509). In a case where the variation degree of the movement amount is greater than the variation degree of the healthy subject (step S3509: Yes), the long-term walking motion evaluation unit 2605 decreases the recovery score by 1 (step S3510).

After the process of step S3510 is ended or when the variation degree of the movement amount is equal to or less than the variation degree of the healthy subject (step S3509: No), the long-term walking motion evaluation unit 2605 extracts the first half section of the i-th long-term walking section (step S3601). In addition, the long-term walking motion evaluation unit 2605 extracts the second half section in the i-th long-term walking section (step S3602).

Next, the long-term walking motion evaluation unit 2605 calculates a ratio of the mediolateral and anteroposterior movement amount of the center of gravity in the extracted first half section to the mediolateral and anteroposterior movement amount of the center of gravity in the extracted second half section, and a ratio of the variation degree of the mediolateral movement amount of the center of gravity in the extracted first half section to the variation degree of the mediolateral movement amount of the center of gravity in the extracted second half section (step S3603). Then, the long-term walking motion evaluation unit 2605 determines whether the calculated ratio of the mediolateral and anteroposterior movement amount and the calculated ratio of the variation degree of the mediolateral movement amount are respectively greater than the scores of the healthy subject (step S3604). Here, for the scores of the healthy subject, the long-term walking motion evaluation unit 2605 compares the calculated ratio of the mediolateral and anteroposterior movement amount with the ratio of the mediolateral and anteroposterior movement amount of the center of gravity in the first half section to the mediolateral and anteroposterior movement amount of the center of gravity in the second half section of the long-term walking section, which is included in the long-term walking motion score of the healthy subject. Similarly, the long-term walking motion evaluation unit 2605 compares the calculated ratio of the variation degree of the mediolateral movement amount with the ratio of the variation degree of the mediolateral movement amount, which is included in the long-term walking motion score of the healthy subject.

When the calculated ratio of the mediolateral and anteroposterior movement amount and the calculated ratio of the variation degree of the mediolateral movement amount are respectively greater than the scores of the healthy subject (step S3604: Yes), the long-term walking motion evaluation unit 2605 decreases the recovery score by 1 (step S3605).

After the process of step S3605 is ended or when at least one of the calculated ratio of the mediolateral and anteroposterior movement amount and the calculated ratio of the variation degree of the mediolateral movement amount is equal to or less than the score of the healthy subject (step S3604: No), the long-term walking motion evaluation unit 2605 calculates the variation degree of each of the walking speed and the leg swing time in the second half section (step S3606). Next, the long-term walking motion evaluation unit 2605 determines whether the calculated variation degree is greater than the variation degree of the healthy subject (step S3607).

When the calculated variation degree is greater than the variation degree of the healthy subject (step S3607: Yes), the long-term walking motion evaluation unit 2605 decreases the recovery score by 1 (step S3608). Here, since there are two calculated variation degrees, the long-term walking motion evaluation unit 2605 compares each of the calculated variation degrees with the corresponding variation degree of the healthy subject, and when the calculated variation degree is greater than the variation degree of the healthy subject, the long-term walking motion evaluation unit 2605 decrease the recovery score by 1. For example, when both the variation degree of n the walking speed and the variation degree of the leg swing time in the second half section are greater than the corresponding variation degree of the healthy subject, the long-term walking motion evaluation unit 2605 decreases the recovery score by 2.

After the process of step S3608 is ended or when the calculated variation degree is equal to or less than the variation degree of the healthy subject (step S3607: No), the long-term walking motion evaluation unit 2605 determines whether the processes of steps S3504 to S3609 are repeated for the number of extracted long-term walking sections (step S3609). In a case where the number of repetitions of the processes of steps S3504 to S3609 is less than the number of extracted long-term walking sections, the long-term walking motion evaluation unit 2605 proceeds to the process of step S3504.

When the number of repetitions of the processes of steps S3504 to S3609 reaches the number of extracted long-term walking sections, the long-term walking motion evaluation unit 2605 outputs the recovery score for each extracted long-term walking section (step S3610). After the process of step S3610 is ended, the long-term walking motion evaluation unit 2605 ends the series of processes.

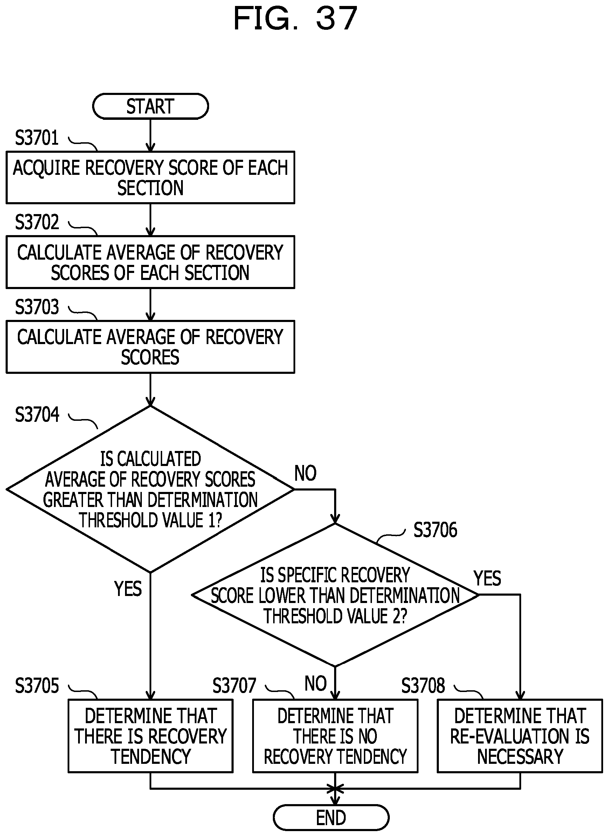

FIG. 37 is a flowchart illustrating an example of a process procedure of the total evaluation unit 2606. The total evaluation unit 2606 acquires the recovery score of each section (step S3701). Here, the recovery score of each section is the recovery scores respectively output by the health state evaluation unit 2601 to the long-term walking motion evaluation unit 2605.

Next, the total evaluation unit 2606 calculates a representative value of the recovery score of each section (step S3702). For example, as the representative value, the total evaluation unit 2606 may use an average, a median, a maximum value, a minimum value, or the like. In step S3702, specifically, regarding the health state evaluation unit 2601, the total evaluation unit 2606 calculates the average of the recovery scores output by the health state evaluation unit 2601, from the recovery score of each day output by the health state evaluation unit 2601. Similarly, regarding the walking start motion evaluation unit 2602, the total evaluation unit 2606 calculates the representative value of the recovery scores output by the walking start motion evaluation unit 2602, from the recovery score for each walking start section output by the walking start motion evaluation unit 2602. Similarly, regarding the direction change motion evaluation unit 2603 to the long-term walking motion evaluation unit 2605, the total evaluation unit 2606 calculates the representative value of the recovery scores output by each of the direction change motion evaluation unit 2603 to the long-term walking motion evaluation unit 2605.

The total evaluation unit 2606 calculates a representative value of the recovery scores aggregating the recovery scores output by the health state evaluation unit 2601 to the long-term walking motion evaluation unit 2605, from the calculated representative values of the recovery scores of the respective sections (step S3703). Next, the total evaluation unit 2606 determines whether the calculated representative value of the recovery score is greater than a determination threshold value 1 preset by a doctor or the like (step S3704).

When the representative value of the recovery score is greater than the determination threshold value 1 (step S3704: Yes), the total evaluation unit 2606 determines that there is a recovery tendency (step S3705). On the other hand, when the representative value of the recovery score is equal to or less than the determination threshold value 1 (step S3704: No), the total evaluation unit 2606 determines whether a specific recovery score preset by a doctor or the like is less than a determination threshold value 2 preset by a doctor or the like (step S3706).

When the specific recovery score preset by the doctor or the like is equal to or greater than the determination threshold value 2 (step S3706: No), the total evaluation unit 2606 determines that there is no recovery tendency (step S3707). On the other hand, when the specific recovery score preset by the doctor or the like is less than the determination threshold value 2 (step S3706: Yes), the total evaluation unit 2606 determines that the re-evaluation is required (step S3708). Here, the reason for determining that the re-evaluation is required in the case of Yes in step S3706, is that the patient is a football player or the like, and there is a possibility that the patient intentionally makes a motion in order to return soon.

### (Specific Example of Example 2)

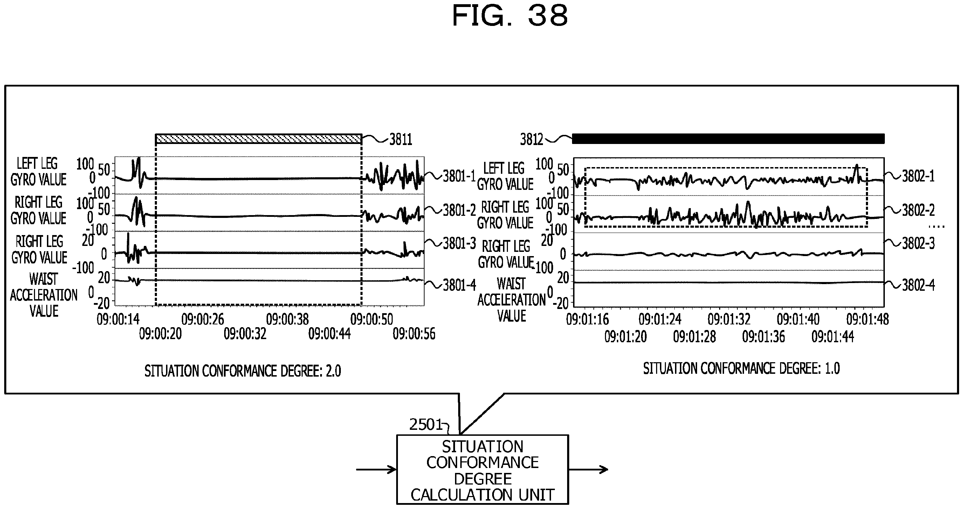

Next, as the specific example of Example 2, a process example of a case of evaluating a concussion patient based on motions during a test in a laboratory environment will be described with reference to FIGs. 38 to 44. In the case of the test in the laboratory environment, it is possible to acquire data labeled as the time of the motion section recorded by the experimenter and data of the sensor installed in the laboratory such as the floor reaction force meter, and the process example using the data will be described. FIGs. 38 to 44 describe a method of acquiring data and calculating a situation conformance degree and a recovery score of when the following four test motions are targeted.

The first test motion is the long-term stationary state during a standing balance test. Here, the standing balance test is, for example, a Balance Error Scoring System (BESS) which is performed when evaluating an exercise ability of a patient. The data section defining unit 402 defines the data measured during the standing balance test by using the motion model in the stationary state. Next, the situation conformance degree calculation unit 2501 determines that a section of which the section length is a certain level or longer, for example, 20 seconds or longer, or a section in the both-legs standing position has a high situation conformance degree. Then, the recovery evaluation unit 2502 compares the waist sway with the score of the healthy subject, and calculates the recovery score.

The second test motion is a long-term walking motion during a walking test. Here, the walking test is, for example, a walking test for a predetermined length, for example, 6 minutes, which is performed when evaluating an exercise ability of a patient. The data section defining unit 402 defines the data measured during the walking test by using the motion model of walking. Next, the situation conformance degree calculation unit 2501 determines that a section of which the section length is a predetermined time, for example, 6 minutes or longer has a high situation conformance degree. Then, the recovery evaluation unit 2502 compares the waist sway with the score of the healthy subject, and calculates the recovery score.

In addition, the recovery evaluation unit 2502 divides a walking section of which the section length is a certain level or longer, into two or four sections having a certain length, compares the waist sway of the divided first and last sections or the feature of walking in the last section with the score of the healthy subject, and calculates the recovery score. At this time, the waist sway is, for example, a ratio of the mediolateral and anteroposterior movement amount of the center of gravity and a ratio of the variation degree of the mediolateral movement amount of the center of gravity. The feature of walking is the variation degree of the walking speed or the leg swing time.

The third test motion is a walking start motion during a walking test. Here, the walking test is, for example, a walking test of a certain distance, for example, 10 m, which is performed when evaluating an exercise ability of a patient. The data section defining unit 402 defines the data measured during the walking test by using the motion model of the walking start. Next, the situation conformance degree calculation unit 2501 determines that a section of walking from the stationary state has a high situation conformance degree. Then, the recovery evaluation unit 2502 compares the waist sway with the score of the healthy subject, and calculates the recovery score.

The fourth test motion is the direction change motion during a mobility test. Here, the mobility test is, for example, a Timed Up and Go test which is performed when evaluating an exercise ability of a patient. The data section defining unit 402 defines the data measured during the mobility test by using the motion model of direction change, walking, standing, and sitting. Next, the situation conformance degree calculation unit 2501 determines that a direction change section between standing and sitting, and a direction change section before and after walking have a high situation conformance degree. Then, the recovery evaluation unit 2502 compares the waist sway with the score of the healthy subject, and calculates the recovery score.

The recovery evaluation unit 2502 compares the time feature quantity of the motion of the direction change section and the walking section immediately before the direction change section, or the time feature quantity of the motion of the direction change section and the direction change section immediately before the seated section with the score of the healthy subject, and calculates the recovery score. The time feature quantity of the operation at this time is, for example, the step interval of one step or the duration time of the motion.

### (Long-Term Stationary State during Standing Balance Test)

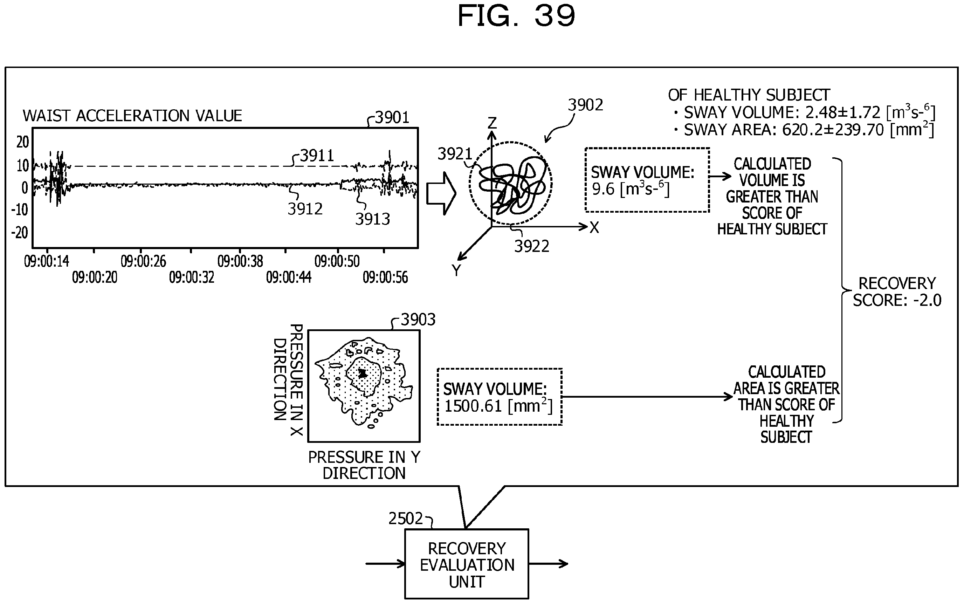

First, an example of calculating a situation conformance degree of a long-term stationary state during the standing balance test, as the first test motion, will be described with reference to FIG. 38 and an example of calculating a recovery score will be described with reference to FIG. 39.

FIG. 38 is an explanatory diagram illustrating an example of calculating a situation conformance degree of the long-term stationary state during a standing balance test. The situation conformance degree calculation unit 2501 calculates the situation conformance degree to be high when the motion of the defined data section is a long-term stationary motion of, for example, 20 seconds or longer, and when the motion is the both-legs standing position. In the example of FIG. 38, for the defined sections, the situation conformance degree calculation unit 2501 sets the initial value of the situation conformance degree of each data section to 0, and calculates the situation conformance degree by adding 1.0 each time the following condition is satisfied.

The first condition is a case where the processing target data section is a section labeled as the long-term stationary state during the standing balance test by the experimenter. The second condition is a case where the processing target data section is a stationary section for, for example, 20 seconds or longer. Here, an example of determining as a stationary section is a case where a section in which the variance value of the three-axis acceleration data of the waist in a certain window section is a certain threshold value or less continues for 20 seconds or longer. The third condition is a case where the processing target data section is the both-legs standing position. For example, the both-legs standing position is a section in which the variance value of the three-axis gyro sensor data of both legs is a certain threshold value or less in the stationary section.

Since a section 3811 of sensor data 3801-1 to sensor data 3801-4 illustrated in the left side of FIG. 38 is the section labeled by the experimenter and the variance value of the gyro value of both legs is a certain value or less, the first condition and the third condition are satisfied. Therefore, the situation conformance degree calculation unit 2501 calculates the situation conformance degree of the section 3811 as 2.0.

Since a stationary section 3812 of sensor data 3802-1 to sensor data 3802-4 illustrated in the right side of FIG. 38 is a section of 20 seconds or longer, the second condition is satisfied. In addition, since the variance value of the gyro value of the both legs in the stationary section 3812 is greater than the certain value, the third condition is not satisfied. Therefore, the situation conformance degree calculation unit 2501 calculates the situation conformance degree of the stationary section 3812 as 1.0.

FIG. 39 is an explanatory diagram illustrating an example of calculating a recovery score in the long-term stationary state during a standing balance test. The recovery evaluation unit 2502 calculates a recovery score of the evaluation condition matching section selected by the evaluation condition matching section selection unit 407 based on the processing or the processing result of the data section defining unit 402 to the habit conformance degree calculation unit 404.

In the example of FIG. 39, the recovery evaluation unit 2502 compares the feature of the waist sway in the long-term stationary section with the feature of the healthy subject and calculates the recovery score. The waist sway is the sway volume calculated from the acceleration sensor of the waist and the sway area calculated from the floor reaction force meter, and when the calculated value is greater than score of the healthy subject, the recovery score is subtracted. Here, for example, the method described in Reference 1 below may be used to calculate the sway volume.

### (Reference 1: Jay L Alberts, 8 others, "Quantification of the Balance Error ScoringSystem with Mobile Technology", 2015, Medicine & Science in Sports & Exercise, Vol. 47, pp 2233-2240)

Further, the method described in reference 2 below may be used to calculate the sway area.

### (Reference 2: Thomas E. Prieto, 4 others, "Measures of Postural Steadiness: Differences Between Healthy Young and Elderly Adults", 1996, IEEE TRANSACTIONS ON BIOMEDICAL ENGINEERING, Vol. 43, pp. 956-966)

Further, in the example of FIG. 39, the sway volume of the healthy subject is set to 2.48 ± 1.72 [m³s⁻⁶] and the sway area of the healthy subject is set to 620.2 ± 239.70 [mm²].

A graph 3901 illustrated in FIG. 39 illustrates the waist acceleration value of the patient with respect to the temporal change. In the graph 3901, a broken line 3911 indicates a waist acceleration value in the Z axis, a solid line 3912 indicates a waist acceleration value in the Y axis, and a dotted line 3913 indicates a waist acceleration value in the X axis.

A graph 3902 illustrated in FIG. 39 illustrates a waist trajectory 3921 in the three-dimensional space and a range 3922 of the waist trajectory based on the graph 3901. The recovery evaluation unit 2502 calculates the sway volume of the patient as 9.6 [m³s⁻⁶]. Then, since the calculated volume is greater than the score of the healthy subject, the recovery evaluation unit 2502 subtracts the recovery score by 1.0.

A pressure distribution 3903 illustrated in FIG. 39 illustrates the pressures in the x and y directions measured by the floor reaction force meter. The filled area in the pressure distribution 3903 indicates that a high pressure is applied, an area with dark shading indicates that a medium pressure is applied, and an area with pale shading indicates that a medium pressure is applied.

The recovery evaluation unit 2502 calculates the sway area of the patient as 1500.61 [mm²]. Then, since the calculated volume is greater than the score of the healthy subject, the recovery evaluation unit 2502 subtracts the recovery score by 1.0. As described above, the recovery evaluation unit 2502 calculates the recovery score of the patient as -2.0.

### (Long-Term Walking Motion during Walking Test)

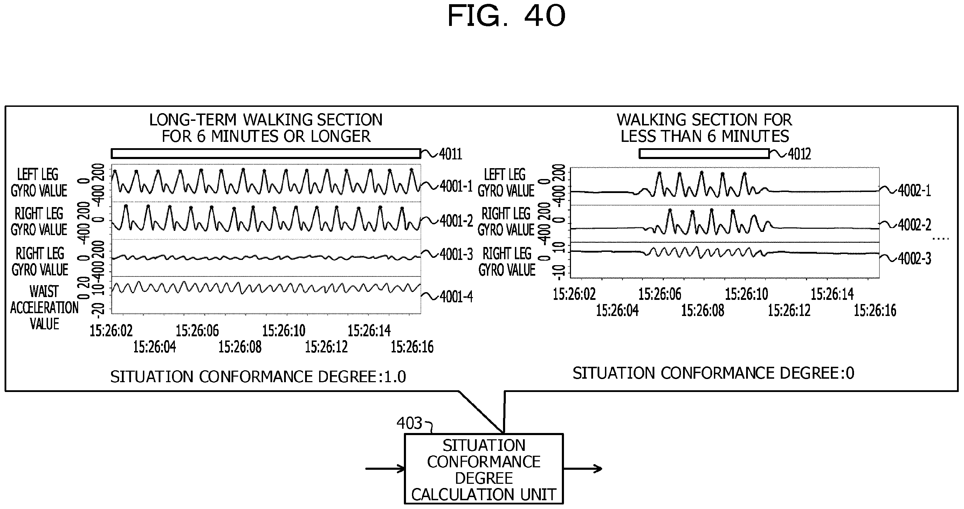

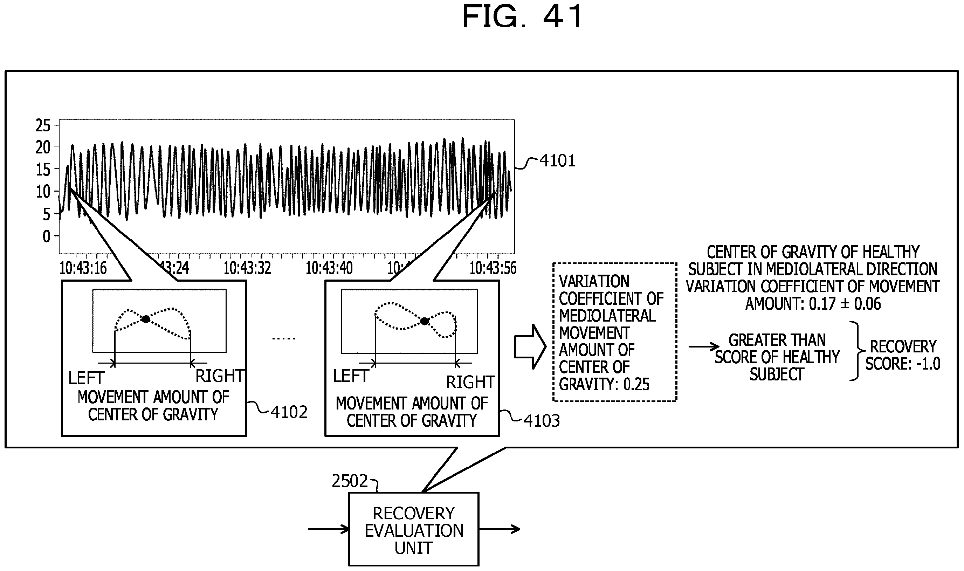

Next, an example of calculating a situation conformance degree of a long-term walking motion during the walking test, as the second test motion, will be described with reference to FIG. 40 and an example of calculating a recovery score will be described with reference to FIG. 41.

FIG. 40 is an explanatory diagram illustrating an example of calculating a situation conformance degree of the long-term walking motion during the walking test. The situation conformance degree calculation unit 2501 calculates a situation conformance degree to be high when the motion of the defined data section is a long-term walking section having a predetermined time, for example, 6 minutes or longer. In the example of FIG. 40, for the defined sections, the situation conformance degree calculation unit 2501 sets the initial value of the situation conformance degree of each data section to 0, and calculates the situation conformance degree by adding 1.0 each time the following condition is satisfied.

The first condition is a case where the processing target data section is a section labeled as the long-term walking state during the walking test by the experimenter. The second condition is a case where the processing target data section is a walking section for 6 minutes or longer. For example, the situation conformance degree calculation unit 2501 determines that the second condition is satisfied when the walking section extracted by the method described in Reference 3 described below has a length of 6 minutes or longer.

### (Reference 3: Paolo Fraccaro, 3 others, "Real-world Gyroscope-based Gait Event Detection and Gait Feature Extraction", eTELEMED 2014, The Sixth International Conference on eHealth, Telemedicine, and Social Medicine, pp. 247-252)

Since the time for a long-term walking section 4011 included in sensor data 4001-1 to sensor data 4001-4 illustrated on the left side of FIG. 40 is a section of 6 minutes or longer, the second condition is satisfied. Therefore, the situation conformance degree calculation unit 2501 calculates the situation conformance degree of the long-term walking section 4011 as 1.0.

On the other hand, since the time for a long-term walking section 4012 included in sensor data 4002-1 to sensor data 4002-4 illustrated on the right side of FIG. 40 is less than 6 minutes, neither condition is satisfied. Therefore, the situation conformance degree calculation unit 2501 calculates the situation conformance degree of the long-term walking section 4012 as 0.

FIG. 41 is an explanatory diagram illustrating an example of calculating a recovery score in a long-term walking motion during a walking test. In the example of FIG. 41, the recovery evaluation unit 2502 compares the feature of the waist sway in the long-term walking section with the feature of the healthy subject and calculates the recovery score. The waist sway is the variation degree of the mediolateral movement amount of the center of gravity calculated from the waist acceleration sensor. The recovery evaluation unit 2502 subtracts the recovery score when the calculated variation degree is greater than the score of the healthy subject. Here, regarding the calculation of the movement amount of the center of gravity, for example, the recovery evaluation unit 2502 may use the method of Reference 4 described below.

### (Reference 4: Japanese Laid-open Patent Publication No. 2013-153975)

In the example of FIG. 41, the variation degree of the mediolateral movement amount of the center of gravity of the healthy subject is 0.17 ± 0.06. A graph 4101 illustrated in FIG. 41 illustrates a waist acceleration value of the patient with respect to the temporal change. Further, graphs 4102 and 4103 indicate the mediolateral movement amount of the center of gravity of the patient for a certain period of time. Next, the recovery evaluation unit 2502 calculates the variation degree of the mediolateral movement amount of the center of gravity of the patient as 0.25. Since the calculated variation coefficient is greater than the score of the healthy subject, the recovery evaluation unit 2502 calculates the recovery score of the patient in the long-term walking motion during the walking test as - 1.0.

### (Walking Start Motion during Walking Test)

Next, an example of calculating a recovery score in a walking start motion during the walking test, as the third test motion, will be described.

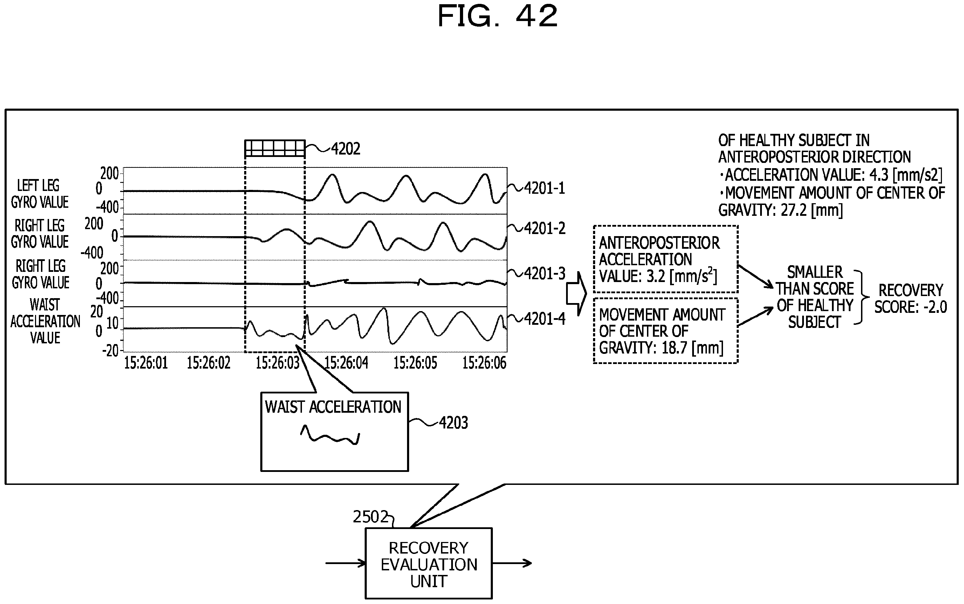

FIG. 42 is an explanatory diagram illustrating an example of calculating the recovery score in the walking start motion during the walking test. In the example of FIG. 42, the recovery evaluation unit 2502 compares the feature quantity of the waist sway in the walking start section with the feature quantity of the healthy subject and calculates the recovery score. The waist sway is the anteroposterior waist acceleration value and the anteroposterior movement amount of the center of gravity calculated from the waist acceleration sensor. The recovery evaluation unit 2502 subtracts the recovery score when the calculated acceleration value or the calculated movement amount of the center of gravity is less than the score of the healthy subject. Further, the recovery evaluation unit 2502 may use, for example, the maximum value of the acceleration sensor peak within the section as the waist acceleration value.

In the example of FIG. 42, the anteroposterior acceleration value of the healthy subject is 4.3 [mm/s²] and the anteroposterior movement amount of the center of gravity of the healthy subject is 27.2 [mm]. Then, the recovery evaluation unit 2502 calculates the anteroposterior acceleration value of the walking start section 4202 included in the sensor data 4201-1 to sensor data 4201-4 illustrated in FIG. 42 as 3.2 [mm/s²], and calculates the movement amount of the center of gravity as 18.7 [mm]. Since each of the calculated values is less than the score of the healthy subject, the recovery evaluation unit 2502 calculates the recovery score of the patient in the walking start motion during the walking test as -2.0.

### (Direction Change Motion during Mobility Test)

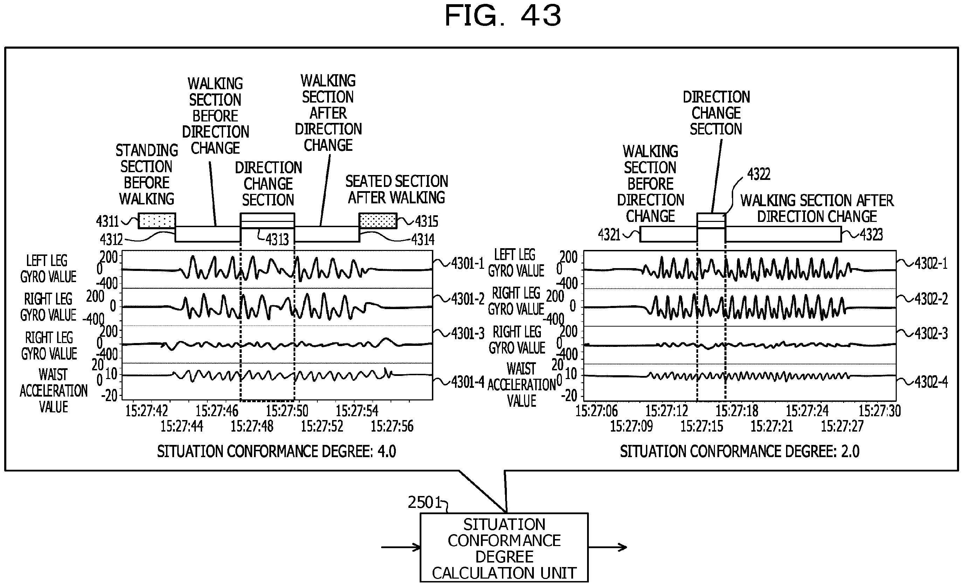

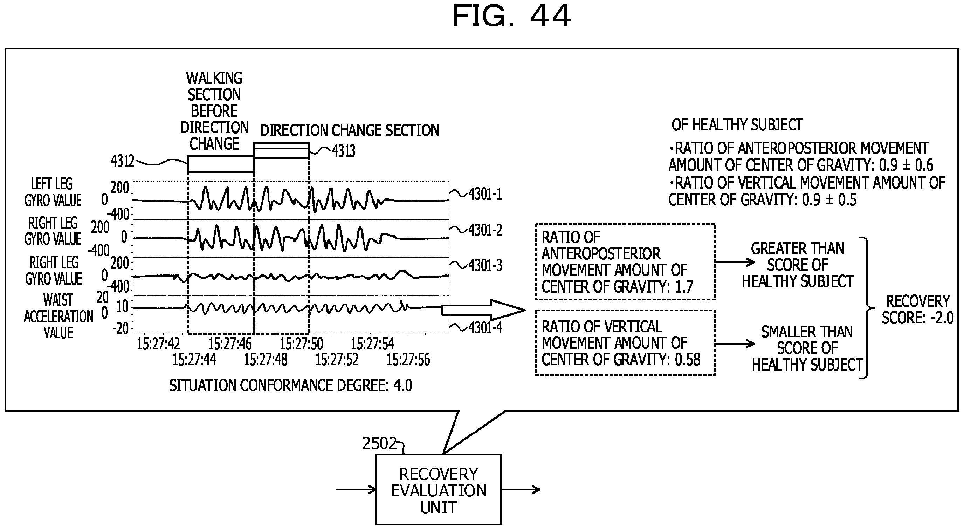

Next, an example of calculating a situation conformance degree of a direction change motion during a mobility test, as the fourth test motion, will be described with reference to FIG. 43 and an example of calculating a recovery score will be described with reference to FIG. 44.

FIG. 43 is an explanatory diagram illustrating an example of calculating the situation conformance degree of the direction change motion during the mobility test. The situation conformance degree calculation unit 2501 calculates the situation conformance degree to be high when the motion of the defined data section is the direction change section. In the example of FIG. 43, for the defined sections, the situation conformance degree calculation unit 2501 sets the initial value of the situation conformance degree of each data section to 0, and calculates the situation conformance degree by adding 1.0 each time the following condition is satisfied.

The first condition is a case where the processing target data section is a section labeled as the direction change motion section during the mobility test by the experimenter. The second condition is a case where the processing target data section is specified as the direction change section. Here, specifically, the situation conformance degree calculation unit 2501 may specify the processing target section as the direction change section by using the method described in Reference 5 described below.

### (Reference 5: Mahmoud El-Gohary, 6 others, "Continuous Monitoring of Turning in Patients with Movement Disability", 2014, Sensors. Vol. 14, pp. 356-369)

The third condition is a case where there is a walking section before and after the processing target data section. The fourth condition is a case where there is a standing section before a walking section before the processing target data section. As the fourth condition, the situation conformance degree calculation unit 2501 may specify the processing target data section as a standing section by using the method described in Reference 6 described below.

### (Reference 6: R. C. Van Lummel, "Automated approach for quantifying the repeated sit-to-stand using one body fixed sensor in young and older adults", 2013, Gait & Posture, Vol. 38, pp. 153-156)

The fifth condition is a case where there is a seated section after a walking section after the processing target data section. As the fifth condition, the situation conformance degree calculation unit 2501 may specify the processing target data section as a seated section by using the method described in Reference 6 described above.

Sensor data 4301-1 to sensor data 4301-4 on the left side of FIG. 43 have, in order from the head, a standing section 4311 before walking, a walking section 4312 before direction change, a direction change section 4313, a walking section 4314 after direction change, and a seated section 4315 after walking. Then, since the direction change section 4313 satisfies the second to fifth conditions, the situation conformance degree calculation section 2501 calculates the situation conformance degree of the direction change section 4313 as 4.0.

Further, sensor data 4302-1 to sensor data 4302-4 illustrated on the right side of FIG. 43 have, in order from the head, a walking section 4321 before direction change, a direction change section 4322, and a walking section 4323 after direction change. Then, since the direction change section 4322 satisfies the second and third conditions, the situation conformance degree calculation section 2501 calculates the situation conformance degree of the direction change section 4322 as 2.0.

FIG. 44 is an explanatory diagram illustrating an example of calculating a recovery score in the direction change motion during the mobility test. In the example of FIG. 44, the recovery evaluation unit 2502 compares the feature quantity of the waist sway in the direction change section with the feature quantity of the healthy subject and calculates the recovery score. Specifically, the recovery evaluation unit 2502 calculates the anteroposterior and vertical movement amounts of the center of gravity of the direction change section and the walking section immediately before the direction change section, which are calculated from the waist acceleration sensor, as the feature quantity of the waist sway. Then, the recovery evaluation unit 2502 calculates a ratio thereof (the movement amount of the center of gravity of the direction change section/the movement amount of the center of gravity of the walking section).

In the example of FIG. 44, the ratio of the anteroposterior movement amount of the center of gravity of the healthy subject is 0.9 ± 0.6 and the ratio of the vertical movement amount of the center of gravity of the healthy subject is 0.9 ± 0.5. Then, the recovery evaluation unit 2502 calculates the ratio of the anteroposterior movement amount of the center of gravity in the direction change section 4313 as 1.8, and since the calculated value is greater than the score of the healthy subject, the recovery evaluation unit 2502 decreases the recovery score by 1.0. In addition, the recovery evaluation unit 2502 calculates the ratio of the vertical movement amount of the center of gravity in the direction change section 4313 as 0.38, and since the calculated value is less than the score of the healthy subject, the recovery evaluation unit 2502 decreases the recovery score by 1.0. As a result, the recovery evaluation unit 2502 calculates the recovery score of the direction change section 4313 in the direction change motion during the mobility test as -2.0.

As described above, the information processing apparatus 101 defines each data section by using a model indicating a state or a motion from time series data obtained by measuring a motion of a patient, and calculates a conformance degree of each data section from a relationship of a data section and a past data section or, in embodiments not covered by the claimed invention, from a relationship of data sections. As a result, since the doctor is able to determine which data is appropriate for the recovery evaluation from the two relationships of the sections, the information processing apparatus 101 is able to improve the reliability for the recovery evaluation of the patient.

In addition, since the information processing apparatus 101 cuts out the data section without borrowing person's hands, it is possible to evaluate a recovery even by performing a test in the related art or during daily life. In addition, since the information processing apparatus 101 operates without borrowing person's hands, it is possible to perform a continuous evaluation for a long time without borrowing anyone's hands. In addition, since the information processing apparatus 101 is able to determine the conformance degree of a scene and distinguish abnormal scenes, it is possible to suppress false detection of the data section. In addition, since the information processing apparatus 101 accumulates data of each data section, it is possible to compare own temporal changes with temporal changes of other persons or to visualize a plurality of pieces of data at the same time.

Further, the information processing apparatus 101 may use a plurality of models indicating different motions or states. As a result, since the information processing apparatus 101 is able to handle a plurality of situations, states, or motions, it is possible to suppress the detection failure of the data section.

In addition, the information processing apparatus 101 calculates at least the habit conformance degree from the conformance degrees including the situation conformance degree, the habit conformance degree, and the temporal-change conformance degree. Thus, in a case where the situation conformance degree is calculated, since the information processing apparatus 101 calculates the situation conformance degree as a high value when the processing target data section is in the same situation as the specific test, it is possible to determine that the processing target data section has data suitable for the recovery evaluation of the patient. In addition, the habit conformance degree is calculated, and since the information processing apparatus 101 calculates the habit conformance degree as a high value when the processing target data section is in the same as usual case of the patient, it is possible to determine that the processing target data section has data suitable for the recovery evaluation of the patient. In addition, in a case where the temporal-change conformance degree is calculated, since the information processing apparatus 101 calculates the temporal-change conformance degree as a high value when the processing target data section is suitable for the state change of the patient, it is possible to determine that the processing target data section has data suitable for the recovery evaluation of the patient.

Further, the information processing apparatus 101 may specify the evaluation condition matching section among data sections based on at least one of the situation conformance degree, the habit conformance degree, and the temporal change adaptability. As a result, the information processing apparatus 101 is able to discard the data of the data section that is not the evaluation condition matching section, and reduce the amount of data to be stored.

Further, the information processing apparatus 101 may select an evaluation condition matching section that satisfies a predetermined condition from the specified evaluation condition matching sections. Thereby, the information processing apparatus 101 is able to select only the section designated in advance by the doctor as the evaluation condition matching section.

Further, the information processing apparatus 101 may calculate the recovery score of the patient based on the data of the evaluation condition matching section. As a result, since the information processing apparatus 101 calculates the recovery score using the data of the section suitable for the recovery evaluation of the patient, it is possible to calculate a recovery score with reliability.

Further, the information processing apparatus 101 may calculate the likelihood of the model in each data section, and define that each data section is a section in which a motion or a state of the model of which the calculated likelihood is a predetermined threshold value or greater is performed. As a result, the information processing apparatus 101 is able to define a likely model.

The information processing method described in the embodiment may be implemented by causing a computer such as a personal computer or a workstation to execute a prepared program. This information processing program is recorded in a computer readable recording medium such as a hard disk, a flexible disk, a Compact Disc-Read Only Memory (CD-ROM), and a Digital Versatile Disk (DVD), and is executed by being read out from the recording medium by the computer. Also, the information processing program may be distributed via a network such as the Internet.

### Reference Signs List

101 INFORMATION PROCESSING APPARATUS
102 SENSOR
111, 112, 114 WALKING SECTION
113 DIRECTION CHANGE SECTION
200, 2500 INFORMATION PROCESSING SYSTEM
400 SENSOR DATA ACQUISITION UNIT
401 SELF-DIAGNOSIS SHEET INPUT RESULT ACQUISITION UNIT
402 DATA SECTION DEFINING UNIT
403, 2501 SITUATION CONFORMANCE DEGREE CALCULATION UNIT
404 HABIT CONFORMANCE DEGREE CALCULATION UNIT
405 TEMPORAL-CHANGE CONFORMANCE DEGREE CALCULATION UNIT
406 EVALUATION CONDITION MATCHING SECTION SPECIFYING UNIT
407 EVALUATION CONDITION MATCHING SECTION SELECTION UNIT
408, 2502 RECOVERY EVALUATION UNIT
410 SENSOR DATA STORAGE UNIT
411 SELF-DIAGNOSIS SHEET INPUT RESULT STORAGE UNIT
413 HEALTHY SUBJECT SCORE DATABASE

## Claims

1. An information processing apparatus (101) comprising:
a control unit (301) configured to
specify, based on time series data obtained from a sensor (102) that measures a state or a motion of a living body and a model having one or more feature quantities indicating a predetermined motion or a predetermined state, each data section in which the living body makes the predetermined motion or is in the predetermined state from the time series data, by determining a score for the time series data based on similar feature quantities appearing for each feature quantity of the model and defining a data section if the score exceeds a threshold value,
store each specified data section in association with the model, and
calculate a conformance degree that is a suitable degree of each data section for recovery evaluation of the living body, based on a relationship between the stored data sections or a relationship between each data section and a past data section that has data measured before data of each data section and is associated with the model by executing a conformance degree calculation process (404, S505) of calculating a conformance degree obtained by comparing an occurrence time of the motion, an occurrence location of the motion, and the feature quantity of the data section with a distribution of past occurrence times of the motions, a distribution of past occurrence locations of the motions, and a distribution of past feature quantities, respectively, in such a manner that a high value is set as the conformance degree when the occurrence time, the occurrence location or the feature quantity is positioned at a corresponding average value or a median value of the corresponding distribution and a low value is set as the conformance degree when the occurrence time, the occurrence location or the feature quantity is not positioned at a corresponding average value or a median value of the corresponding distribution, wherein the conformance degree is based on the position of each of the time, the location, and the motion feature quantity.

2. The information processing apparatus (101) according to claim 1, wherein the control unit (301) is configured to
specify, based on a plurality of models indicating different motions or states, or same motions or states having different parameter values, and the time series data, each data section in which the living body makes a motion of any one of the plurality of models or is in a state of any one of the plurality of models, from the time series data,
store each specified data section in association with the any one of the plurality of models, and
calculate a conformance degree of the data section, based on a relationship between the stored data sections or a relationship between each data section and a past data section that has data measured before each data section and is associated with the any one of the plurality of models.

3. The information processing apparatus (101) according to claim 1 or 2,
wherein the sensor (102) includes a first sensor attached to the living body and a second sensor attached to an environment of the living body.

4. The information processing apparatus (101) according to claim 2 or 3,
wherein the control unit (301) is configured to
specify, based on the plurality of models indicating different motions or states, and the time series data, each data section in which the living body makes a motion of any one of the plurality of models or is in a state of any one of the plurality of models from the time series data,
calculate a likelihood degree indicating likelihood that the living body makes the motion of the any one model or is in the state of the any one model in each data section, and
store each data section in association with a model of which the calculated likelihood degree is a predetermined threshold value or greater.

5. The information processing apparatus (101) according to claim 1, wherein:
the living body is a patient;
the information processing apparatus (101) further comprises a storage unit (302) configured to store, in association with the living body and a healthy subject, scores obtained by evaluating a health state, a restriction degree of an activity, a degree of physical health, an amount of social activity, a degree of pain, a degree of energy and emotion, and severity and the number of diseases in a self-diagnosis sheet; and
the control unit (301) is configured to
refer to the storage unit (302), and
calculate a recovery degree of the living body based on a comparison result of a score corresponding to the living body and a score corresponding to the healthy subject.

6. An information processing system comprising:
a sensor (102) configured to measure a state or a motion of a living body; and
an information processing apparatus (101),
wherein the information processing apparatus (101) is configured to
specify, based on time series data obtained from the sensor (102) and a model having one or more feature quantities indicating a predetermined motion or a predetermined state, each data section in which the living body makes the predetermined motion or is in the predetermined state from the time series data, by determining a score for the time series data based on similar feature quantities appearing for each feature quantity of the model and defining a data section if the score exceeds a threshold value,
store the specified data section in association with the model, and
calculate a conformance degree that is a suitable degree of each data section for recovery evaluation of the living body, based on a relationship between the stored data sections or a relationship between each data section and a past data section that has data measured before data of each data section and is associated with the model by executing a conformance degree calculation process (404, S505) of calculating a conformance degree obtained by comparing an occurrence time of the motion, an occurrence location of the motion, and the feature quantity of the data section with a distribution of past occurrence times of the motions, a distribution of past occurrence locations of the motions, and a distribution of past feature quantities, respectively, in such a manner that a high value is set as the conformance degree when the occurrence time, the occurrence location or the feature quantity is positioned at a corresponding average value or a median value of the corresponding distribution and a low value is set as the conformance degree when the occurrence time, the occurrence location or the feature quantity is not positioned at a corresponding average value or a median value of the corresponding distribution, wherein the conformance degree is based on the position of each of the time, the location, and the motion feature quantity.

7. An information processing method of causing a computer to execute a process, the process comprising:
specifying, based on time series data obtained from a sensor that measures a state or a motion of a living body, and a model having one or more feature quantities indicating a predetermined motion or a predetermined state, each data section in which the living body makes the predetermined motion or is in the predetermined state from the time series data, by determining a score for the time series data based on similar feature quantities appearing for each feature quantity of the model and defining a data section if the score exceeds a threshold value;
storing the specified data section in association with the model; and
calculating a conformance degree that is a suitable degree of each data section for recovery evaluation of the living body, based on a relationship between the stored data sections or a relationship between each data section and a past data section that has data measured before data of each data section and is associated with the model by executing a conformance degree calculation process (404, S505) of calculating a conformance degree obtained by comparing an occurrence time of the motion, an occurrence location of the motion, and a feature quantity of the data section with a distribution of past occurrence times of the motions, a distribution of past occurrence locations of the motions, and a distribution of past feature quantities, respectively, in such a manner that a high value is set as the conformance degree when the occurrence time, the occurrence location or the feature quantity is positioned at a corresponding average value or a median value of the corresponding distribution and a low value is set as the conformance degree when the occurrence time, the occurrence location or the feature quantity is not positioned at a corresponding average value or a median value of the corresponding distribution, wherein the conformance degree is based on the position of each of the time, the location, and the motion feature quantity.

## Patentansprüche

1. Informationsverarbeitungsvorrichtung (101), umfassend:
eine Steuereinheit (301), die ausgelegt ist zum
Spezifizieren, basierend auf Zeitreihendaten, die von einem Sensor (102) erhalten werden, der einen Zustand oder eine Bewegung eines lebenden Körpers misst, und einem Modell mit einer oder mehreren Merkmalsgrößen, die eine vorbestimmte Bewegung oder einen vorbestimmten Zustand angeben, jedes Datenabschnitts, in dem der lebende Körper die vorbestimmte Bewegung ausführt oder sich in dem vorbestimmten Zustand befindet, aus den Zeitreihendaten, durch Bestimmen eines Scores für die Zeitreihendaten basierend auf ähnlichen Merkmalsgrößen, die für jede Merkmalsgröße des Modells erscheinen, und Definieren eines Datenabschnitts, wenn der Score einen Schwellenwert überschreitet, Speichern jedes spezifizierten Datenabschnitts in Zuordnung zu dem Modell, und Berechnen eines Konformitätsgrades, der ein geeigneter Grad jedes Datenabschnitts für eine Erholungsbewertung des lebenden Körpers ist, basierend auf einer Beziehung zwischen den gespeicherten Datenabschnitten oder einer Beziehung zwischen jedem Datenabschnitt und einem vergangenen Datenabschnitt, der Daten aufweist, die vor Daten eines jeden Datenabschnitts gemessen wurden, und dem Modell zugeordnet ist, durch Ausführen eines Konformitätsgrad-Berechnungsprozesses (404, S505) zum Berechnen eines Konformitätsgrads, der durch Vergleichen einer Auftrittszeit der Bewegung, eines Auftrittsorts der Bewegung, und der Merkmalsgröße des Datenabschnitts mit einer Verteilung vergangener Auftrittszeiten der Bewegungen, einer Verteilung vergangener Auftrittsorte der Bewegungen bzw. einer Verteilung vergangener Merkmalsgrößen erhalten wird, auf eine Weise, dass ein hoher Wert als Konformitätsgrad eingestellt wird, wenn die Auftrittszeit, der Auftrittsort oder die Merkmalsgröße auf einen entsprechenden Mittelwert oder Medianwert der entsprechenden Verteilung positioniert sind, und ein niedriger Wert als Konformitätsgrad eingestellt wird, wenn die Auftrittszeit, der Auftrittsort oder die Merkmalsgröße nicht auf einem entsprechenden Durchschnittswert oder Medianwert der entsprechenden Verteilung positioniert sind, wobei der Konformitätsgrad auf der jeweiligen Position der Zeit, des Ortes und der Bewegungsmerkmalsgröße basiert.

2. Informationsverarbeitungsvorrichtung (101) nach Anspruch 1,
wobei die Steuereinheit (301) ausgelegt ist zum
Spezifizieren, basierend auf einer Vielzahl von Modellen, die unterschiedliche Bewegungen oder Zustände oder gleiche Bewegungen oder Zustände mit unterschiedlichen Parameterwerten angeben, und der Zeitreihendaten, jedes Datenabschnitts, in dem der lebende Körper eine Bewegung eines der Vielzahl von Modellen ausführt, oder sich in einem Zustand eines der Vielzahl von Modellen befindet, aus den Zeitreihendaten, Speichern jeden spezifizierten Datenabschnitts in Zuordnung zu dem einen der Vielzahl von Modellen, und
Berechnen eines Konformitätsgrades des Datenabschnitts basierend auf einer Beziehung zwischen den gespeicherten Datenabschnitten oder einer Beziehung zwischen jedem Datenabschnitt und einem vergangenen Datenabschnitt, der Daten aufweist, die vor jedem Datenabschnitt gemessen wurden, und dem einen der Vielzahl von Modellen zugeordnet ist.

3. Informationsverarbeitungsvorrichtung (101) nach Anspruch 1 oder 2,
wobei der Sensor (102) einen an dem lebenden Körper angebrachten ersten Sensor und einen in einer Umgebung des lebenden Körpers angebrachten zweiten Sensor aufweist.

4. Informationsverarbeitungsvorrichtung (101) nach Anspruch 2 oder 3,
wobei die Steuereinheit (301) ausgelegt ist zum
Spezifizieren, basierend auf der Vielzahl von Modellen, die unterschiedliche Bewegungen oder Zustände angeben, und den Zeitreihendaten, jedes Datenabschnitts, in dem der lebende Körper eine Bewegung eines der Vielzahl von Modellen ausführt oder sich in einem Zustand eines der Vielzahl von Modellen befindet, aus den Zeitreihendaten, Berechnen eines Wahrscheinlichkeitsgrades, der eine Wahrscheinlichkeit angibt, dass der lebende Körper die Bewegung des einen Modells ausführt oder sich in dem Zustand des einen Modells befindet, in jedem Datenabschnitt, und
Speichern jedes Datenabschnitts in Zuordnung zu einem Modell, dessen berechneter Wahrscheinlichkeitsgrad ein vorbestimmter Schwellenwert oder größer ist.

5. Informationsverarbeitungsvorrichtung (101) nach Anspruch 1, wobei:
der lebende Körper ein Patient ist;
die Informationsverarbeitungsvorrichtung (101) ferner eine Speichereinheit (302) umfasst, die dazu ausgelegt ist, in Zuordnung zu dem lebenden Körper und einer gesunden Person Scores zu speichern, die durch Bewerten eines Gesundheitszustands, eines Aktivitätseinschränkungsgrads,
eines Grads an körperlicher Gesundheit, eines Umfangs an sozialer Aktivität, eines Schmerzgrads, eines Grads an Energie und Emotion, sowie des Schweregrads und der Anzahl von Krankheiten in einem Selbstdiagnosebogen erhalten werden; und
die Steuereinheit (301) ausgelegt ist zum
Heranziehen der Speichereinheit (302), und
Berechnen eines Erholungsgrads des lebenden Körpers basierend auf einem Vergleichsergebnis eines dem lebenden Körper entsprechenden Scores und eines der gesunden Person entsprechenden Scores.

6. Informationsverarbeitungssystem, umfassend:
einen Sensor (102), der dazu ausgelegt ist, einen Zustand oder eine Bewegung eines lebenden Körpers zu messen; und
eine Informationsverarbeitungsvorrichtung (101),
wobei die Informationsverarbeitungsvorrichtung (101) ausgelegt ist zum
Spezifizieren, basierend auf Zeitreihendaten, die von dem Sensor (102) erhalten werden, und einem Modell mit einer oder mehreren Merkmalsgrößen, die eine vorbestimmte Bewegung oder einen vorbestimmten Zustand angeben, jedes Datenabschnitts, in dem der lebende Körper die vorbestimmte Bewegung ausführt oder sich in dem vorbestimmten Zustand befindet, aus den Zeitreihendaten, durch Bestimmen eines Scores für die Zeitreihendaten basierend auf ähnlichen Merkmalsgrößen, die für jede Merkmalsgröße des Modells erscheinen, und Definieren eines Datenabschnitts, wenn der Score einen Schwellenwert überschreitet,
Speichern des spezifizierten Datenabschnitts in Zuordnung zu dem Modell, und Berechnen eines Konformitätsgrades, der ein geeigneter Grad jedes Datenabschnitts für eine Wiederherstellungsbewertung des lebenden Körpers ist, basierend auf einer Beziehung zwischen den gespeicherten Datenabschnitten oder einer Beziehung zwischen jedem Datenabschnitt und einem vergangenen Datenabschnitt, der Daten aufweist, die vor den Daten eines jeden Datenabschnitts gemessen wurden, und dem Modell zugeordnet ist, durch Ausführen eines Konformitätsgrad-Berechnungsprozesses (404, S505) zum Berechnen eines Konformitätsgrads, der durch Vergleichen einer Auftrittszeit der Bewegung, eines Auftrittsorts der Bewegung, und der Merkmalsgröße des Datenabschnitts mit einer Verteilung vergangener Auftrittszeiten der Bewegungen, einer Verteilung vergangener Auftrittsorte der Bewegungen bzw. einer Verteilung vergangener Merkmalsgrößen erhalten wird, auf eine Weise, dass ein hoher Wert als Konformitätsgrad eingestellt wird, wenn die Auftrittszeit, der Auftrittsort oder die Merkmalsgröße auf einen entsprechenden Mittelwert oder Medianwert der entsprechenden Verteilung positioniert sind, und ein niedriger Wert als Konformitätsgrad eingestellt wird, wenn die Auftrittszeit, der Auftrittsort oder die Merkmalsgröße nicht auf einem entsprechenden Durchschnittswert oder Medianwert der entsprechenden Verteilung positioniert sind, wobei der Konformitätsgrad auf der jeweiligen Position der Zeit, des Ortes und der Bewegungsmerkmalsgröße basiert.

7. Informationsverarbeitungsverfahren zum Bewirken, dass ein Computer einen Prozess ausführt, wobei der Prozess umfasst:
Spezifizieren, basierend auf Zeitreihendaten, die von einem Sensor erhalten werden, der einen Zustand oder eine Bewegung eines lebenden Körpers misst, und einem Modell mit einer oder mehreren Merkmalsgrößen, die eine vorbestimmte Bewegung oder einen vorbestimmten Zustand angeben, jedes Datenabschnitts, in dem der lebende Körper die vorgegebene Bewegung ausführt oder sich in dem vorgegebenen Zustand befindet, aus den Zeitreihendaten, durch Bestimmen eines Scores für die Zeitreihendaten basierend auf ähnlichen Merkmalsgrößen, die für jede Merkmalsgröße des Modells erscheinen, und Definieren eines Datenabschnitts, wenn der Score einen Schwellenwert überschreitet;
Speichern des spezifizierten Datenabschnitts in Zuordnung zu dem Modell; und
Berechnen eines Konformitätsgrades, der ein geeigneter Grad jedes Datenabschnitts für eine Erholungsbewertung des lebenden Körpers ist, basierend auf einer Beziehung zwischen den gespeicherten Datenabschnitten oder einer Beziehung zwischen jedem Datenabschnitt und einem vergangenen Datenabschnitt, der Daten aufweist, die vor den Daten eines jeden Datenabschnitts gemessen wurden, und dem Modell zugeordnet ist, durch Ausführen eines Konformitätsgrad-Berechnungsprozesses (404, S505) zum Berechnen eines Konformitätsgrads, der durch Vergleichen einer Auftrittszeit der Bewegung, eines Auftrittsorts der Bewegung, und einer Merkmalsgröße des Datenabschnitts mit einer Verteilung vergangener Auftrittszeiten der Bewegungen, einer Verteilung vergangener Auftrittsorte der Bewegungen, bzw. einer Verteilung vergangener Merkmalsgrößen erhalten wird, auf eine Weise, dass ein hoher Wert als Konformitätsgrad eingestellt wird, wenn die Auftrittszeit, der Auftrittsort oder die Merkmalsgröße auf einen entsprechenden Mittelwert oder Medianwert der entsprechenden Verteilung positioniert sind, und ein niedriger Wert als Konformitätsgrad eingestellt wird, wenn die Auftrittszeit, der Auftrittsort oder die Merkmalsgröße nicht auf einem entsprechenden Durchschnittswert oder Medianwert der entsprechenden Verteilung positioniert sind, wobei der Konformitätsgrad auf der jeweiligen Position der Zeit, des Ortes und der Bewegungsmerkmalsgröße basiert.

## Revendications

1. Un appareil de traitement d'informations (101) comprenant :
une unité de commande (301) configurée pour
spécifier, sur la base de données de série temporelle obtenues à partir d'un capteur (102) qui mesure un état ou un mouvement d'un corps vivant et d'un modèle ayant une ou plusieurs quantités caractéristiques indiquant un mouvement prédéterminé ou un état prédéterminé, chaque section de données dans laquelle le corps vivant effectue le mouvement prédéterminé ou est dans l'état prédéterminé à partir des données de série temporelle, en déterminant un score pour les données de série temporelle sur la base de quantités caractéristiques similaires apparaissant pour chaque quantité caractéristique du modèle et en définissant une section de données si le score dépasse une valeur seuil,
stocker chaque section de données spécifiée en association avec le modèle, et
calculer un degré de conformité qui est un degré approprié de chaque section de données pour une évaluation de récupération du corps vivant, sur la base d'une relation entre les sections de données stockées ou d'une relation entre chaque section de données et une section de données passée qui a des données mesurées avant les données de chaque section de données et est associée au modèle en exécutant un processus de calcul de degré de conformité (404, S505) de calcul d'un degré de conformité obtenu en comparant un temps d'occurrence du mouvement, un emplacement d'occurrence du mouvement, et la quantité caractéristique de la section de données avec une distribution de temps d'occurrence passés des mouvements, une distribution d'emplacements d'occurrence passés des mouvements, et une distribution de quantités caractéristiques passées, respectivement, de sorte qu'une valeur élevée est définie comme le degré de conformité lorsque le temps d'occurrence, l'emplacement d'occurrence ou la quantité caractéristique est positionné à une valeur moyenne correspondante ou une valeur médiane de la distribution correspondante et une valeur faible est définie comme le degré de conformité lorsque le temps d'occurrence, l'emplacement d'occurrence ou la quantité caractéristique n'est pas positionné à une valeur moyenne correspondante ou une valeur médiane de la distribution correspondante, dans lequel le degré de conformité est basé sur la position de chacun du temps, de l'emplacement et de la quantité caractéristique du mouvement.

2. L'appareil de traitement d'informations (101) selon la revendication 1,
dans lequel l'unité de commande (301) est configurée pour spécifier, sur la base d'une pluralité de modèles indiquant différents mouvements ou états, ou les mêmes mouvements ou états ayant différentes valeurs de paramètres, et les données de série temporelle, chaque section de données dans laquelle le corps vivant effectue un mouvement de l'un quelconque de la pluralité de modèles ou est dans un état de l'un quelconque de la pluralité de modèles, à partir des données de série temporelle,
stocker chaque section de données spécifiée en association avec l'un quelconque de la pluralité de modèles, et
calculer un degré de conformité de la section de données, sur la base d'une relation entre les sections de données stockées ou d'une relation entre chaque section de données et une section de données passée qui a des données mesurées avant chaque section de données et est associée à l'un quelconque de la pluralité de modèles.

3. L'appareil de traitement d'informations (101) selon la revendication 1 ou 2, dans lequel le capteur (102) comprend un premier capteur fixé au corps vivant et un deuxième capteur fixé à un environnement du corps vivant.

4. L'appareil de traitement d'informations (101) selon la revendication 2 ou 3,
dans lequel l'unité de commande (301) est configurée pour spécifier, sur la base de la pluralité de modèles indiquant différents mouvements ou états, et des données de série temporelle, chaque section de données dans laquelle le corps vivant effectue un mouvement de l'un quelconque de la pluralité de modèles ou est dans un état de l'un quelconque de la pluralité de modèles à partir des données de série temporelle,
calculer un degré de vraisemblance indiquant la vraisemblance que le corps vivant effectue le mouvement de l'un quelconque des modèles ou est dans l'état de l'un quelconque des modèles dans chaque section de données, et stocker chaque section de données en association avec un modèle dont le degré de vraisemblance calculé est une valeur seuil prédéterminée ou supérieure.

5. L'appareil de traitement d'informations (101) selon la revendication 1, dans lequel :
le corps vivant est un patient ;
l'appareil de traitement d'informations (101) comprend en outre une unité de stockage (302) configurée pour stocker, en association avec le corps vivant et un sujet sain, des scores obtenus en évaluant un état de santé, un degré de restriction d'une activité, un degré de santé physique, une quantité d'activité sociale, un degré de douleur, un degré d'énergie et d'émotion, et la gravité et le nombre de maladies dans une fiche d'autodiagnostic ; et
l'unité de commande (301) est configurée pour
se référer à l'unité de stockage (302), et
calculer un degré de récupération du corps vivant sur la base d'un résultat de comparaison d'un score correspondant au corps vivant et d'un score correspondant au sujet sain.

6. Un système de traitement d'informations comprenant :
un capteur (102) configuré pour mesurer un état ou un mouvement d'un corps vivant ; et
un appareil de traitement d'informations (101),
dans lequel l'appareil de traitement d'informations (101) est configuré pour spécifier, sur la base de données de série temporelle obtenues à partir du capteur (102) et d'un modèle ayant une ou plusieurs quantités caractéristiques indiquant un mouvement prédéterminé ou un état prédéterminé, chaque section de données dans laquelle le corps vivant effectue le mouvement prédéterminé ou est dans l'état prédéterminé à partir des données de série temporelle, en déterminant un score pour les données de série temporelle sur la base de quantités caractéristiques similaires apparaissant pour chaque quantité caractéristique du modèle et en définissant une section de données si le score dépasse une valeur seuil,
stocker la section de données spécifiée en association avec le modèle, et
calculer un degré de conformité qui est un degré approprié de chaque section de données pour l'évaluation de récupération du corps vivant, sur la base d'une relation entre les sections de données stockées ou d'une relation entre chaque section de données et une section de données passée qui a des données mesurées avant les données de chaque section de données et est associée au modèle en exécutant un processus de calcul de degré de conformité (404, S505) de calcul d'un degré de conformité obtenu en comparant un temps d'occurrence du mouvement, un emplacement d'occurrence du mouvement, et la quantité caractéristique de la section de données avec une distribution de temps d'occurrence passés des mouvements, une distribution d'emplacements d'occurrence passés des mouvements, et une distribution de quantités caractéristiques passées, respectivement, de sorte qu'une valeur élevée est définie comme le degré de conformité lorsque le temps d'occurrence, l'emplacement d'occurrence ou la quantité caractéristique est positionné à une valeur moyenne correspondante ou une valeur médiane de la distribution correspondante et une valeur faible est définie comme le degré de conformité lorsque le temps d'occurrence, l'emplacement d'occurrence ou la quantité caractéristique n'est pas positionné à une valeur moyenne correspondante ou une valeur médiane de la distribution correspondante, dans lequel le degré de conformité est basé sur la position de chacun du temps, de l'emplacement et de la quantité caractéristique du mouvement.

7. Un procédé de traitement d'informations consistant à faire en sorte qu'un ordinateur exécute un processus, le processus comprenant :
la spécification, sur la base de données de série temporelle obtenues à partir d'un capteur qui mesure un état ou un mouvement d'un corps vivant, et d'un modèle ayant une ou plusieurs quantités caractéristiques indiquant un mouvement prédéterminé ou un état prédéterminé, de chaque section de données dans laquelle le corps vivant effectue le mouvement prédéterminé ou est dans l'état prédéterminé à partir des données de série temporelle, en déterminant un score pour les données de série temporelle sur la base de quantités caractéristiques similaires apparaissant pour chaque quantité caractéristique du modèle et en définissant une section de données si le score dépasse une valeur seuil ;
le stockage de la section de données spécifiée en association avec le modèle ; et
le calcul d'un degré de conformité qui est un degré approprié de chaque section de données pour l'évaluation de récupération du corps vivant, sur la base d'une relation entre les sections de données stockées ou d'une relation entre chaque section de données et une section de données passée qui a des données mesurées avant les données de chaque section de données et est associée au modèle en exécutant un processus de calcul de degré de conformité (404, S505) de calcul d'un degré de conformité obtenu en comparant un temps d'occurrence du mouvement, un emplacement d'occurrence du mouvement, et une quantité caractéristique de la section de données avec une distribution de temps d'occurrence passés des mouvements, une distribution d'emplacements d'occurrence passés des mouvements, et une distribution de quantités caractéristiques passées, respectivement, de sorte qu'une valeur élevée est définie comme le degré de conformité lorsque le temps d'occurrence, l'emplacement d'occurrence ou la quantité caractéristique est positionné à une valeur moyenne correspondante ou une valeur médiane de la distribution correspondante et une valeur faible est définie comme le degré de conformité lorsque le temps d'occurrence, l'emplacement d'occurrence ou la quantité caractéristique n'est pas positionné à une valeur moyenne correspondante ou une valeur médiane de la distribution correspondante, dans lequel le degré de conformité est basé sur la position de chacun du temps, de l'emplacement et de la quantité caractéristique du mouvement.
